(19) Europäisches Patentamt
European Patent Office
Office européen des brevets

(11) EP 3 466 949 B1

(12) EUROPEAN PATENT SPECIFICATION

(45) Date of publication and mention of the grant of the patent:
**23.12.2020 Bulletin 2020/52**

(51) Int Cl.:
***C07D 471/04** (2006.01)* ***A61K 31/437** (2006.01)*
***A61P 35/00** (2006.01)*

(21) Application number: **18192207.1**

(22) Date of filing: **23.12.2014**

(54) **TRICYCLIC COMPOUND AS ANTICANCER AGENTS**

TRICYCLISCHE VERBINDUNG ALS ANTIKREBSMITTEL

COMPOSÉ TRICYCLIQUES EN TANT QU'AGENTS ANTICANCÉREUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2013 US 201361920500 P**

(43) Date of publication of application:
**10.04.2019 Bulletin 2019/15**

(62) Document number(s) of the earlier application(s) in accordance with Art. 76 EPC:
**14824743.0 / 3 087 071**

(73) Proprietor: **Bristol-Myers Squibb Company**
**Princeton, NJ 08543 (US)**

(72) Inventors:
- **NORRIS, Derek J.**
  **Princeton, NJ 08543 (US)**
- **DELUCCA, George V.**
  **Princeton, NJ 08543 (US)**
- **GAVAI, Ashvinikumar V.**
  **Princeton, NJ 08543 (US)**
- **QUESNELLE, Claude A.**
  **Princeton, NJ 08543 (US)**
- **GILL, Patrice**
  **Princeton, NJ 08543 (US)**
- **O'MALLEY, Daniel**
  **Princeton, NJ 08543 (US)**
- **VACCARO, Wayne**
  **Princeton, NJ 08543 (US)**
- **LEE, Francis Y.**
  **Princeton, NJ 08543 (US)**
- **DEBENEDETTO, Mikkel V.**
  **Wallingford, Connecticut 06492 (US)**
- **DEGNAN, Andrew P.**
  **Wallingford, Connecticut 06492 (US)**
- **FANG, Haiquan**
  **Madison, Connecticut 06443 (US)**
- **HILL, Matthew D.**
  **Wallingford, Connecticut 06492 (US)**
- **HUANG, Hong**
  **Durham, Connecticut 06422 (US)**
- **SCHMITZ, William D.**
  **Wallingford, Connecticut 06492 (US)**
- **STARRETT, JR, John E.**
  **Waterford, Connecticut 06385 (US)**
- **HAN, Wen-Ching**
  **Wallingford, Connecticut 06492 (US)**
- **TOKARSKI, John S.**
  **Wallingford, Connecticut 06492 (US)**
- **MANDAL, Sunil Kumar**
  **Karnataka 56009 (IN)**

(74) Representative: **Mewburn Ellis LLP**
**Aurora Building**
**Counterslip**
**Bristol BS1 6BX (GB)**

(56) References cited:
**WO-A1-2012/145173 WO-A1-2013/046635**
**WO-A1-2014/086739 WO-A1-2014/134232**
**WO-A1-2014/134267 WO-A1-2014/164596**

- **CONWAY: ACS MED. CHEM. LETT., vol. 3, 2012, pages 691-694, XP055124436,**
- **HEWINGS ET AL.: J. MED. CHEM., vol. 55, 2012, pages 9393-9413, XP055124430,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• GÖRLITZER K ET AL: "Reaktionen von 4,5-Dihydro-4-oxo-1H-pyrido(3,2-b)indol-2-carbonsäure-estern 1-3", DIE PHARMAZIE, GOVI VERLAG PHARMAZEUTISCHER VERLAG GMBH, ESCHBORN, DE, vol. 55, no. 4, 1 January 2000 (2000-01-01), pages 273-281, XP001526711, ISSN: 0031-7144

**Description**

FIELD OF THE INVENTION

The invention provides novel tricyclic compounds, pharmaceutical compositions comprising the compounds, and their use in treating a patient, for example, for the treatment of certain cancers.

BACKGROUND OF THE INVENTION

The genomes of eukaryotic organisms are highly organized within the nucleus of the cell. The long strands of duplex DNA are wrapped around an octomer of histone proteins to form a nucleosome. This basic unit is then further compressed by the aggregation and folding of nucleosomes to form a highly condensed chromatin structure. A range of different states of condensation are possible, and the tightness of this structure varies during the cell cycle, being most compact during the process of cell division. There has been appreciation recently that chromatin templates form a fundamentally important set of gene control mechanisms referred to as epigenetic regulation. By conferring a wide range of specific chemical modifications to histones and DNA (such as acetylation, methylation, phosphorylation, ubiquitinylation and SUMOylation) epigenetic regulators modulate the structure, function and accessibility of our genome, thereby exerting a huge impact in gene expression.

Histone acetylation is most usually associated with the activation of gene transcription, as the modification loosens the interaction of the DNA and the histone octomer by changing the electrostatics. In addition to this physical change, specific proteins bind to acetylated lysine residues within histones to read the epigenetic code. Bromodomains are small (~110 amino acid) distinct domains within proteins that bind to acetylated lysine residues commonly but not exclusively in the context of histones. There is a family of around 50 proteins known to contain bromodomains, and they have a range of functions within the cell. The BET family of bromodomain containing proteins comprises 4 proteins (BRD2, BRD3, BRD4 and BRD-T) which contain tandem bromodomains capable of binding to two acetylated lysine residues in close proximity, increasing the specificity of the interaction.

BRD2 and BRD3 are reported to associate with histones along actively transcribed genes and may be involved in facilitating transcriptional elongation (Leroy et al., Mol. Cell. 2008 30(1):51-60), while BRD4 appears to be involved in the recruitment of the pTEF-I3 complex to inducible genes, resulting in phosphorylation of RNA polymerase and increased transcriptional output (Hargreaves et al., Cell, 2009 138(1): 1294145). All family members have been reported to have some function in controlling or executing aspects of the cell cycle, and have been shown to remain in complex with chromosomes during cell division - suggesting a role in the maintenance of epigenetic memory. In addition some viruses make use of these proteins to tether their genomes to the host cell chromatin, as part of the process of viral replication (You et al., Cell, 2004 117(3):349-60).

Recent articles relating to this target include Prinjha et al., Trends in Pharmacological Sciences, March 2012, Vol. 33, No. 3, pp. 146-153; Conway, ACS Med. Chem. Lett., 2012, 3, 691-694 and Hewings et al., J. Med. Chem., 2012, 55, 9393-9413.

Small molecule BET inhibitors that are reported to be in development include GSK-525762A, OTX-015, TEN-010 as well as others from the University of Oxford and Constellation Pharmaceuticals Inc.

Hundreds of epigenetic effectors have been identified, many of which are chromatin-binding proteins or chromatin-modifying enzymes. These proteins have been associated with a variety of disorders such as neurodegenerative disorders, metabolic diseases, inflammation and cancer. Thus, these compounds which inhibit the binding of a bromodomain with its cognate acetylated proteins, promise new approaches in the treatment of a range of autoimmune and inflammatory diseases or conditions and in the treatment of various types of cancer.

SUMMARY OF THE INVENTION

In a first aspect the invention relates to compounds of the formula

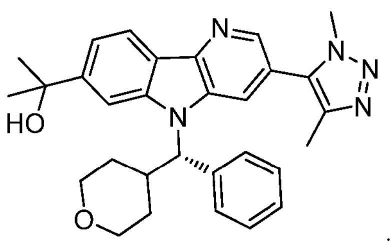

.

In a second aspect the invention relates to a pharmaceutically acceptable salt of compounds having the structure

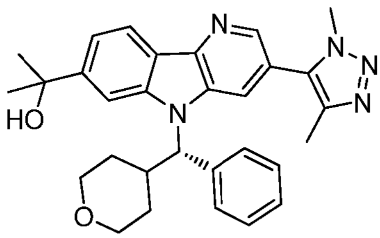

.

In a third aspect the invention relates to pharmaceutical compositions which comprise a compound of the first aspect of the invention or a pharmaceutically acceptable salt thereof and one or more pharmaceutically acceptable carriers, diluents or excipients.

In a fourth aspect the invention relates to compounds of the first aspect of the invention for use in treating cancer in a patient wherein the cancer is small cell lung cancer, non-small cell lung cancer, colorectal cancer, multiple myeloma or AML.

In a fith aspect the invention relates to pharmaceutically acceptable salts of the second aspect of the invention for use in treating cancer in a patient wherein the cancer is small cell lung cancer, non-small cell lung cancer, colorectal cancer, multiple myeloma or AML

There is disclosed a compound of formula (I)

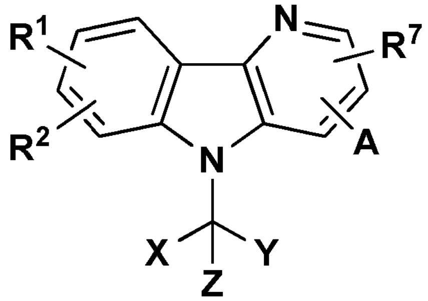

(I)

wherein:

A is optionally substituted heterocyclo or optionally substituted heteroaryl, wherein the substituents are one or more R;
R is independently one or more hydrogen, $CD_3$, halogen, haloalkyl, hydroxyalkyl, CN, $CF_3$, $CH_2F$, $CHF_2$, optionally substituted ($C_1$-$C_6$)alkyl, optionally substituted ($C_1$-$C_6$)alkoxy, optionally substituted ($C_3$-$C_6$)cycloalkyl, optionally substituted heterocyclo,-$OR^4$, -$CONR^3R^4$, -$NR^3R^4$, $NR^3R^4$($C_1$-$C_6$)alkyl-, -$NR^6OCOR^3$, -$NR^6COR^3$, $NR^6COR^3$($C_1$-$C_6$)alkyl-, -$NR^6CO_2R^3$, $NR^6CO_2R^3$($C_1$-$C_6$)alkyl-,-$NR^6CONR^3R^4$, -$SO_2NR^3R^4$, $SO_2$($C_1$-$C_6$)alkyl-, -$NR^6SO_2NR^3R^4$, -$NR^6SO_2R^4$ or $NR^6SO_2R^4$($C_1$-$C_6$)alkyl-;
X and Y are independently selected from hydrogen, optionally substituted ($C_1$-$C_6$)alkyl, optionally substituted ($C_3$-$C_8$)cycloalkyl, optionally substituted aryl, optionally substituted heteroaryl or optionally substituted heterocyclo;
Z is hydrogen, halogen, -OH, ($C_1$-$C_6$)alkyl, ($C_1$-$C_6$)alkoxy, -$NR^3R^4$, -$CONR^3R^4$,-$OCONR^3R^4$, -$NR^6OCOR^3$, -$NR^6CONR^3R^4$, -$NR^6SO_2NR^3R^4$ or -$NR^6SO_2R^4$;
$R^1$ is, independently at each occurrence, one or more hydrogen, halogen, -CN,-$OR^4$, -$NR^3R^4$, -$CONR^3R^4$, -COOH, -$OCONR^3R^4$, -$NR^6OCOR^3$, -$NR^6CONR^3R^4$,-$NR^6SO_2NR^3R^4$, -$NR^6SO_2R^4$, optionally substituted ($C_1$-$C_6$)alkyl, optionally substituted ($C_2$-$C_6$)alkenyl, optionally substituted ($C_2$-$C_6$)alkynyl, optionally substituted ($C_1$-$C_6$)alkoxy, optionally substituted ($C_3$-$C_8$)cycloalkyl, optionally substituted ($C_3$-$C_8$)cycloalkyl ($C_1$-$C_6$)alkyl, optionally substituted ($C_3$-$C_8$)cycloalkyl-CO-, optionally substituted ($C_3$-$C_8$)cycloalkyl-$SO_2$-, optionally substituted aryl ($C_1$-$C_6$)alkoxy, optionally substituted ($C_3$-$C_8$)cycloalkyl ($C_1$-$C_6$)alkoxy, optionally substituted heterocyclyl-CO-, optionally substituted heterocyclyl, optionally substituted ($C_1$-$C_6$)alkyl-$SO_2$-, -$NR^6SO_2$-optionally substituted ($C_1$-$C_6$)alkyl, - $NR^6SO_2$-optionally substituted heterocyclo, optionally substituted ($C_1$-$C_6$)alkyl-$NR^6SO_2$- or optionally substituted heterocyclo-$NR^6SO_2$-;

$R^2$ is hydrogen, halogen, -CN, OH, -$CONR^3R^4$, -$NR^6COOR^4$, -$NR^6CONR^3R^4$, -$NR^6COR^4$, -$NR^6SO_2R^5$, -$SO_2NR^3R^4$, -$NR^6SO_2NR^3R^4$, optionally substituted ($C_1$-$C_6$)alkyl, optionally substituted ($C_3$-$C_8$)cycloalkyl, optionally substituted ($C_1$-$C_6$) alkoxy, optionally substituted aryl, optionally substituted ($C_1$-$C_6$)alkyl-$SO_2$-, optionally substituted aryl-$SO_2$, optionally substituted heteroaryl or optionally substituted heterocyclo;

$R^3$ is hydrogen, optionally substituted ($C_1$-$C_6$)alkyl, optionally substituted ($C_3$-$C_8$)cycloalkyl, optionally substituted ($C_2$-$C_6$)alkenyl, optionally substituted ($C_2$-$C_6$)alkynyl, cyano($C_1$-$C_6$)alkyl, hydroxy($C_1$-$C_6$)alkyl, optionally substituted aryl, optionally substituted aryl($C_1$-$C_6$)alkyl, optionally substituted aryloxy($C_1$-$C_6$)alkyl, optionally substituted ($C_1$-$C_6$)alkyl-$SO_2$-, optionally substituted heterocyclyl, optionally substituted heterocyclyl($C_1$-$C_6$)alkyl, optionally substituted heteroaryl or optionally substituted heteroaryl($C_1$-$C_6$)alkyl,

$R^4$ is hydrogen, optionally substituted ($C_1$-$C_6$)alkyl or optionally substituted ($C_3$-$C_8$)cycloalkyl;

or $R^3$ and $R^4$ may be taken together with the nitrogen atom to which they are attached to form an optionally substituted ($C_4$-$C_8$) heteroaryl or ($C_4$-$C_8$) heterocyclic ring;

$R^5$ is hydrogen, optionally substituted ($C_1$-$C_6$)alkyl, optionally substituted ($C_3$-$C_8$)cycloalkyl, optionally substituted ($C_2$-$C_6$)alkenyl, optionally substituted ($C_2$-$C_6$)alkynyl, cyano($C_1$-$C_6$)alkyl, hydroxy($C_1$-$C_6$)alkyl, optionally substituted aryl, optionally substituted aryl($C_1$-$C_6$)alkyl, optionally substituted aryloxy($C_1$-$C_6$)alkyl, optionally substituted ($C_1$-$C_6$)alkyl-$SO_2$-, optionally substituted heterocyclyl, optionally substituted heterocyclyl($C_1$-$C_6$)alkyl, optionally substituted heteroaryl or optionally substituted heteroaryl($C_1$-$C_6$)alkyl;

$R^6$ is hydrogen or optionally substituted ($C_1$-$C_6$)alkyl;

$R^7$ is hydrogen, optionally substituted ($C_1$-$C_6$)alkyl, -$OR^4$, CN or halogen;

and/or a pharmaceutically acceptable salt, tautomer or stereoisomer thereof.

In another aspect, there is provided a pharmaceutical composition comprising a compound of the invention or a pharmaceutically acceptable salt thereof and one or more pharmaceutically acceptable carriers, diluents or excipients.

In another aspect of the disclosure, there is provided a compound of the invention or a pharmaceutically acceptable salt thereof for use in therapy. In particular, for use in the treatment of a disease or condition for which a bromodomain inhibitor is indicated.

In another aspect of the disclosure, there is provided a method of treating autoimmune and inflammatory diseases or conditions which comprises administering to a subject in need thereof a therapeutically effective amount of a bromodomain inhibitor.

In another aspect of the disclosure, there is provided a method of treating cancer which comprises administering to a subject in need thereof a therapeutically effective amount of a bromodomain inhibitor.

In another aspect of the present disclosure, there is provided a method for treating a bromodomain-containing protein mediated disorder in a patient in need thereof, comprising the step of administering to said patient a compound of the invention.

DETAILED DESCRIPTION OF THE INVENTION

In a first aspect of the present disclosure, there is provided a compound of formula (I)

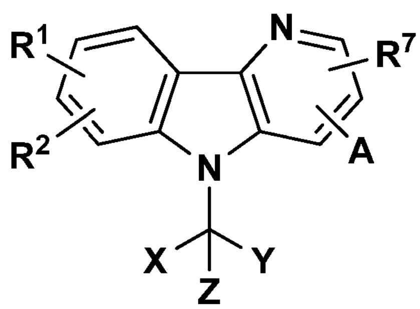

(I)

wherein:

A is optionally substituted heterocyclo or optionally substituted heteroaryl, wherein the substituents are one or more R;

R is independently one or more hydrogen, $CD_3$, halogen, haloalkyl, hydroxyalkyl, CN, $CF_3$, $CH_2F$, $CHF_2$, optionally

substituted ($C_1$-$C_6$)alkyl, optionally substituted ($C_1$-$C_6$)alkoxy, optionally substituted ($C_3$-$C_6$)cycloalkyl, optionally substituted heterocyclo,-$OR^4$, -$CONR^3R^4$, -$NR^3R^4$, $NR^3R^4$($C_1$-$C_6$)alkyl-, -$NR^6OCOR^3$, -$NR^6COR^3$, $NR^6COR^3$($C_1$-$C_6$)alkyl-, -$NR^6CO_2R^3$, $NR^6CO_2R^3$($C_1$-$C_6$)alkyl-,-$NR^6CONR^3R^4$, -$SO_2NR^3R^4$, $SO_2$($C_1$-$C_6$)alkyl-, -$NR^6SO_2NR^3R^4$, -$NR^6SO_2R^4$ or $NR^6SO_2R^4$($C_1$-$C_6$)alkyl-;

X and Y are independently selected from hydrogen, optionally substituted ($C_1$-$C_6$)alkyl, optionally substituted ($C_3$-$C_8$)cycloalkyl, optionally substituted aryl, optionally substituted heteroaryl or optionally substituted heterocyclo;

Z is hydrogen, halogen, -OH, ($C_1$-$C_6$)alkyl, ($C_1$-$C_6$)alkoxy, -$NR^3R^4$, -$CONR^3R^4$,-$OCONR^3R^4$, -$NR^6OCOR^3$, -$NR^6CONR^3R^4$, -$NR^6SO_2NR^3R^4$ or -$NR^6SO_2R^4$;

$R^1$ is, independently at each occurrence, one or more hydrogen, halogen, -CN,-$OR^4$, -$NR^3R^4$, -$CONR^3R^4$, -COOH, -$OCONR^3R^4$, -$NR^6OCOR^3$, -$NR^6CONR^3R^4$,-$NR^6SO_2NR^3R^4$, -$NR^6SO_2R^4$, optionally substituted ($C_1$-$C_6$)alkyl, optionally substituted ($C_2$-$C_6$)alkenyl, optionally substituted ($C_2$-$C_6$)alkynyl, optionally substituted ($C_1$-$C_6$)alkoxy, optionally substituted ($C_3$-$C_8$)cycloalkyl, optionally substituted ($C_3$-$C_8$)cycloalkyl ($C_1$-$C_6$)alkyl, optionally substituted ($C_3$-$C_8$)cycloalkyl-CO-, optionally substituted ($C_3$-$C_8$)cycloalkyl-$SO_2$-, optionally substituted aryl ($C_1$-$C_6$)alkoxy, optionally substituted ($C_3$-$C_8$)cycloalkyl ($C_1$-$C_6$)alkoxy, optionally substituted heterocyclyl-CO-, optionally substituted heterocyclyl, optionally substituted ($C_1$-$C_6$)alkyl-$SO_2$-, -$NR^6SO_2$-optionally substituted ($C_1$-$C_6$)alkyl, - $NR^6SO_2$-optionally substituted heterocyclo, optionally substituted ($C_1$-$C_6$)alkyl-$NR^6SO_2$- or optionally substituted heterocyclo-$NR^6SO_2$-;

$R^2$ is hydrogen, halogen, -CN, OH, -$CONR^3R^4$, -$NR^6COOR^4$, -$NR^6CONR^3R^4$, -$NR^6COR^4$, -$NR^6SO_2R^5$, -$SO_2NR^3R^4$, -$NR^6SO_2NR^3R^4$, optionally substituted ($C_1$-$C_6$)alkyl, optionally substituted ($C_3$-$C_8$)cycloalkyl, optionally substituted ($C_1$-$C_6$) alkoxy, optionally substituted aryl, optionally substituted ($C_1$-$C_6$)alkyl-$SO_2$-, optionally substituted aryl-$SO_2$, optionally substituted heteroaryl or optionally substituted heterocyclo;

$R^3$ is hydrogen, optionally substituted ($C_1$-$C_6$)alkyl, optionally substituted ($C_3$-$C_8$)cycloalkyl, optionally substituted ($C_2$-$C_6$)alkenyl, optionally substituted ($C_2$-$C_6$)alkynyl, cyano($C_1$-$C_6$)alkyl, hydroxy($C_1$-$C_6$)alkyl, optionally substituted aryl, optionally substituted aryl($C_1$-$C_6$)alkyl, optionally substituted aryloxy($C_1$-$C_6$)alkyl, optionally substituted ($C_1$-$C_6$)alkyl-$SO_2$-, optionally substituted heterocyclyl, optionally substituted heterocyclyl($C_1$-$C_6$)alkyl, optionally substituted heteroaryl or optionally substituted heteroaryl($C_1$-$C_6$)alkyl,

$R^4$ is hydrogen, optionally substituted ($C_1$-$C_6$)alkyl or optionally substituted ($C_3$-$C_8$)cycloalkyl;

or $R^3$ and $R^4$ may be taken together with the nitrogen atom to which they are attached to form an optionally substituted ($C_4$-$C_8$) heteroaryl or ($C_4$-$C_8$) heterocyclic ring;

$R^5$ is hydrogen, optionally substituted ($C_1$-$C_6$)alkyl, optionally substituted ($C_3$-$C_8$)cycloalkyl, optionally substituted ($C_2$-$C_6$)alkenyl, optionally substituted ($C_2$-$C_6$)alkynyl, cyano($C_1$-$C_6$)alkyl, hydroxy($C_1$-$C_6$)alkyl, optionally substituted aryl, optionally substituted aryl($C_1$-$C_6$)alkyl, optionally substituted aryloxy($C_1$-$C_6$)alkyl, optionally substituted ($C_1$-$C_6$)alkyl-$SO_2$-, optionally substituted heterocyclyl, optionally substituted heterocyclyl($C_1$-$C_6$)alkyl, optionally substituted heteroaryl or optionally substituted heteroaryl($C_1$-$C_6$)alkyl;

$R^6$ is hydrogen or optionally substituted ($C_1$-$C_6$)alkyl;

$R^7$ is hydrogen, optionally substituted ($C_1$-$C_6$)alkyl, -$OR^4$, CN or halogen;

and/or a pharmaceutically acceptable salt, tautomer or stereoisomer thereof.

In a second aspect of the disclosure, there is provided a compound according to claim 1 of formula (II)

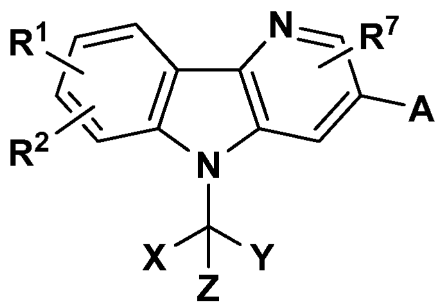

(II)

wherein:

A is optionally substituted heterocyclo or optionally substituted heteroaryl, wherein the substituents are one or more R;

R is independently one or more hydrogen, $CD_3$,halogen, haloalkyl, hydroxyalkyl, CN, $CF_3$, $CH_2F$, $CHF_2$, optionally substituted $(C_1-C_6)$alkyl, optionally substituted $(C_1-C_6)$alkoxy, optionally substituted $(C_3-C_6)$cycloalkyl, optionally substituted heterocyclo,-$OR^4$, -$CONR^3R^4$, -$NR^3R^4$, $NR^3R^4(C_1-C_6)$alkyl-, -$NR^6OCOR^3$, -$NR^6COR^3$, $NR^6COR^3(C_1-C_6)$alkyl-, -$NR^6CO_2R^3$, $NR^6CO_2R^3(C_1-C_6)$alkyl-, -$NR^6CONR^3R^4$, -$SO_2NR^3R^4$, $SO_2(C_1-C_6)$alkyl-, -$NR^6SO_2NR^3R^4$, -$NR^6SO_2R^4$ or $NR^6SO_2R^4(C_1-C_6)$alkyl-;

X and Y are independently selected from hydrogen, optionally substituted $(C_1-C_6)$alkyl, optionally substituted $(C_3-C_8)$cycloalkyl, optionally substituted aryl, optionally substituted heteroaryl or optionally substituted heterocyclo;

Z is hydrogen, halogen, -OH, $(C_1-C_6)$alkyl, $(C_1-C_6)$alkoxy, -$NR^3R^4$, -$CONR^3R^4$,-$OCONR^3R^4$, -$NR^6OCOR^3$, -$NR^6CONR^3R^4$, -$NR^6SO_2NR^3R^4$ or -$NR^6SO_2R^4$;

$R^1$ is, independently at each occurrence, one or more hydrogen, halogen, -CN,-$OR^4$, -$NR^3R^4$, -$CONR^3R^4$, -COOH, -$OCONR^3R^4$, -$NR^6OCOR^3$, -$NR^6CONR^3R^4$,-$NR^6SO_2NR^3R^4$, -$NR^6SO_2R^4$, optionally substituted $(C_1-C_6)$alkyl, optionally substituted $(C_2-C_6)$alkenyl, optionally substituted $(C_2-C_6)$alkynyl, optionally substituted $(C_1-C_6)$alkoxy, optionally substituted $(C_3-C_8)$cycloalkyl, optionally substituted $(C_3-C_8)$cycloalkyl $(C_1-C_6)$alkyl, optionally substituted $(C_3-C_8)$cycloalkyl-CO-, optionally substituted $(C_3-C_8)$cycloalkyl-$SO_2$-, optionally substituted aryl $(C_1-C_6)$alkoxy, optionally substituted $(C_3-C_8)$cycloalkyl $(C_1-C_6)$alkoxy, optionally substituted heterocyclyl-CO-, optionally substituted heterocyclyl, optionally substituted $(C_1-C_6)$alkyl-$SO_2$-, -$NR^6SO_2$-optionally substituted $(C_1-C_6)$alkyl, - $NR^6SO_2$-optionally substituted heterocyclo, optionally substituted $(C_1-C_6)$alkyl-$NR^6SO_2$- or optionally substituted heterocyclo-$NR^6SO_2$-;

$R^2$ is hydrogen, halogen, -CN, OH, -$CONR^3R^4$, -$NR^6COOR^4$, -$NR^6CONR^3R^4$, -$NR^6COR^4$, -$NR^6SO_2R^5$, -$SO_2NR^3R^4$, -$NR^6SO_2NR^3R^4$, optionally substituted $(C_1-C_6)$alkyl, optionally substituted $(C_3-C_8)$cycloalkyl, optionally substituted $(C_1-C_6)$ alkoxy, optionally substituted aryl, optionally substituted $(C_1-C_6)$alkyl-$SO_2$-, optionally substituted aryl-$SO_2$, optionally substituted heteroaryl or optionally substituted heterocyclo;

$R^3$ is hydrogen, optionally substituted $(C_1-C_6)$alkyl, optionally substituted $(C_3-C_8)$cycloalkyl, optionally substituted $(C_2-C_6)$alkenyl, optionally substituted $(C_2-C_6)$alkynyl, cyano$(C_1-C_6)$alkyl, hydroxy$(C_1-C_6)$alkyl, optionally substituted aryl, optionally substituted aryl$(C_1-C_6)$alkyl, optionally substituted aryloxy$(C_1-C_6)$alkyl, optionally substituted $(C_1-C_6)$alkyl-$SO_2$-, optionally substituted heterocyclyl, optionally substituted heterocyclyl$(C_1-C_6)$alkyl, optionally substituted heteroaryl or optionally substituted heteroaryl$(C_1-C_6)$alkyl,

$R^4$ is hydrogen, optionally substituted $(C_1-C_6)$alkyl or optionally substituted $(C_3-C_8)$cycloalkyl;

or $R^3$ and $R^4$ may be taken together with the nitrogen atom to which they are attached to form an optionally substituted $(C_4-C_8)$ heteroaryl or $(C_4-C_8)$ heterocyclic ring;

$R^5$ is hydrogen, optionally substituted $(C_1-C_6)$alkyl, optionally substituted $(C_3-C_8)$cycloalkyl, optionally substituted $(C_2-C_6)$alkenyl, optionally substituted $(C_2-C_6)$alkynyl, cyano$(C_1-C_6)$alkyl, hydroxy$(C_1-C_6)$alkyl, optionally substituted aryl, optionally substituted aryl$(C_1-C_6)$alkyl, optionally substituted aryloxy$(C_1-C_6)$alkyl, optionally substituted $(C_1-C_6)$alkyl-$SO_2$-, optionally substituted heterocyclyl, optionally substituted heterocyclyl$(C_1-C_6)$alkyl, optionally substituted heteroaryl or optionally substituted heteroaryl$(C_1-C_6)$alkyl;

$R^6$ is hydrogen or optionally substituted $(C_1-C_6)$alkyl;

$R^7$ is hydrogen, optionally substituted $(C_1-C_6)$alkyl, -$OR^4$, CN or halogen;

and/or a pharmaceutically acceptable salt, tautomer or stereoisomer thereof.

In a third aspect of the disclosure within the scope of the first two aspects, there is provided a compound of formula (II)

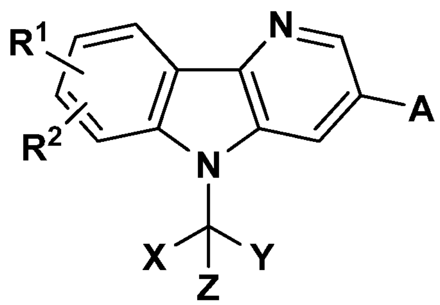

(II)

wherein:

A is optionally substituted heterocyclo or optionally substituted heteroaryl, wherein the substituents are one or more R;

R is independently one or more hydrogen, $CD_3$, halogen, haloalkyl, hydroxyalkyl, CN, $CF_3$, $CH_2F$, $CHF_2$, optionally substituted $(C_1-C_6)$alkyl, optionally substituted $(C_1-C_6)$alkoxy, optionally substituted $(C_3-C_6)$cycloalkyl, optionally substituted heterocyclo,$-OR^4$, $-CONR^3R^4$, $-NR^3R^4$, $NR^3R^4(C_1-C_6)$alkyl-, $-NR^6OCOR^3$, $-NR^6COR^3$, $NR^6COR^3(C_1-C_6)$alkyl-, $-NR^6CO_2R^3$, $NR^6CO_2R^3(C_1-C_6)$alkyl-,$-NR^6CONR^3R^4$, $-SO_2NR^3R^4$, $SO_2(C_1-C_6)$alkyl-, $-NR^6SO_2NR^3R^4$, $-NR^6SO_2R^4$ or $NR^6SO_2R^4(C_1-C_6)$alkyl-;

X and Y are independently selected from hydrogen, optionally substituted $(C_1-C_6)$alkyl, optionally substituted $(C_3-C_8)$cycloalkyl, optionally substituted aryl, optionally substituted heteroaryl or optionally substituted heterocyclo;

Z is hydrogen, halogen, -OH, $(C_1-C_6)$alkyl, $(C_1-C_6)$alkoxy, $-NR^3R^4$, $-CONR^3R^4$,$-OCONR^3R^4$, $-NR^6OCOR^3$, $-NR^6CONR^3R^4$, $-NR^6SO_2NR^3R^4$ or $-NR^6SO_2R^4$;

$R^1$ is, independently at each occurrence, one or more hydrogen, halogen, -CN,$-OR^4$, $-NR^3R^4$, $-CONR^3R^4$, -COOH, $-OCONR^3R^4$, $-NR^6OCOR^3$, $-NR^6CONR^3R^4$,$-NR^6SO_2NR^3R^4$, $-NR^6SO_2R^4$, optionally substituted $(C_1-C_6)$alkyl, optionally substituted $(C_2-C_6)$alkenyl, optionally substituted $(C_2-C_6)$alkynyl, optionally substituted $(C_1-C_6)$alkoxy, optionally substituted $(C_3-C_8)$cycloalkyl, optionally substituted $(C_3-C_8)$cycloalkyl $(C_1-C_6)$alkyl, optionally substituted $(C_3-C_8)$cycloalkyl-CO-, optionally substituted $(C_3-C_8)$cycloalkyl-$SO_2$-, optionally substituted aryl $(C_1-C_6)$alkoxy, optionally substituted $(C_3-C_8)$cycloalkyl $(C_1-C_6)$alkoxy, optionally substituted heterocyclyl-CO-, optionally substituted heterocyclyl, optionally substituted $(C_1-C_6)$alkyl-$SO_2$-, $-NR^6SO_2$-optionally substituted $(C_1-C_6)$alkyl, - $NR^6SO_2$-optionally substituted heterocyclo, optionally substituted $(C_1-C_6)$alkyl-$NR^6SO_2$- or optionally substituted heterocyclo-$NR^6SO_2$-;

$R^2$ is hydrogen, halogen, -CN, OH, optionally substituted $(C_1-C_6)$alkyl, optionally substituted $(C_3-C_8)$cycloalkyl, optionally substituted $(C_1-C_6)$ alkoxy, optionally substituted aryl, optionally substituted heteroaryl or optionally substituted heterocyclo;

$R^3$ is hydrogen, optionally substituted $(C_1-C_6)$alkyl, optionally substituted $(C_3-C_8)$cycloalkyl, optionally substituted $(C_2-C_6)$alkenyl, optionally substituted $(C_2-C_6)$alkynyl, cyano$(C_1-C_6)$alkyl, hydroxy$(C_1-C_6)$alkyl, optionally substituted aryl, optionally substituted aryl$(C_1-C_6)$alkyl, optionally substituted aryloxy$(C_1-C_6)$alkyl, optionally substituted $(C_1-C_6)$alkyl-$SO_2$-, optionally substituted heterocyclyl, optionally substituted heterocyclyl$(C_1-C_6)$alkyl, optionally substituted heteroaryl or optionally substituted heteroaryl$(C_1-C_6)$alkyl,

$R^4$ is hydrogen, optionally substituted $(C_1-C_6)$alkyl or optionally substituted $(C_3-C_8)$cycloalkyl;

or $R^3$ and $R^4$ may be taken together with the nitrogen atom to which they are attached to form an optionally substituted $(C_4-C_8)$ heteroaryl or $(C_4-C_8)$ heterocyclic ring;

$R^6$ is hydrogen or optionally substituted $(C_1-C_6)$alkyl;

and/or a pharmaceutically acceptable salt, tautomer or stereoisomer thereof.

In a 4th aspect within the scope of the prior aspects, there is provided a compound of the formula

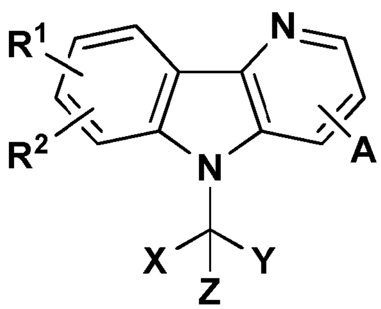

wherein

A is

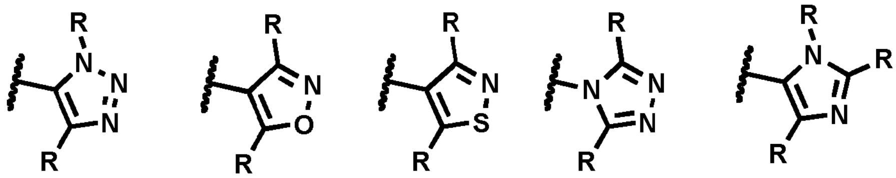

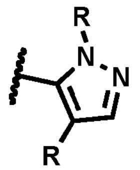 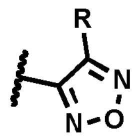 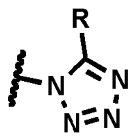 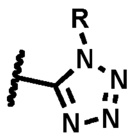 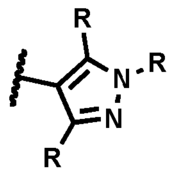

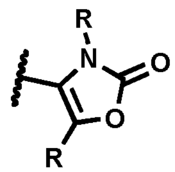 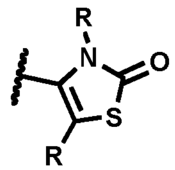 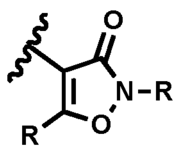 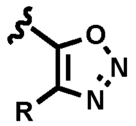 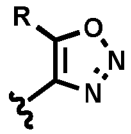

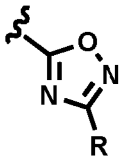 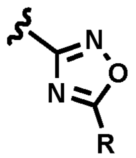 ;

R is independently one or more hydrogen, $CD_3$, halogen, haloalkyl, hydroxyalkyl, CN, $CF_3$, $CH_2F$, $CHF_2$, optionally substituted ($C_1$-$C_6$)alkyl, optionally substituted ($C_1$-$C_6$)alkoxy, optionally substituted ($C_3$-$C_6$)cycloalkyl, optionally substituted heterocyclo,-$OR^4$, -$CONR^3R^4$, -$NR^3R^4$, $NR^3R^4$($C_1$-$C_6$)alkyl-, -$NR^6OCOR^3$, -$NR^6COR^3$, $NR^6COR^3$($C_1$-$C_6$)alkyl-, -$NR^6CO_2R^3$, $NR^6CO_2R^3$($C_1$-$C_6$)alkyl-,-$NR^6CONR^3R^4$, -$SO_2NR^3R^4$, $SO_2$($C_1$-$C_6$)alkyl-, -$NR^6SO_2NR^3R^4$, -$NR^6SO_2R^4$ or $NR^6SO_2R^4$($C_1$-$C_6$)alkyl-;

X and Y are independently selected from hydrogen, optionally substituted ($C_1$-$C_6$)alkyl, optionally substituted ($C_3$-$C_8$)cycloalkyl, optionally substituted aryl, optionally substituted heteroaryl or optionally substituted heterocyclo;

Z is hydrogen, halogen, -OH, ($C_1$-$C_6$)alkyl, ($C_1$-$C_6$)alkoxy, -$NR^3R^4$, -$CONR^3R^4$,-$OCONR^3R^4$, -$NR^6OCOR^3$, -$NR^6CONR^3R^4$, -$NR^6SO_2NR^3R^4$ or -$NR^6SO_2R^4$;

$R^1$ is, independently at each occurrence, one or more hydrogen, halogen, -CN,-$OR^4$, -$NR^3R^4$, -$CONR^3R^4$, -COOH, -$OCONR^3R^4$, -$NR^6OCOR^3$, -$NR^6CONR^3R^4$,-$NR^6SO_2NR^3R^4$, -$NR^6SO_2R^4$, optionally substituted ($C_1$-$C_6$)alkyl, optionally substituted ($C_2$-$C_6$)alkenyl, optionally substituted ($C_2$-$C_6$)alkynyl, optionally substituted ($C_1$-$C_6$)alkoxy, optionally substituted ($C_3$-$C_8$)cycloalkyl, optionally substituted ($C_3$-$C_8$)cycloalkyl ($C_1$-$C_6$)alkyl, optionally substituted ($C_3$-$C_8$)cycloalkyl-CO-, optionally substituted ($C_3$-$C_8$)cycloalkyl-$SO_2$-, optionally substituted aryl ($C_1$-$C_6$)alkoxy, optionally substituted ($C_3$-$C_8$)cycloalkyl ($C_1$-$C_6$)alkoxy, optionally substituted heterocyclyl-CO-, optionally substituted heterocyclyl, optionally substituted ($C_1$-$C_6$)alkyl-$SO_2$-, -$NR^6SO_2$-optionally substituted ($C_1$-$C_6$)alkyl, - $NR^6SO_2$-optionally substituted heterocyclo, optionally substituted ($C_1$-$C_6$)alkyl-$NR^6SO_2$- or optionally substituted heterocyclo-$NR^6SO_2$-;

$R^2$ is hydrogen, halogen, -CN, OH, optionally substituted ($C_1$-$C_6$)alkyl, optionally substituted ($C_3$-$C_8$)cycloalkyl, optionally substituted ($C_1$-$C_6$) alkoxy, optionally substituted aryl, optionally substituted heteroaryl or optionally substituted heterocyclo;

$R^3$ is hydrogen, optionally substituted ($C_1$-$C_6$)alkyl, optionally substituted ($C_3$-$C_8$)cycloalkyl, optionally substituted ($C_2$-$C_6$)alkenyl, optionally substituted ($C_2$-$C_6$)alkynyl, cyano($C_1$-$C_6$)alkyl, hydroxy($C_1$-$C_6$)alkyl, optionally substituted aryl, optionally substituted aryl($C_1$-$C_6$)alkyl, optionally substituted aryloxy($C_1$-$C_6$)alkyl, optionally substituted ($C_1$-$C_6$)alkyl-$SO_2$-, optionally substituted heterocyclyl, optionally substituted heterocyclyl($C_1$-$C_6$)alkyl, optionally substituted heteroaryl or optionally substituted heteroaryl($C_1$-$C_6$)alkyl,

$R^4$ is hydrogen, optionally substituted ($C_1$-$C_6$)alkyl or optionally substituted ($C_3$-$C_8$)cycloalkyl;

or $R^3$ and $R^4$ may be taken together with the nitrogen atom to which they are attached to form an optionally substituted ($C_4$-$C_8$) heteroaryl or ($C_4$-$C_8$) heterocyclic ring;

$R^6$ is hydrogen or optionally substituted ($C_1$-$C_6$)alkyl;

and/or a pharmaceutically acceptable salt, tautomer or stereoisomer thereof.

In a 5th aspect of the disclosure within the scope of the prior aspects, there is provided a compound of the formula

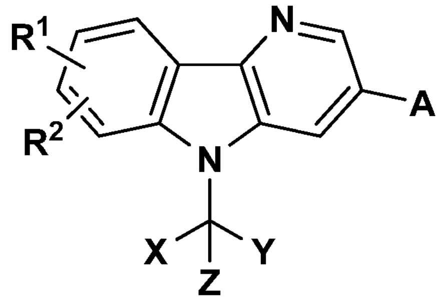

(II)

wherein:

A is

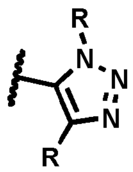 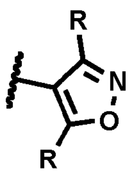 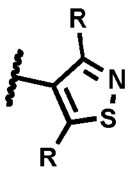 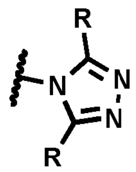 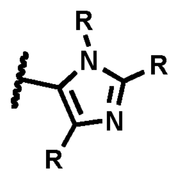

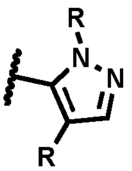 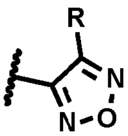 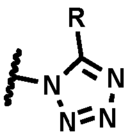 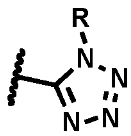 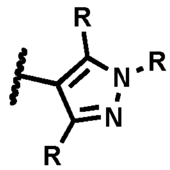

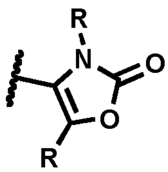 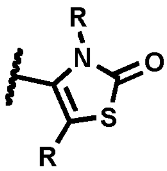 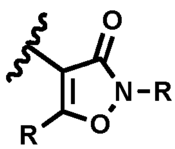 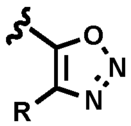 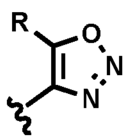

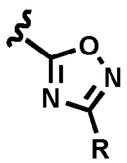 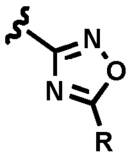

;

R is independently one or more hydrogen, $CD_3$, halogen, haloalkyl, hydroxyalkyl, CN, $CF_3$, $CH_2F$, $CHF_2$, optionally substituted ($C_1$-$C_6$)alkyl, optionally substituted ($C_1$-$C_6$)alkoxy, optionally substituted ($C_3$-$C_6$)cycloalkyl, optionally substituted heterocyclo,-$OR^4$, -$CONR^3R^4$, -$NR^3R^4$, $NR^3R^4$($C_1$-$C_6$)alkyl-, -$NR^6OCOR^3$, -$NR^6COR^3$, $NR^6COR^3$($C_1$-$C_6$)alkyl-, -$NR^6CO_2R^3$, $NR^6CO_2R^3$($C_1$-$C_6$)alkyl-, -$NR^6CONR^3R^4$, -$SO_2NR^3R^4$, $SO_2$($C_1$-$C_6$)alkyl-, -$NR^6SO_2NR^3R^4$, -$NR^6SO_2R^4$ or $NR^6SO_2R^4$($C_1$-$C_6$)alkyl-;

X and Y are independently selected from hydrogen, optionally substituted ($C_1$-$C_6$)alkyl, optionally substituted ($C_3$-$C_8$)cycloalkyl, optionally substituted aryl, optionally substituted heteroaryl or optionally substituted heterocyclo;

Z is hydrogen, halogen, -OH, ($C_1$-$C_6$)alkyl, ($C_1$-$C_6$)alkoxy, -$NR^3R^4$, -$CONR^3R^4$,-$OCONR^3R^4$, -$NR^6OCOR^3$, -$NR^6CONR^3R^4$, -$NR^6SO_2NR^3R^4$ or -$NR^6SO_2R^4$;

$R^1$ is, independently at each occurrence, one or more hydrogen, halogen, -CN,-$OR^4$, -$NR^3R^4$, -$CONR^3R^4$, -COOH, -$OCONR^3R^4$, -$NR^6OCOR^3$, -$NR^6CONR^3R^4$,-$NR^6SO_2NR^3R^4$, -$NR^6SO_2R^4$, optionally substituted ($C_1$-$C_6$)alkyl, optionally substituted ($C_2$-$C_6$)alkenyl, optionally substituted ($C_2$-$C_6$)alkynyl, optionally substituted ($C_1$-$C_6$)alkoxy, optionally substituted ($C_3$-$C_8$)cycloalkyl, optionally substituted ($C_3$-$C_8$)cycloalkyl ($C_1$-$C_6$)alkyl, optionally substituted

$(C_3-C_8)$cycloalkyl-CO-, optionally substituted $(C_3-C_8)$cycloalkyl-$SO_2$-, optionally substituted aryl $(C_1-C_6)$alkoxy, optionally substituted $(C_3-C_8)$cycloalkyl $(C_1-C_6)$alkoxy, optionally substituted heterocyclyl-CO-, optionally substituted heterocyclyl, optionally substituted $(C_1-C_6)$alkyl-$SO_2$-, -$NR^6SO_2$-optionally substituted $(C_1-C_6)$alkyl, - $NR^6SO_2$-optionally substituted heterocyclo, optionally substituted $(C_1-C_6)$alkyl-$NR^6SO_2$- or optionally substituted heterocyclo-$NR^6SO_2$-;

$R^2$ is hydrogen, halogen, -CN, OH, optionally substituted $(C_1-C_6)$alkyl, optionally substituted $(C_3-C_8)$cycloalkyl, optionally substituted $(C_1-C_6)$ alkoxy, optionally substituted aryl, optionally substituted heteroaryl or optionally substituted heterocyclo;

$R^3$ is hydrogen, optionally substituted $(C_1-C_6)$alkyl, optionally substituted $(C_3-C_8)$cycloalkyl, optionally substituted $(C_2-C_6)$alkenyl, optionally substituted $(C_2-C_6)$alkynyl, cyano$(C_1-C_6)$alkyl, hydroxy$(C_1-C_6)$alkyl, optionally substituted aryl, optionally substituted aryl$(C_1-C_6)$alkyl, optionally substituted aryloxy$(C_1-C_6)$alkyl, optionally substituted $(C_1-C_6)$alkyl-$SO_2$-, optionally substituted heterocyclyl, optionally substituted heterocyclyl$(C_1-C_6)$alkyl, optionally substituted heteroaryl or optionally substituted heteroaryl$(C_1-C_6)$alkyl,

$R^4$ is hydrogen, optionally substituted $(C_1-C_6)$alkyl or optionally substituted $(C_3-C_8)$cycloalkyl;

or $R^3$ and $R^4$ may be taken together with the nitrogen atom to which they are attached to form an optionally substituted $(C_4-C_8)$ heteroaryl or $(C_4-C_8)$ heterocyclic ring;

$R^6$ is hydrogen or optionally substituted $(C_1-C_6)$alkyl;

and/or a pharmaceutically acceptable salt, tautomer or stereoisomer thereof.

In a 6th aspect of the disclosure within the scope of the prior aspects, there is provided a compound of the formula

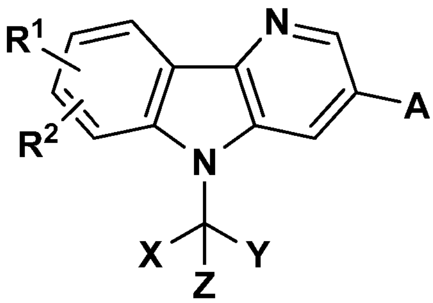

(II)

wherein:

A is

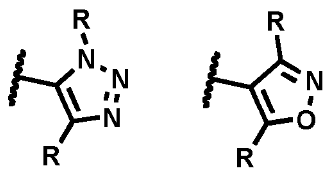

;

R is independently one or more hydrogen, $CD_3$, halogen, haloalkyl, hydroxyalkyl, CN, $CF_3$, $CH_2F$, $CHF_2$, optionally substituted $(C_1-C_6)$alkyl, optionally substituted $(C_1-C_6)$alkoxy, optionally substituted $(C_3-C_6)$cycloalkyl, optionally substituted heterocyclo,-$OR^4$, -$CONR^3R^4$, -$NR^3R^4$, $NR^3R^4(C_1-C_6)$alkyl-, -$NR^6OCOR^3$, -$NR^6COR^3$, $NR^6COR^3(C_1-C_6)$alkyl-, -$NR^6CO_2R^3$, $NR^6CO_2R^3(C_1-C_6)$alkyl-,-$NR^6CONR^3R^4$, -$SO_2NR^3R^4$, $SO_2(C_1-C_6)$alkyl-, -$NR^6SO_2NR^3R^4$, -$NR^6SO_2R^4$ or $NR^6SO_2R^4(C_1-C_6)$alkyl-;

X and Y are independently selected from hydrogen, optionally substituted $(C_1-C_6)$alkyl, optionally substituted $(C_3-C_8)$cycloalkyl, optionally substituted aryl, optionally substituted heteroaryl or optionally substituted heterocyclo;

Z is hydrogen, halogen, -OH, $(C_1-C_6)$alkyl, $(C_1-C_6)$alkoxy, -$NR^3R^4$, -$CONR^3R^4$,-$OCONR^3R^4$, -$NR^6OCOR^3$, -$NR^6CONR^3R^4$, -$NR^6SO_2NR^3R^4$ or -$NR^6SO_2R^4$;

$R^1$ is, independently at each occurrence, one or more hydrogen, halogen, -CN,-$OR^4$, -$NR^3R^4$, -$CONR^3R^4$, -COOH, -$OCONR^3R^4$, -$NR^6OCOR^3$, -$NR^6CONR^3R^4$,-$NR^6SO_2NR^3R^4$, -$NR^6SO_2R^4$, optionally substituted $(C_1-C_6)$alkyl, optionally substituted $(C_2-C_6)$alkenyl, optionally substituted $(C_2-C_6)$alkynyl, optionally substituted $(C_1-C_6)$alkoxy, optionally substituted $(C_3-C_8)$cycloalkyl, optionally substituted $(C_3-C_8)$cycloalkyl $(C_1-C_6)$alkyl, optionally substituted

$(C_3-C_8)$cycloalkyl-CO-, optionally substituted $(C_3-C_8)$cycloalkyl-$SO_2$-, optionally substituted aryl $(C_1-C_6)$alkoxy, optionally substituted $(C_3-C_8)$cycloalkyl $(C_1-C_6)$alkoxy, optionally substituted heterocyclyl-CO-, optionally substituted heterocyclyl, optionally substituted $(C_1-C_6)$alkyl-$SO_2$-, -$NR^6SO_2$-optionally substituted $(C_1-C_6)$alkyl, - $NR^6SO_2$-optionally substituted heterocyclo, optionally substituted $(C_1-C_6)$alkyl-$NR^6SO_2$- or optionally substituted heterocyclo-$NR^6SO_2$-;
$R^2$ is hydrogen, halogen, -CN, OH, optionally substituted $(C_1-C_6)$alkyl, optionally substituted $(C_3-C_8)$cycloalkyl, optionally substituted $(C_1-C_6)$ alkoxy, optionally substituted aryl, optionally substituted heteroaryl or optionally substituted heterocyclo;
$R^3$ is hydrogen, optionally substituted $(C_1-C_6)$alkyl, optionally substituted $(C_3-C_8)$cycloalkyl, optionally substituted $(C_2-C_6)$alkenyl, optionally substituted $(C_2-C_6)$alkynyl, cyano$(C_1-C_6)$alkyl, hydroxy$(C_1-C_6)$alkyl, optionally substituted aryl, optionally substituted aryl$(C_1-C_6)$alkyl, optionally substituted aryloxy$(C_1-C_6)$alkyl, optionally substituted $(C_1-C_6)$alkyl-$SO_2$-, optionally substituted heterocyclyl, optionally substituted heterocyclyl$(C_1-C_6)$alkyl, optionally substituted heteroaryl or optionally substituted heteroaryl$(C_1-C_6)$alkyl,
$R^4$ is hydrogen, optionally substituted $(C_1-C_6)$alkyl or optionally substituted $(C_3-C_8)$cycloalkyl;
or $R^3$ and $R^4$ may be taken together with the nitrogen atom to which they are attached to form an optionally substituted $(C_4-C_8)$ heteroaryl or $(C_4-C_8)$ heterocyclic ring;
$R^6$ is hydrogen or optionally substituted $(C_1-C_6)$alkyl;
and/or a pharmaceutically acceptable salt, tautomer or stereoisomer thereof.

In a 7th aspect of the disclosure within the scope of the prior aspects, there is provided a compound of the formula

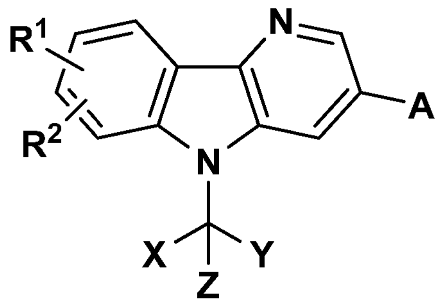

(II)

wherein:

A is

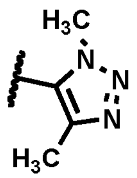
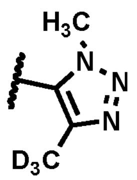
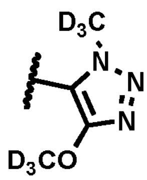
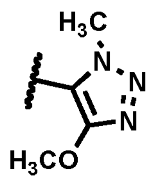
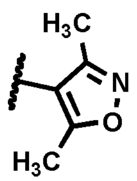
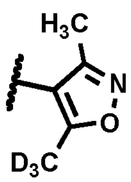
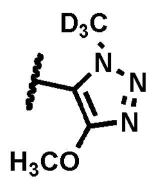
;

X and Y are independently selected from hydrogen, optionally substituted $(C_1-C_6)$alkyl, optionally substituted $(C_3-C_8)$cycloalkyl, optionally substituted aryl, optionally substituted heteroaryl or optionally substituted heterocyclo;
Z is hydrogen, halogen, -OH, $(C_1-C_6)$alkyl, $(C_1-C_6)$alkoxy, -$NR^3R^4$, -$CONR^3R^4$,-$OCONR^3R^4$, -$NR^6OCOR^3$, -$NR^6CONR^3R^4$, -$NR^6SO_2NR^3R^4$ or -$NR^6SO_2R^4$;
$R^1$ is, independently at each occurrence, one or more hydrogen, halogen, -CN,-$OR^4$, -$NR^3R^4$, -$CONR^3R^4$, -COOH,

$-OCONR^3R^4$, $-NR^6OCOR^3$, $-NR^6CONR^3R^4$,$-NR^6SO_2NR^3R^4$, $-NR^6SO_2R^4$, optionally substituted ($C_1$-$C_6$)alkyl, optionally substituted ($C_2$-$C_6$)alkenyl, optionally substituted ($C_2$-$C_6$)alkynyl, optionally substituted ($C_1$-$C_6$)alkoxy, optionally substituted ($C_3$-$C_8$)cycloalkyl, optionally substituted ($C_3$-$C_8$)cycloalkyl ($C_1$-$C_6$)alkyl, optionally substituted ($C_3$-$C_8$)cycloalkyl-CO-, optionally substituted ($C_3$-$C_8$)cycloalkyl-$SO_2$-, optionally substituted aryl ($C_1$-$C_6$)alkoxy, optionally substituted ($C_3$-$C_8$)cycloalkyl ($C_1$-$C_6$)alkoxy, optionally substituted heterocyclyl-CO-, optionally substituted heterocyclyl, optionally substituted ($C_1$-$C_6$)alkyl-$SO_2$-, $-NR^6SO_2$-optionally substituted ($C_1$-$C_6$)alkyl, - $NR^6SO_2$-optionally substituted heterocyclo, optionally substituted ($C_1$-$C_6$)alkyl-$NR^6SO_2$- or optionally substituted heterocyclo-$NR^6SO_2$-;

$R^2$ is hydrogen, halogen, -CN, OH, optionally substituted ($C_1$-$C_6$)alkyl, optionally substituted ($C_3$-$C_8$)cycloalkyl, optionally substituted ($C_1$-$C_6$) alkoxy, optionally substituted aryl, optionally substituted heteroaryl or optionally substituted heterocyclo;

$R^3$ is hydrogen, optionally substituted ($C_1$-$C_6$)alkyl, optionally substituted ($C_3$-$C_8$)cycloalkyl, optionally substituted ($C_2$-$C_6$)alkenyl, optionally substituted ($C_2$-$C_6$)alkynyl, cyano($C_1$-$C_6$)alkyl, hydroxy($C_1$-$C_6$)alkyl, optionally substituted aryl, optionally substituted aryl($C_1$-$C_6$)alkyl, optionally substituted aryloxy($C_1$-$C_6$)alkyl, optionally substituted ($C_1$-$C_6$)alkyl-$SO_2$-, optionally substituted heterocyclyl, optionally substituted heterocyclyl($C_1$-$C_6$)alkyl, optionally substituted heteroaryl or optionally substituted heteroaryl($C_1$-$C_6$)alkyl,

$R^4$ is hydrogen, optionally substituted ($C_1$-$C_6$)alkyl or optionally substituted ($C_3$-$C_8$)cycloalkyl;

or $R^3$ and $R^4$ may be taken together with the nitrogen atom to which they are attached to form an optionally substituted ($C_4$-$C_8$) heteroaryl or ($C_4$-$C_8$) heterocyclic ring;

$R^6$ is hydrogen or optionally substituted ($C_1$-$C_6$)alkyl;

and/or a pharmaceutically acceptable salt, tautomer or stereoisomer thereof.

In another aspect of the disclosure, there is provided a compound selected from the exemplified examples within the scope of the first aspect, or a pharmaceutically acceptable salt, tautomer or stereoisomer thereof.

In another aspect of the disclosure, there is provided a compound selected from any subset list of compounds within the scope of any of the above aspects.

In another aspect of the disclosure, there is provided a compound selected from the following

2-[3-(dimethyl-1H-1,2,3-tnazol-5-yl)-5-(1,1,1,7,7,7-hexafluoroheptan-4-yl)-5H-pyrido[3,2-b]indol-7-yl]propan-2-ol,
2-[3-(dimethyl-1,2-oxazol-4-yl)-5-[(S)-oxan-4-yl(phenyl)methyl]-5H-pyrido[3,2-b]indol-7-yl]propan-2-ol,
2-[3-(dimethyl-1H-1,2,3-triazol-5-yl)-5-[(1S)-4,4,4-trifluoro-1-phenylbutyl]-5H-pyrido[3,2-b]indol-7-yl]propan-2-ol,
2-[3-(dimethyl-1H-1,2,3-triazol-5-yl)-5-[(S)-oxan-4-yl(phenyl)methyl]-5H-pyrido[3,2-b]indol-7-yl]propan-2-ol,
2-[3-(dimethyl-1,2-oxazol-4-yl)-5-[(S)-(4-fluorophenyl)(oxan-4-yl)methyl]-5H-pyrido[3,2-b]indol-7-yl]propan-2-ol,
2-[3-(dimethyl-1H-1,2,3-triazol-5-yl)-5-[(4-fluorophenyl)(oxan-4-yl)methyl]-5H-pyrido[3,2-b]indol-7-yl]propan-2-ol,
2-[3-(dimethyl-1H-1,2,3-triazol-5-yl)-6-fluoro-5-[(S)-oxan-4-yl(phenyl)methyl]-5H-pyrido[3,2-b]indol-7-yl]propan-2-ol,
2- {3-[4-(hydroxymethyl)-1-methyl- 1H-1,2,3-triazol-5-yl]-5-[(S)-oxan-4-yl(phenyl)methyl]-5H-pyrido[3,2-b]indol-7-yl}propan-2-ol,
5-{7-methanesulfonyl-5-[(S)-oxan-4-yl(phenyl)methyl]-5H-pyrido[3,2-b]indo1-3-yl}-4-($^2H_3$)methyl-1-methyl-1H-1H-1,2,3-triazole,
5-{5-[(S)-(4-fluorophenyl)(oxan-4-yl)methyl]-7-methanesulfonyl-5H-pyrido[3,2-b]indol-3-yl}-4-($^2H_3$)methyl-1-methyl- 1H-1H-1,2,3-triazole,
2-{5-[(S)-(4-fluorophenyl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
(1R)-1-cyclopropyl-1-[3-(dimethyl-1H-1,2,3-triazol-5-yl)-6-fluoro-5-[(S)-oxan-4-yl(phenyl)methyl]-5H-pyrido[3,2-b]indol-7-yl]ethan-1-ol,
2-{3-[5-($^2H_3$)methyl-3-methyl-1,2-oxazol-4-yl]-5-[(S)-oxan-4-yl(phenyl)methyl]-5H-pyrido[3,2-b]indol-7-yl}propan-2-ol
2-{3-[4-($^2H_3$)methoxy-1-methyl-1H-1,2,3-triazol-5-yl]-5-[(S)-oxan-4-yl(phenyl)methyl]-5H-pyrido[3,2-b]indol-7-yl}propan-2-ol,
2-[3-(4-methoxy-1-methyl-1H-1,2,3-triazol-5-yl)-5-[(S)-oxan-4-yl(phenyl)methyl]-5H-pyrido[3,2-b]indol-7-yl]propan-2-ol,
(1R)-1-cyclopropyl-1-[3-(dimethyl-1H-1,2,3-triazol-5-yl)-6-fluoro-5-[(S)-(2-fluorophenyl)(oxan-4-yl)methyl]-5H-pyrido[3,2-b]indol-7-yl]ethan-1-ol,
2-{6-fluoro-5-[(S)-(2-fluorophenyl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
(1S)-1-cyclopropyl-1-{6-fluoro-3-[4-($^2H_3$)methyl-1-methyl-1H-1,2,3-triazol-5-yl]-5-[(S)-oxan-4-yl(phenyl)methyl]-5H-pyrido[3,2-b]indol-7-yl}ethan-1-ol,

(1R)-1-cyclopropyl-1-{6-fluoro-3-[4-($^2H_3$)methyl-1-methyl-1H-1,2,3-triazol-5-yl]-5-[(S)-oxan-4-yl(phenyl)methyl]-5H-pyrido[3,2-b]indol-7-yl}ethan-1-ol,
2-{5-[(3-fluoropyridin-2-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{8-fluoro-5-[(S)-(2-fluorophenyl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{6-fluoro-3-[4-($^2H_3$)methyl-1-methyl-1H-1,2,3-triazol-5-yl]-5-[(S)-oxan-4-yl(phenyl)methyl]-5H-pyrido[3,2-b]indol-7-yl}propan-2-ol,
2-{5-[(S)-(4,4-difluorocyclohexyl)(phenyl)methyl]-3-[4-($^2H_3$)methyl-1-methyl-1H-1,2,3-triazol-5-yl]-5H-pyrido[3,2-b]indol-7-yl}propan-2-ol,
2-{8-fluoro-3-[4-($^2H_3$)methyl-1-methyl-1H-1,2,3-triazol-5-yl]-5-[(S)-oxan-4-yl(phenyl)methyl]-5H-pyrido[3,2-b]indol-7-yl}propan-2-ol,
(1R)-1-cyclopropyl-1-{6-fluoro-5-[(S)-(2-fluorophenyl)(oxan-4-yl)methyl]-3-[4-($^2H_3$)methyl-1-methyl-1H-1,2,3-triazol-5-yl]-5H-pyrido[3,2-b]indol-7-yl}ethan-1-ol,
2-{6-fluoro-5-[(5-methyl-1,2-oxazol-3-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{6-chloro-3-[4-($^2H_3$)methyl-1-methyl-1H-1,2,3-triazol-5-yl]-5-[(S)-oxan-4-yl(phenyl)methyl]-5H-pyrido[3,2-b]indol-7-yl}propan-2-ol,
2-[(R)-(4,4-difluorocyclohexyl)({9-fluoro-7-methanesulfonyl-3-[4-($^2H_3$)methyl-1-methyl-1H-1,2,3-triazol-5-yl]-5H-pyrido[3,2-b]indol-5-yl})methyl]-3-fluoropyridine,
(1S)-1-cyclopropyl-1-{6-fluoro-5-[(S)-(2-fluorophenyl)(oxan-4-yl)methyl]-3-[4-($^2H_3$)methyl-1-methyl-1H-1,2,3-triazol-5-yl]-5H-pyrido[3,2-b]indol-7-yl}ethan-1-ol,
2-{8-fluoro-5-[(5-methyl-1,2-oxazol-3-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{5-[(5-chloropyridin-2-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{5-[(3-chloropyridin-2-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{5-[(4-chloropyridin-2-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{5-[(4,4-difluorocyclohexyl)(3-fluoropyridin-2-yl)methyl]-6-fluoro-3-[4-($^2H_3$)methyl-1-methyl-1H-1,2,3-triazol-5-yl]-5H-pyrido[3,2-b]indol-7-yl}propan-2-ol,
2-{6-fluoro-5-[(3-fluoropyridin-2-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{8-fluoro-5-[(3-fluoropyridin-2-yl)(oxan-4-yl)methyl]-3-[4-($^2H_3$)methyl-1-methyl-1H-1,2,3-triazol-5-yl]-5H-pyrido[3,2-b]indol-7-yl}propan-2-ol,
2-{3-[4-($^2H_3$)methoxy-1-($^2H_3$)methyl-1H-1,2,3-triazol-5-yl]-5-[(S)-oxan-4-yl(phenyl)methyl]-5H-pyrido[3,2-b]indol-7-yl}propan-2-ol,
2-{3-[4-methoxy-1-($^2H_3$)methyl-1H-1,2,3-triazol-5-yl]-5-[(S)-oxan-4-yl(phenyl)methyl]-5H-pyrido[3,2-b]indol-7-yl}propan-2-ol,
5-{7-methanesulfonyl-5-[(S)-oxan-4-yl(phenyl)methyl]-5H-pyrido[3,2-b]indol-3-yl}-4-methoxy-1-methyl-1H-1,2,3-triazole,
2-[3-(dimethyl-1H-1,2,3-triazol-5-yl)-6-fluoro-5-[(S)-oxan-4-yl(phenyl)methyl]-5H-pyrido[3,2-b]indol-7-yl]propan-2-amine,
N-{2-[3-(dimethyl-1H-1,2,3-triazol-5-yl)-6-fluoro-5-[(S)-oxan-4-yl(phenyl)methyl]-5H-pyrido[3,2-b]indol-7-yl]propan-2-yl}acetamide,
N-{2-[3-(dimethyl-1H-1,2,3-triazol-5-yl)-6-fluoro-5-[(S)-oxan-4-yl(phenyl)methyl]-5H-pyrido[3,2-b]indol-7-yl]propan-2-yl}methanesulfonamide,
methyl N-{2-[3-(dimethyl-1H-1,2,3-triazol-5-yl)-6-fluoro-5-[(S)-oxan-4-yl(phenyl)methyl]-5H-pyrido[3,2-b]indol-7-yl]propan-2-yl}carbamate,
5-{6-methanesulfonyl-5-[(S)-oxan-4-yl(phenyl)methyl]-5H-pyrido[3,2-b]indol-3-yl}-1,4-dimethyl-1H-1,2,3-triazole,
5-{9-fluoro-6-methanesulfonyl-5-[(S)-oxan-4-yl(phenyl)methyl]-5H-pyrido[3,2-b]indol-3-yl}-4-($^2H_3$)methyl-1-methyl-1H-1,2,3-triazole,
5-{6-methanesulfonyl-9-methoxy-5-[(S)-oxan-4-yl(phenyl)methyl]-5H-pyrido[3,2-b]indol-3-yl}-1,4-dimethyl-1H-1,2,3-triazole,
N-[3-(dimethyl-1H-1,2,3-triazol-5-yl)-6-methanesulfonyl-5-[(S)-oxan-4-yl(phenyl)methyl]-5H-pyrido[3,2-b]indol-9-yl]cyclopropanesulfonamide,
5-{9-methanesulfonyl-5-[(S)-oxan-4-yl(phenyl)methyl]-5H-pyrido[3,2-b]indol-3-yl}-1,4-dimethyl-1H-1,2,3-triazole,

5-{9-methanesulfonyl-6,7-dimethoxy-5-[(S)-oxan-4-yl(phenyl)methyl]-5H-pyrido[3,2-b]indol-3-yl}-4-($^2H_3$)methyl-1-methyl-1H-1,2,3-triazole, and

2-{3-[4-($^2H_3$)methyl-1-methyl-1H-1,2,3-triazol-5-yl]-5-[(S)-oxan-4-yl(phenyl)methyl]-5H-pyrido[3,2-b]indol-7-yl}propan-2-ol

and/or a pharmaceutically acceptable salt, tautomer or stereoisomer thereof.

One embodiment of the disclosure provides compounds wherein A is optionally substituted heterocyclo or optionally substituted heteroaryl, wherein the substituents are one or more R;

Another embodiment of the disclosure provides compounds wherein A is

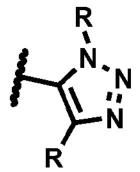
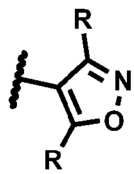
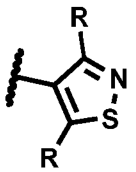
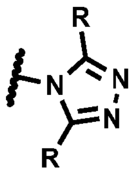
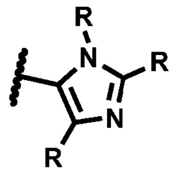
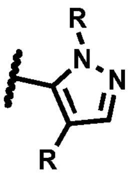
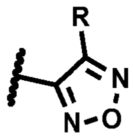
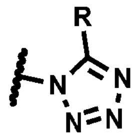
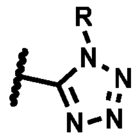
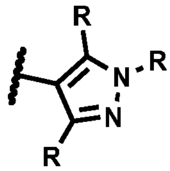
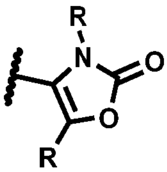
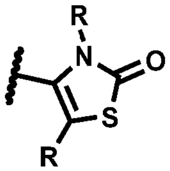
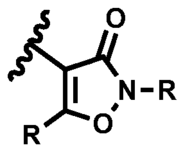
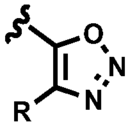
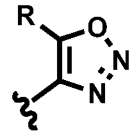
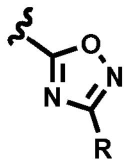
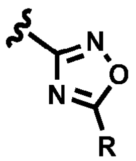
;

and R is independently one or more hydrogen, $CD_3$, $OCD_3$, $CF_3$, $CHF_2$ or ($C_1$-$C_3$)alkyl.

Another embodiment of the disclosure provides compounds wherein A is

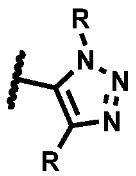
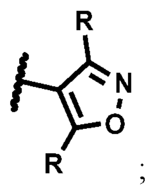
;

and R is independently one or more hydrogen, $CD_3$, $OCD_3$, $CF_3$, $CHF_2$ or ($C_1$-$C_3$)alkyl.

In another embodiment, the compounds of the invention have $IC_{50}$ values ≤ 250 nM.

In another embodiment, the compounds of the invention have $IC_{50}$ values ≤25 nM

In another embodiment, the compounds of the invention have $IC_{50}$ values ≤ 5 nM.

OTHER EMBODIMENTS OF THE INVENTION

In another embodiment, the invention provides a pharmaceutical composition, comprising a pharmaceutically acceptable carrier and a therapeutically effective amount of at least one of the compounds of the invention or a stereoisomer, a tautomer, a pharmaceutically acceptable salt, or a solvate thereof.

In another embodiment, the disclosure provides a process for making a compound of the invention or a stereoisomer, a tautomer, a pharmaceutically acceptable salt, or a solvate thereof.

In another embodiment, the disclosure provides a method for inhibiting activity of a bromodomain-containing protein mediated disorder in a patient in need thereof comprising the step of administering to said patient at least one compound of the invention.

In another embodiment, the invention relates to one or more compounds of the invention or pharmaceutically acceptable salts thereof, alone, or, optionally, in combination with another compound of the invention and/or at least one other type of therapeutic agent for use in treatingcancer in a patient, wherein the cancer is small cell lung cancer, non-small cell lung cancer, colorectal cancer, multiple myeloma or AML, optionally comprising administering to a patient in need of such treatment and/or prophylaxis.

In another embodiment, the invention relates to compounds of the invention or pharmaceutically acceptable salts thereof for use in treating of cancer in a patient, wherein the cancer is small cell lung cancer, non-small cell lung cancer, colorectal cancer, multiple myeloma or AML.

In another embodiment, the disclosure provides a compound of the present invention for use in therapy.

In another embodiment, the invention provides a combined preparation of a compound of the present invention and additional therapeutic agent(s) for simultaneous, separate or sequential use in therapy.

In another embodiment, the disclosure provides a method of inhibiting a bromodomain-containing protein comprising contacting said protein with any exemplified compound or a pharmaceutically acceptable salt or composition thereof.

## THERAPEUTIC APPLICATIONS

The compounds of formula (I) of the invention are bromodomain inhibitors and have potential utility in the treatment of diseases and conditions for which a bromodomain inhibitor is indicated.

In one embodiment there is provided a method for the treatment of a disease or condition, for which a bromodomain inhibitor is indicated, in a subject in need thereof which comprises administering a therapeutically effective amount of compound of formula (I) or a pharmaceutically acceptable salt thereof.

In another embodiment there is provided a method for treatment of a chronic autoimmune and/or inflammatory condition, in a subject in need thereof which comprises administering a therapeutically effective amount of one or more compounds of formula (I) or a pharmaceutically acceptable salt thereof.

In a further embodiment there is provided a method for treatment of cancer in a subject in need thereof which comprises administering a therapeutically effective amount of one or more compounds of formula (I) or a pharmaceutically acceptable salt thereof.

In one embodiment the subject in need thereof is a mammal, particularly a human.

Bromodomain inhibitors are believed to be useful in the treatment of a variety of diseases or conditions related to systemic or tissue inflammation, inflammatory responses to infection or hypoxia, cellular activation and proliferation, lipid metabolism, fibrosis and in the prevention and treatment of viral infections.

Bromodomain inhibitors may be useful in the treatment of a wide variety of chronic autoimmune and inflammatory conditions such as rheumatoid arthritis, osteoarthritis, acute gout, psoriasis, systemic lupus erythematosus, multiple sclerosis, inflammatory bowel disease (Crohn's disease and Ulcerative colitis), asthma, chronic obstructive airways disease, pneumonitis, myocarditis, pericarditis, myositis, eczema, dermatitis, alopecia, vitiligo, bullous skin diseases, nephritis, vasculitis, atherosclerosis, Alzheimer's disease, depression, retinitis, uveitis, scleritis, hepatitis, pancreatitis, primary biliary cirrhosis, sclerosing cholangitis, Addison's disease, hypophysitis, thyroiditis, type I diabetes and acute rejection of transplanted organs.

Bromodomain inhibitors may be useful in the treatment of a wide variety of acute inflammatory conditions such as acute gout, giant cell arteritis, nephritis including lupus nephritis, vasculitis with organ involvement such as glomerulonephritis, vasculitis including giant cell arteritis, Wegener's granulomatosis, Polyarteritis nodosa, Behcet's disease, Kawasaki disease, Takayasu's Arteritis and acute rejection of transplanted organs.

Bromodomain inhibitors may be useful in the prevention or treatment of diseases or conditions which involve inflammatory responses to infections with bacteria, viruses, fungi, parasites or their toxins, such as sepsis, sepsis syndrome, septic shock, endotoxaemia, systemic inflammatory response syndrome (SIRS), multi-organ dysfunction syndrome, toxic shock syndrome, acute lung injury, ARDS (adult respiratory distress syndrome), acute renal failure, fulminant hepatitis, burns, acute pancreatitis, post-surgical syndromes, sarcoidosis, Herxheimer reactions, encephalitis, myelitis, meningitis, malaria, SIRS associated with viral infections such as influenza, herpes zoster, herpes simplex and coronavirus.

Bromodomain inhibitors may be useful in the prevention or treatment of conditions associated with ischaemia-reperfusion injury such as myocardial infarction, cerebrovascular ischaemia (stroke), acute coronary syndromes, renal reperfusion injury, organ transplantation, coronary artery bypass grafting, cardio-pulmonary bypass procedures and

pulmonary, renal, hepatic, gastro-intestinal or peripheral limb embolism.

Bromodomain inhibitors may be useful in the treatment of disorders of lipid metabolism via the regulation of APO-A1 such as hypercholesterolemia, atherosclerosis and Alzheimer's disease.

Bromodomain inhibitors may be useful in the treatment of fibrotic conditions such as idiopathic pulmonary fibrosis, renal fibrosis, post-operative stricture, keloid formation, scleroderma and cardiac fibrosis.

Bromodomain inhibitors may be useful in the prevention and treatment of viral infections such as herpes virus, human papilloma virus, adenovirus, poxvirus and other DNA viruses.

Bromodomain inhibitors may also be useful in the treatment of cancer, including hematological, epithelial including lung, breast and colon carcinomas, midline carcinomas, mesenchymal, hepatic, renal and neurological tumours.

In one embodiment the disease or condition for which a bromodomain inhibitor is indicated is selected from diseases associated with systemic inflammatory response syndrome, such as sepsis, burns, pancreatitis, major trauma, hemorrhage and ischemia. In this embodiment, the bromodomain inhibitor would be administered at the point of diagnosis to reduce the incidence of SIRS, the onset of shock, multi-organ dysfunction syndrome, which includes the onset of acute lung injury, ARDS, acute renal, hepatic, cardiac and gastro-intestinal injury and mortality. In another embodiment the bromodomain inhibitor would be administered prior to surgical or other procedures associated with a high risk of sepsis, hemorrhage, extensive tissue damage, SIRS or MODS (multiple organ dysfunction syndrome). In a particular embodiment the disease or condition for which a bromodomain inhibitor is indicated is sepsis, sepsis syndrome, septic shock and endotoxemia. In another embodiment, the bromodomain inhibitor is indicated for the treatment of acute or acute on chronic pancreatitis. In another embodiment the bromodomain inhibitor is indicated for the treatment of burns.

In one embodiment the disease or condition for which a bromodomain inhibitor is indicated is selected from herpes simplex infections and reactivations, cold sores, herpes zoster infections and reactivations, chickenpox, shingles, human papilloma virus, cervical neoplasia, adenovirus infections, including acute respiratory disease, and poxvirus infections such as cowpox and smallpox and African swine fever virus.

The term "diseases or conditions for which a bromodomain inhibitor is indicated" is intended to include any of or all of the above disease states.

In one embodiment, there is provided a method for inhibiting a bromodomain which comprises contacting the bromodomain with a compound of formula (1) or a pharmaceutically acceptable salt thereof.

While it is possible that for use in therapy, a compound of formula (I) as well as pharmaceutically acceptable salts thereof may be administered as the compound itself, it is more commonly presented as a pharmaceutical composition.

Pharmaceutical compositions may be presented in unit dose forms containing a predetermined amount of active ingredient pep unit dose. Preferred unit dosage compositions are those containing a daily dose or sub-dose, or an appropriate fraction thereof, of an active ingredient. Such unit doses may therefore be administered more than once a day. Preferred unit dosage compositions are those containing a daily dose or sub-dose (for administration more than once a day), as herein above recited, or an appropriate fraction thereof, of an active ingredient.

Types of cancers that may be treated with the compounds of this invention include, but are not limited to, brain cancers, skin cancers, bladder cancers, ovarian cancers, breast cancers, gastric cancers, pancreatic cancers, prostate cancers, colon cancers, blood cancers, lung cancers and bone cancers. Examples of such cancer types include neuroblastoma, intestine carcinoma such as rectum carcinoma, colon carcinoma, familiar adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroid carcinoma, papillary thyroid carcinoma, renal carcinoma, kidney parenchymal carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, diffuse large B-cell lymphoma (DLBCL), hepatocellular carcinoma, gall bladder carcinoma, bronchial carcinoma, small cell lung carcinoma, non-small cell lung carcinoma, multiple myeloma, basalioma, teratoma, retinoblastoma, choroid melanoma, seminoma, rhabdomyosarcoma, craniopharyngioma, osteosarcoma, chondrosarcoma, myosarcoma, liposarcoma, fibrosarcoma, Ewing sarcoma and plasmocytoma.

In addition to apoptosis defects found in tumors, defects in the ability to eliminate self- reactive cells of the immune system due to apoptosis resistance are considered to play a key role in the pathogenesis of autoimmune diseases. Autoimmune diseases are characterized in that the cells of the immune system produce antibodies against its own organs and molecules or directly attack tissues resulting in the destruction of the latter. A failure of those self-reactive cells to undergo apoptosis leads to the manifestation of the disease. Defects in apoptosis regulation have been identified in autoimmune diseases such as systemic lupus erythematosus or rheumatoid arthritis.

Thus, according to another embodiment, the disclosure provides a method of treating an autoimmune disease by providing to a patient in need thereof a compound or composition of the present disclosure. Examples of such autoimmune diseases

include, but are not limited to, collagen diseases such as rheumatoid arthritis, systemic lupus erythematosus. Sharp's syndrome, CREST syndrome (calcinosis, Raynaud's syndrome, esophageal dysmotility, telangiectasia), dermatomyositis, vasculitis (Morbus Wegener's) and Sjogren's syndrome, renal diseases such as Goodpasture's syndrome, rapidly-progressing glomerulonephritis and membrano-proliferative glomerulonephritis type II, endocrine diseases such as type-I diabetes, autoimmune polyendocrinopathy-candidiasis-ectodermal dystrophy (APECED), autoimmune parathyroidism, pernicious anemia, gonad insufficiency, idiopathic Morbus Addison's, hyperthyreosis, Hashimoto's thyroiditis and primary myxedema, skin diseases such as pemphigus vulgaris, bullous pemphigoid, herpes gestationis, epidermolysis bullosa and erythema multiforme major, liver diseases such as primary biliary cirrhosis, autoimmune cholangitis, autoimmune hepatitis type-1, autoimmune hepatitis type-2, primary sclerosing cholangitis, neuronal diseases such as multiple sclerosis, myasthenia gravis, myasthenic Lambert-Eaton syndrome, acquired neuromyotomy, Guillain-Barre syndrome (Muller-Fischer syndrome), stiff-man syndrome, cerebellar degeneration, ataxia, opsoclonus, sensoric neuropathy and achalasia, blood diseases such as autoimmune hemolytic anemia, idiopathic thrombocytopenic purpura (Morbus Werlhof), infectious diseases with associated autoimmune reactions such as AIDS, malaria and Chagas disease.

Compounds of the invention are useful for the treatment of certain types of cancer by themselves or in combination or co-administration with other therapeutic agents or radiation therapy. Thus, in one embodiment, the compounds of the invention are co-administered with radiation therapy or a second therapeutic agent with cytostatic or antineoplastic activity. Suitable cytostatic chemotherapy compounds include, but are not limited to (i) antimetabolites; (ii) DNA-fragmenting agents, (iii) DNA-crosslinking agents, (iv) intercalating agents (v) protein synthesis inhibitors, (vi) topoisomerase I poisons, such as camptothecin or topotecan; (vii) topoisomerase II poisons, (viii) microtubule-directed agents, (ix) kinase inhibitors (x) miscellaneous investigational agents (xi) hormones and (xii) hormone antagonists. It is contemplated that compounds of the invention may be useful in combination with any known agents falling into the above 12 classes as well as any future agents that are currently in development. In particular, it is contemplated that compounds of the invention may be useful in combination with current Standards of Care as well as any that evolve over the foreseeable future. Specific dosages and dosing regimens would be based on physicians' evolving knowledge and the general skill in the art.

Further, the invention relates to compounds for use in methods of treatment wherein compounds of the invention are administered with one or more immuno-oncology agents. The immuno-oncology agents used herein, also known as cancer immunotherapies, are effective to enhance, stimulate, and/or up-regulate immune responses in a subject. In one aspect, the administration of a compound of the invention with an immuno-oncology agent has a synergic effect in inhibiting tumor growth.

In one aspect, the compound(s) of the invention are sequentially administered prior to administration of the immuno-oncology agent. In another aspect, compound(s) of the invention are administered concurrently with the immunology-oncology agent. In yet another aspect, compound(s) of the invention are sequentially administered after administration of the immuno-oncology agent.

In another aspect, compounds of the invention may be co-formulated with an immuno-oncology agent.

Immuno-oncology agents include, for example, a small molecule drug, antibody, or other biologic or small molecule. Examples of biologic immuno-oncology agents include, but are not limited to, cancer vaccines, antibodies, and cytokines. In one aspect, the antibody is a monoclonal antibody. In another aspect, the monoclonal antibody is humanized or human.

In one aspect, the immuno-oncology agent is (i) an agonist of a stimulatory (including a co-stimulatory) receptor or (ii) an antagonist of an inhibitory (including a co-inhibitory) signal on T cells, both of which result in amplifying antigen-specific T cell responses (often referred to as immune checkpoint regulators).

Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAILR2/DR5, TRAILR3, TRAILR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDAR, EDA1, XEDAR, EDA2, TNFR1, Lymphotoxin α/TNFβ, TNFR2, TNFα, LTβR, Lymphotoxin α 1β2, FAS, FASL, RELT, DR6, TROY, NGFR.

In another aspect, the immuno-oncology agent is a cytokine that inhibits T cell activation (e.g., IL-6, IL-10, TGF-β, VEGF, and other immunosuppressive cytokines) or a cytokine that stimulates T cell activation, for stimulating an immune response.

In one aspect, T cell responses can be stimulated by a combination of a compound of the invention and one or more of (i) an antagonist of a protein that inhibits T cell activation (e.g., immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4, and (ii) an agonist of a protein that stimulates T cell activation such

as B7-1, B7-2, CD28, 4-1BB (CD137), 4-1BBL, ICOS, ICOS-L, OX40, OX40L, GITR, GITRL, CD70, CD27, CD40, DR3 and CD28H.

Other agents that can be combined with compounds of the invention for the treatment of cancer include antagonists of inhibitory receptors on NK cells or agonists of activating receptors on NK cells. For example, compounds of the invention can be combined with antagonists of KIR, such as lirilumab.

Yet other agents for combination therapies include agents that inhibit or deplete macrophages or monocytes, including but not limited to CSF-1R antagonists such as CSF-1R antagonist antibodies including RG7155 (WO11/70024, WO11/107553, WO11/131407, WO13/87699, WO13/119716, WO13/132044) or FPA-008 (WO11/140249; WO13169264; WO14/036357).

In another aspect, compounds of the invention can be used with one or more of agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (e.g., block inhibitory receptor engagement (e.g., PD-L1/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell anergy or exhaustion) and agents that trigger innate immune activation and/or inflammation at tumor sites.

In one aspect, the immuno-oncology agent is a CTLA-4 antagonist, such as an antagonistic CTLA-4 antibody. Suitable CTLA-4 antibodies include, for example, YERVOY (ipilimumab) or tremelimumab.

In another aspect, the immuno-oncology agent is a PD-1 antagonist, such as an antagonistic PD-1 antibody. Suitable PD-1 antibodies include, for example, OPDIVO (nivolumab), KEYTRUDA (pembrolizumab), or MEDI-0680 (AMP-514; WO2012/145493). The immuno-oncology agent may also include pidilizumab (CT-011), though its specificity for PD-1 binding has been questioned. Another approach to target the PD-1 receptor is the recombinant protein composed of the extracellular domain of PD-L2 (B7-DC) fused to the Fc portion of IgG1, called AMP-224

In another aspect, the immuno-oncology agent is a PD-L1 antagonist, such as an antagonistic PD-L1 antibody. Suitable PD-L1 antibodies include, for example, MPDL3280A (RG7446; WO2010/077634), durvalumab (MEDI4736), BMS-936559 (WO2007/005874), and MSB0010718C (WO2013/79174).

In another aspect, the immuno-oncology agent is a LAG-3 antagonist, such as an antagonistic LAG-3 antibody. Suitable LAG3 antibodies include, for example, BMS-986016 (WO10/19570, WO14/08218), or IMP-731 or IMP-321 (WO08/132601, WO09/44273).

In another aspect, the immuno-oncology agent is a CD137 (4-1BB) agonist, such as an agonistic CD137 antibody. Suitable CD137 antibodies include, for example, urelumab and PF-05082566 (WO12/32433).

In another aspect, the immuno-oncology agent is a GITR agonist, such as an agonistic GITR antibody. Suitable GITR antibodies include, for example, BMS-986153, BMS-986156, TRX-518 (WO06/105021, WO09/009116) and MK-4166 (WO11/028683).

In another aspect, the immuno-oncology agent is an IDO antagonist. Suitable IDO antagonists include, for example, INCB-024360 (WO2006/122150, WO07/75598, WO08/36653, WO08/36642), indoximod, or NLG-919 (WO09/73620, WO09/1156652, WO11/56652, WO12/142237).

In another aspect, the immuno-oncology agent is an OX40 agonist, such as an agonistic OX40 antibody. Suitable OX40 antibodies include, for example, MEDI-6383 or MEDI-6469.

In another aspect, the immuno-oncology agent is an OX40L antagonist, such as an antagonistic OX40 antibody. Suitable OX40L antagonists include, for example, RG-7888 (WO06/029879).

In another aspect, the immuno-oncology agent is a CD40 agonist, such as an agonistic CD40 antibody. In yet another embodiment, the immuno-oncology agent is a CD40 antagonist, such as an antagonistic CD40 antibody. Suitable CD40 antibodies include, for example, lucatumumab or dacetuzumab.

In another aspect, the immuno-oncology agent is a CD27 agonist, such as an agonistic CD27 antibody. Suitable CD27 antibodies include, for example, varlilumab.

In another aspect, the immuno-oncology agent is MGA271 (to B7H3) (WO11/109400).

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy

also can embrace the administration of the therapeutic agents as described above in further combination with other biologically active ingredients and non-drug therapies (*e.g.,* surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

This invention encompasses all combinations of preferred aspects of the invention noted herein. It is understood that any and all embodiments of the present invention may be taken in conjunction with any other embodiment or embodiments to describe additional embodiments. It is also understood that each individual element of the embodiments is its own independent embodiment. Furthermore, any element of an embodiment is meant to be combined with any and all other elements from any embodiment to describe an additional embodiment.

## PHARMACEUTICAL COMPOSITIONS AND DOSING

The invention also provides pharmaceutically acceptable compositions which comprise a therapeutically effective amount of the compound of Example 1 or a pharmaceutically acceptable salt thereof, formulated together with one or more pharmaceutically acceptable carriers (additives) and/or diluents, and optionally, one or more additional therapeutic agents described above. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained release formulation; (3) topical application, for example, as a cream, ointment, or a controlled release patch or spray applied to the skin; (4) intravaginally or intrarectally, for example, as a pessary, cream or foam; (5) sublingually; (6) ocularly; (7) transdermally; or (8) nasally.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the patient being treated and the particular mode of administration. The amount of active ingredient which can be combined with a carrier material to produce a single dosage form will generally be that amount of the compound which produces a therapeutic effect. Generally, out of one hundred percent, this amount will range from about 0.1 percent to about ninety-nine percent

of active ingredient, preferably from about 5 percent to about 70 percent, most preferably from about 10 percent to about 30 percent.

In certain embodiments, a formulation of the present invention comprises an excipient selected from the group consisting of cyclodextrins, celluloses, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules, troches and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide,

bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically acceptable sterile isotonic aqueous or non-aqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsuled matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, oral, intravenous, intracerebroventricular and subcutaneous doses of the compounds of this invention for a patient will range from about 0.01 to about 50 mg per kilogram of body weight per day.

If desired, the effective daily dose of the active compound may be administered as two, three, four, five, six or more sub-doses administered separately at appropriate intervals throughout the day, optionally, in unit dosage forms. In certain aspects of the invention, dosing is one administration per day.

While it is possible for a compound of the present invention to be administered alone, it is preferable to administer the compound as a pharmaceutical formulation (composition).

## DEFINITIONS

Unless specifically stated otherwise herein, references made in the singular may also include the plural. For example, "a" and "an" may refer to either one, or one or more.

Unless otherwise indicated, any heteroatom with unsatisfied valences is assumed to have hydrogen atoms sufficient to satisfy the valences.

Throughout the specification and the appended claims, a given chemical formula or name shall encompass all stereo and optical isomers and racemates thereof where such isomers exist. Unless otherwise indicated, all chiral (enantiomeric and diastereomeric) and racemic forms are within the scope of the invention. Many geometric isomers of C=C double bonds, C=N double bonds, ring systems, and the like can also be present in the compounds, and all such stable isomers are contemplated in the present invention. Cis- and trans- (or *E*- and *Z*-) geometric isomers of the compounds of the present invention are described and may be isolated as a mixture of isomers or as separated isomeric forms. The present compounds can be isolated in optically active or racemic forms. Optically active forms may be prepared by resolution of racemic forms or by synthesis from optically active starting materials. All processes used to prepare compounds of the present invention and intermediates made therein are considered to be part of the present disclosure. When enantiomeric or diastereomeric products are prepared, they may be separated by conventional methods, for example, by chromatography or fractional crystallization. Depending on the process conditions the end products of the present invention are obtained either in free (neutral) or salt form. Both the free form and the salts of these end products are within the scope of the invention. If so desired, one form of a compound may be converted into another form. A free base or acid may be converted into a salt; a salt may be converted into the free compound or another salt; a mixture of isomeric compounds of the present invention may be separated into the individual isomers. Compounds of the present invention, free form and salts thereof, may exist in multiple tautomeric forms, in which hydrogen atoms are transposed to other parts of the molecules and the chemical bonds between the atoms of the molecules are consequently rearranged. It should be understood that all tautomeric forms, insofar as they may exist, are included within the invention.

When a substituent is noted as "optionally substituted", the substituents are selected from, for example, substituents such as alkyl, cycloalkyl, aryl, heterocyclo, halo, hydroxy, alkoxy, oxo, alkanoyl, aryloxy, alkanoyloxy, amino, alkylamino, arylamino, arylalkylamino, disubstituted amines in which the 2 amino substituents are selected from alkyl, aryl or arylalkyl; alkanoylamino, aroylamino, aralkanoylamino, substituted alkanoylamino, substituted arylamino, substituted aralkanoylamino, thiol, alkylthio, arylthio, arylalkylthio, alkylthiono, arylthiono, arylalkylthiono, alkylsulfonyl, arylsulfonyl, arylalkylsulfonyl, sulfonamido, e.g. $-SO_2NH_2$, substituted sulfonamido, nitro, cyano, carboxy, carbamyl, e.g. $-CONH_2$, substituted carbamyl e.g. -CONHalkyl,-CONHaryl, -CONHarylalkyl or cases where there are two substituents on the nitrogen selected from alkyl, aryl or arylalkyl; alkoxycarbonyl, aryl, substituted aryl, guanidino, heterocyclyl, e.g., indolyl, imidazolyl, furyl, thienyl, thiazolyl, pyrrolidyl, pyridyl, pyrimidyl, pyrrolidinyl, piperidinyl, morpholinyl, piperazinyl, homopiperazinyl and the like, and substituted heterocyclyl, unless otherwise defined.

For purposes of clarity and in accordance with standard convention in the art, the symbol

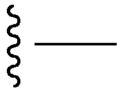

is used in formulas and tables to show the bond that is the point of attachment of the moiety or substituent to the core/nucleus of the structure.

Additonally, for purposes of clarity, where a substituent has a dash (-) that is not between two letters or symbols; this is used to indicate a point of attachment for a substituent. For example, $-CONH_2$ is attached through the carbon atom.

Additionally, for purposes of clarity, when there is no substituent shown at the end of a solid line, this indicates that there is a methyl ($CH_3$) group connected to the bond.

As used herein, the term "alkyl" or "alkylene" is intended to include both branched and straight-chain saturated aliphatic hydrocarbon groups having the specified number of carbon atoms. For example, "$C_1$-$C_6$ alkyl" denotes alkyl having 1 to 6 carbon atoms. Example alkyl groups include, but are not limited to, methyl (Me), ethyl (Et), propyl (*e.g.*, n-propyl and isopropyl), butyl (*e.g.*, n-butyl, isobutyl, *t*-butyl), and pentyl (*e.g.*, n-pentyl, isopentyl, neopentyl).

The term "alkenyl" denotes a straight- or branch-chained hydrocarbon radical containing one or more double bonds and typically from 2 to 20 carbon atoms in length. For example, "$C_2$-$C_8$ alkenyl" contains from two to eight carbon atoms. Alkenyl groups include, but are not limited to, for example, ethenyl, propenyl, butenyl, 1-methyl-2-buten-1-yl, heptenyl, octenyl and the like.

The term "alkynyl" denotes a straight- or branch-chained hydrocarbon radical containing one or more triple bonds and typically from 2 to 20 carbon atoms in length. For example, "$C_2$-$C_8$ alkenyl" contains from two to eight carbon atoms. Representative alkynyl groups include, but are not limited to, for example, ethynyl, 1-propynyl, 1-butynyl, heptynyl, octynyl and the like.

The term "alkoxy" or "alkyloxy" refers to an -O-alkyl group. "$C_{1-6}$ alkoxy" (or alkyloxy), is intended to include $C_1$, $C_2$, $C_3$, $C_4$, $C_5$, and $C_6$ alkoxy groups. Example alkoxy groups include, but are not limited to, methoxy, ethoxy, propoxy (*e.g.*, n-propoxy and isopropoxy), and *t*-butoxy. Similarly, "alkylthio" or "thioalkoxy" represents an alkyl group as defined above with the indicated number of carbon atoms attached through a sulphur bridge; for example methyl-S- and ethyl-S-.

The term "aryl", either alone or as part of a larger moiety such as "aralkyl", "aralkoxy", or aryloxyalkyl", refers to monocyclic, bicyclic and tricyclic ring systems having a total of five to 15 ring members, wherein at least one ring in the system is aromatic and wherein each ring in the system contains three to seven ring members. In certain embodiments of the invention, "aryl" refers to an aromatic ring system which includes, but not limited to phenyl, biphenyl, indanyl, 1-naphthyl, 2-naphthyl and terahydronaphthyl. The term "aralkyl" or "arylalkyl" refers to an alkyl residue attached to an aryl ring. Non-limiting examples include benzyl, phenethyl and the like. The fused aryls may be connected to another group either at a suitable position on the cycloalkyl ring or the aromatic ring. For example:

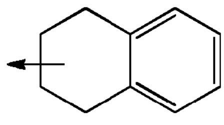

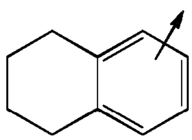

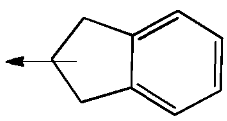

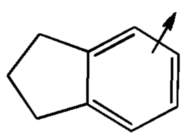

Arrowed lines drawn from the ring system indicate that the bond may be attached to any of the suitable ring atoms.

The term "cycloalkyl" refers to cyclized alkyl groups. $C_{3-6}$ cycloalkyl is intended to include $C_3$, $C_4$, $C_5$, and $C_6$ cycloalkyl groups. Example cycloalkyl groups include, but are not limited to, cyclopropyl, cyclobutyl, cyclopentyl, cyclohexyl, and norbornyl. Branched cycloalkyl groups such as 1-methylcyclopropyl and 2-methylcyclopropyl are included in the definition of "cycloalkyl". The term "cycloalkenyl" refers to cyclized alkenyl groups. $C_{4-6}$ cycloalkenyl is intended to include $C_4$, $C_5$, and $C_6$ cycloalkenyl groups. Example cycloalkenyl groups include, but are not limited to, cyclobutenyl, cyclopentenyl, and cyclohexenyl.

The term "cycloalkylalkyl"refers to a cycloalkyl or substituted cycloalkyl bonded to an alkyl group connected to the carbazole core of the compound.

"Halo" or "halogen" includes fluoro, chloro, bromo, and iodo. "Haloalkyl" is intended to include both branched and straight-chain saturated aliphatic hydrocarbon groups having the specified number of carbon atoms, substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" that is intended to include both branched and straight-chain saturated aliphatic hydrocarbon groups having the specified number of carbon atoms, substituted with 1 or more fluorine atoms.

"Haloalkoxy" or "haloalkyloxy" represents a haloalkyl group as defined above with the indicated number of carbon atoms attached through an oxygen bridge. For example, "$C_{1-6}$ haloalkoxy", is intended to include $C_1$, $C_2$, $C_3$, $C_4$, $C_5$, and $C_6$ haloalkoxy groups. Examples of haloalkoxy include, but are not limited to, trifluoromethoxy, 2,2,2-trifluoroethoxy, and pentafluorothoxy. Similarly, "haloalkylthio" or "thiohaloalkoxy" represents a haloalkyl group as defined above with the indicated number of carbon atoms attached through a sulphur bridge; for example trifluoromethyl-S-, and pentafluoroethyl-S-.

The term "benzyl," as used herein, refers to a methyl group on which one of the hydrogen atoms is replaced by a phenyl group.

As used herein, the term "heterocycle," "heterocyclyl," or "heterocyclic group" is intended to mean a stable 3-, 4-, 5-, 6-, or 7-membered monocyclic or bicyclic or 7-, 8-, 9-, 10-, 11-, 12-, 13-, or 14-membered polycyclic heterocyclic ring that is saturated, partially unsaturated, or fully unsaturated, and that contains carbon atoms and 1, 2, 3 or 4 heteroatoms independently selected from the group consisting of N, O and S; and including any polycyclic group in which any of the above-defined heterocyclic rings is fused to a benzene ring. The nitrogen and sulfur heteroatoms may optionally be oxidized (*i.e.*, N→O and $S(O)_p$, wherein p is 0, 1 or 2). The nitrogen atom may be substituted or unsubstituted (*i.e.*, N or NR wherein R is H or another substituent, if defined). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocycle may optionally be quaternized. It is preferred that when the total number of S and O atoms in the heterocycle exceeds 1, then these heteroatoms are not adjacent to one another. It is preferred that the total number of S and O atoms in the heterocycle is not more than 1. When the term "heterocycle" is used, it is intended to include heteroaryl.

Examples of heterocycles include, but are not limited to, acridinyl, azetidinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, 1*H*-indazolyl, imidazolopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinylperimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2H-pyrrolyl, pyrrolyl, quinazolinyl, quinolinyl, 4*H*-quinolizinyl, quinoxalinyl, quinuclidinyl, tetrazolyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridinyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl. Also included are fused ring and spiro compounds containing, for example, the above heterocycles.

As used herein, the term "bicyclic heterocycle" or "bicyclic heterocyclic group" is intended to mean a stable 9- or 10-membered heterocyclic ring system which contains two fused rings and consists of carbon atoms and 1, 2, 3, or 4 heteroatoms independently selected from the group consisting of N, O and S. Of the two fused rings, one ring is a 5- or 6-membered monocyclic aromatic ring comprising a 5-membered heteroaryl ring, a 6-membered heteroaryl ring or a benzo ring, each fused to a second ring. The second ring is a 5- or 6-membered monocyclic ring which is saturated, partially unsaturated, or unsaturated, and comprises a 5-membered heterocycle, a 6-membered heterocycle or a carbocycle (provided the first ring is not benzo when the second ring is a carbocycle).

The bicyclic heterocyclic group may be attached to its pendant group at any heteroatom or carbon atom which results in a stable structure. The bicyclic heterocyclic group described herein may be substituted on carbon or on a nitrogen atom if the resulting compound is stable. It is preferred that when the total number of S and O atoms in the heterocycle exceeds 1, then these heteroatoms are not adjacent to one another. It is preferred that the total number of S and O atoms in the heterocycle is not more than 1.

Examples of a bicyclic heterocyclic group are, but not limited to, quinolinyl, isoquinolinyl, phthalazinyl, quinazolinyl, indolyl, isoindolyl, indolinyl, 1H-indazolyl, benzimidazolyl, 1,2,3,4-tetrahydroquinolinyl, 1,2,3,4-tetrahydroisoquinolinyl, 5,6,7,8-tetrahydro-quinolinyl, 2,3-dihydro-benzofuranyl, chromanyl, 1,2,3,4-tetrahydro-quinoxalinyl and 1,2,3,4-tetrahydro-quinazolinyl.

As used herein, the term "aromatic heterocyclic group" or "heteroaryl" is intended to mean stable monocyclic and polycyclic aromatic hydrocarbons that include at least one heteroatom ring member such as sulfur, oxygen, or nitrogen. Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl and benzodioxane. Heteroaryl groups are substituted or unsubstituted. The nitrogen atom is substituted or unsubstituted (*i.e.*, N or NR wherein R is H or another substituent, if defined). The nitrogen and sulfur heteroatoms may optionally be oxidized (*i.e.*, $N \rightarrow O$ and $S(O)_p$, wherein p is 0, 1 or 2).

Bridged rings are also included in the definition of heterocycle. A bridged ring occurs when one or more, preferably one to three, atoms (*i.e.*, C, O, N, or S) link two non-adjacent carbon or nitrogen atoms. Examples of bridged rings include, but are not limited to, one carbon atom, two carbon atoms, one nitrogen atom, two nitrogen atoms, and a carbon-nitrogen group. It is noted that a bridge always converts a monocyclic ring into a tricyclic ring. When a ring is bridged, the substituents recited for the ring may also be present on the bridge.

The term "heterocyclylalkyl"refers to a heterocyclyl or substituted heterocyclyl bonded to an alkyl group connected to the carbazole core of the compound.

The term "counter ion" is used to represent a negatively charged species such as chloride, bromide, hydroxide, acetate, and sulfate or a positively charged species such as sodium (Na+), potassium (K+), ammonium ($R_nNH_m$+ where n=0-4 and m=0-4) and the like.

The term "electron withdrawing group" (EWG) refers to a substituent which polarizes a bond, drawing electron density towards itself and away from other bonded atoms. Examples of EWGs include, but are not limited to, $CF_3$, $CF_2CF_3$, CN, halogen, haloalkyl, $NO_2$, sulfone, sulfoxide, ester, sulfonamide, carboxamide, alkoxy, alkoxyether, alkenyl, alkynyl, OH, C(O)alkyl, $CO_2H$, phenyl, heteroaryl, -O-phenyl, and -O-heteroaryl. Preferred examples of EWG include, but are not limited to, $CF_3$, $CF_2CF_3$, CN, halogen, $SO_2(C_{1-4}$ alkyl), CONH($C_{1-4}$ alkyl), CON($C_{1-4}$ alkyl$)_2$, and heteroaryl. More preferred examples of EWG include, but are not limited to, $CF_3$ and CN.

As used herein, the term "amine protecting group" means any group known in the art of organic synthesis for the protection of amine groups which is stable to an ester reducing agent, a disubstituted hydrazine, R4-M and R7-M, a nucleophile, a hydrazine reducing agent, an activator, a strong base, a hindered amine base and a cyclizing agent. Such amine protecting groups fitting these criteria include those listed in Wuts, P. G. M. and Greene, T.W. Protecting Groups in Organic Synthesis, 4th Edition, Wiley (2007) and The Peptides: Analysis, Synthesis, Biology, Vol. 3, Academic Press, New York (1981. Examples of amine protecting groups include, but are not limited to, the following: (1) acyl types such as formyl, trifluoroacetyl, phthalyl, and p-toluenesulfonyl; (2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); (3) aliphatic carbamate types such as *tert*-butyloxycarbonyl (Boc), ethoxycarbonyl, diisopropylmethoxycarbonyl, and allyloxycarbonyl; (4) cyclic alkyl carbamate types such as cyclopentyloxycarbonyl and adamantyloxycarbonyl; (5) alkyl types such as triphenylmethyl and benzyl; (6) trialkylsilane such as trimethylsilane; (7) thiol containing types such as phenylthiocarbonyl and dithiasuccinoyl; and (8) alkyl types such as triphenylmethyl, methyl, and benzyl; and substituted alkyl types such as 2,2,2-trichloroethyl, 2-phenylethyl, and *t*-butyl; and trialkylsilane types such as trimethylsilane.

As referred to herein, the term "substituted" means that at least one hydrogen atom is replaced with a non-hydrogen group, provided that normal valencies are maintained and that the substitution results in a stable compound. Ring double bonds, as used herein, are double bonds that are formed between two adjacent ring atoms (*e.g.*, C=C, C=N, or N=N).

In cases wherein there are nitrogen atoms (*e.g.*, amines) on compounds of the present invention, these may be converted to N-oxides by treatment with an oxidizing agent (*e.g.*, mCPBA and/or hydrogen peroxides) to afford other compounds of this invention. Thus, shown and claimed nitrogen atoms are considered to cover both the shown nitrogen and its N-oxide ($N \rightarrow O$) derivative.

When any variable occurs more than one time in any constituent or formula for a compound, its definition at each occurrence is independent of its definition at every other occurrence. Thus, for example, if a group is shown to be substituted with 0-3 R, then said group may optionally be substituted with up to three R groups, and at each occurrence R is selected independently from the definition of R. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent. Combinations of substituents and/or variables are permissible only if such combinations result in stable compounds.

The present invention is intended to include all isotopes of atoms occurring in the present compounds. Isotopes include those atoms having the same atomic number but different mass numbers. By way of general example and without

limitation, isotopes of hydrogen include deuterium and tritium. The isotopes of hydrogen can be denoted as $^{1}H$ (hydrogen), $^{2}H$ (deuterium) and $^{3}H$ (tritium). They are also commonly denoted as D for deuterium and T for tritium. In the application, $CD_3$ denotes a methyl group wherein all of the hydrogen atoms are deuterium. Isotopes of carbon include $^{13}C$ and $^{14}C$. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic groups such as amines; and alkali or organic salts of acidic groups such as carboxylic acids. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington: The Science and Practice of Pharmacy, 22nd Edition, Allen, L. V. Jr., Ed.; Pharmaceutical Press, London, UK (2012).

In addition, compounds of formula I may have prodrug forms. Any compound that will be converted *in vivo* to provide the bioactive agent (*i.e.*, a compound of formula I) is a prodrug. Various forms of prodrugs are well known in the art. For examples of such prodrug derivatives, see:

a) Bundgaard, H., ed., Design of Prodrugs, Elsevier (1985), and Widder, K. et al., eds., Methods in Enzymology, 112:309-396, Academic Press (1985);
b) Bundgaard, H., Chapter 5, "Design and Application of Prodrugs," A Textbook of Drug Design and Development, pp. 113-191, Krosgaard-Larsen, P. et al., eds., Harwood Academic Publishers (1991);
c) Bundgaard, H., Adv. Drug Deliv. Rev., 8:1-38 (1992);
d) Bundgaard, H. et al., J. Pharm. Sci., 77:285 (1988);
e) Kakeya, N. et al., Chem. Pharm. Bull., 32:692 (1984); and
f) Rautio, J (Editor). Prodrugs and Targeted Delivery (Methods and Principles in Medicinal Chemistry), Vol 47, Wiley-VCH, 2011.

Compounds containing a carboxy group can form physiologically hydrolyzable esters that serve as prodrugs by being hydrolyzed in the body to yield formula I compounds *per se.* Such prodrugs are preferably administered orally since hydrolysis in many instances occurs principally under the influence of the digestive enzymes. Parenteral administration may be used where the ester *per se* is active, or in those instances where hydrolysis occurs in the blood. Examples of physiologically hydrolyzable esters of compounds of formula I include $C_{1-6}$alkyl, $C_{1-6}$alkylbenzyl, 4-methoxybenzyl, indanyl, phthalyl, methoxymethyl, $C_{1-6}$ alkanoyloxy-$C_{1-6}$alkyl (*e.g.*, acetoxymethyl, pivaloyloxymethyl or propionyloxymethyl), $C_{1-6}$alkoxycarbonyloxy-$C_{1-6}$alkyl (*e.g.*, methoxycarbonyl-oxymethyl or ethoxycarbonyloxymethyl, glycyloxymethyl, phenylglycyloxymethyl, (5-methyl-2-oxo-1,3-dioxolen-4-yl)-methyl), and other well known physiologically hydrolyzable esters used, for example, in the penicillin and cephalosporin arts. Such esters may be prepared by conventional techniques known in the art. Preparation of prodrugs is well known in the art and described in, for example, King, F.D., ed., Medicinal Chemistry: Principles and Practice, The Royal Society of Chemistry, Cambridge, UK (2nd edition, reproduced, 2006); Testa, B. et al., Hydrolysis in Drug and Prodrug Metabolism. Chemistry, Biochemistry and Enzymology, VCHA and Wiley-VCH, Zurich, Switzerland (2003); Wermuth, C.G., ed., The Practice of Medicinal Chemistry, 3rd edition, Academic Press, San Diego, CA (2008).

The term "solvate" means a physical association of a compound of this invention with one or more solvent molecules, whether organic or inorganic. This physical association includes hydrogen bonding. In certain instances the solvate will be capable of isolation, for example when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Exemplary solvates include, but are not limited to, hydrates, ethanolates, methanolates, and isopropanolates. Methods of solvation are generally known in the art.

As used herein, the term "patient" refers to organisms to be treated by the methods of the present disclosure.

Such organisms preferably include, but are not limited to, mammals (*e.g.*, murines, simians, equines, bovines, porcines, canines, felines, and the like), and most preferably refers to humans.

As used herein, the term "effective amount" means that amount of a drug or pharmaceutical agent, i.e., a compound of the invention, that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. An effective amount can be administered in one or more administrations, applications or dosages and is not intended to be limited to a particular formulation or administration route. The term also includes within its scope amounts effective to enhance normal physiological function

As used herein, the term "treating" includes any effect, *e.g.,* lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

Examples of bases include, but are not limited to, alkali metals (*e.g.*, sodium) hydroxides, alkaline earth metals (*e.g.*, magnesium), hydroxides, ammonia, and compounds of formula $NW_4^+$, wherein W is $C_{1-4}$ alkyl, and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

METHODS OF PREPARATION

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Preferred methods include, but are not limited to, those described below.

The compounds of this invention may be prepared using the reactions and techniques described in this section. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and work up procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents that are compatible with the reaction conditions will be readily apparent to one skilled in the art and alternate methods must then be used. This will sometimes require a judgment to modify the order of the synthetic steps or to select one particular process scheme over another in order to obtain a desired compound of the invention. It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene and Wuts (Protective Groups In Organic Synthesis, Third Edition, Wiley and Sons, 1999).

Compounds of Formula (I) may be prepared by reference to the methods illustrated in the following Schemes. As shown therein the end product is a compound having the same structural formula as Formula (I). It will be understood that any compound of Formula (I) may be produced by the schemes by the suitable selection of reagents with appropriate substitution. Solvents, temperatures, pressures, and other reaction conditions may readily be selected by one of ordinary skill in the art. Starting materials are commercially available or readily prepared by one of ordinary skill in the art. Constituents of compounds are as defined herein or elsewhere in the specification.

## Scheme 1

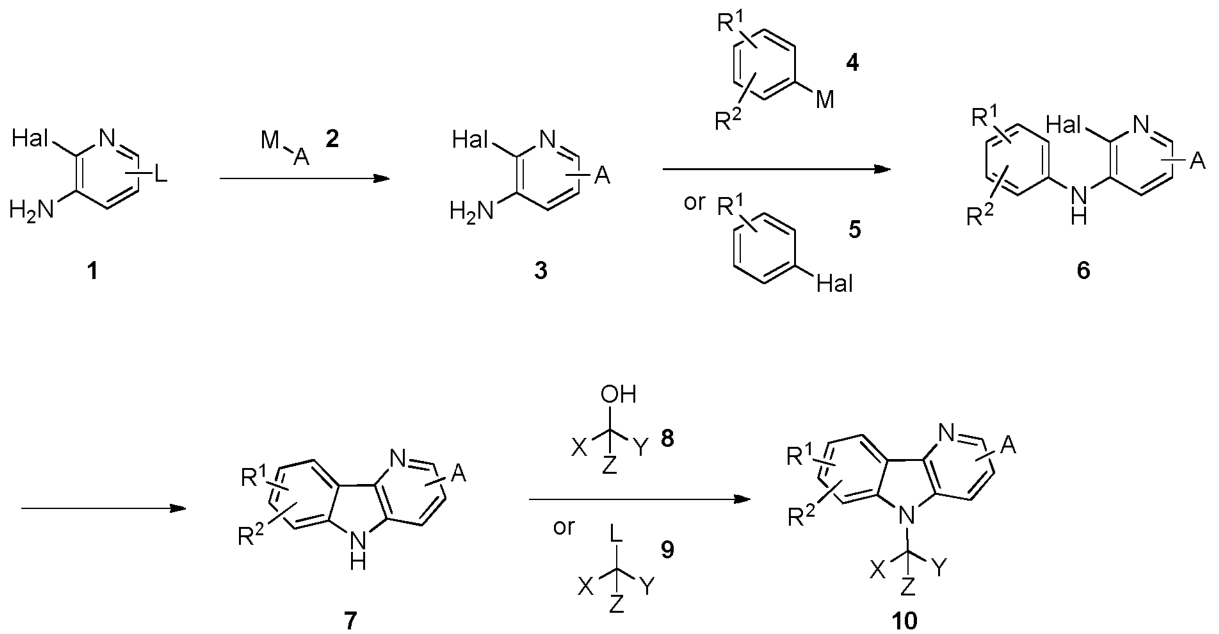

1 2 3 4 or 5 6

7 8 or 9 10

General routes to compounds described in the invention are illustrated in Schemes 1-13, where the $R^1$, $R^2$, X, Y, Z and A substituents are defined previously in the text or a functional group that can be converted to the desired final substituent. The substituent Hal is a halide. L is a leaving group such as a halide or OH that can be easily converted to a leaving group such as a triflate. As shown in Scheme 1, a general procedure for the preparation of compounds of the invention involves starting with the substituted aminopyridine **1**. Coupling of **1** with the aromatic heterocycle A (**2**, where M is a suitable coupling partner, such as boronic acid, boronic ester or stannane) using a suitable catalyst can yield functionalized aminopyridines **3**. For example, **3** could arise from a Suzuki coupling reaction between 5-bromo-2-chloropyridin-3-amine and a heteroaromatic boronic acid using Pd(dppf)$Cl_2$ as a catalyst. Subsequent coupling to give the functionalized aniline **6** can be achieved using a variety of conditions known in the literature. For example, aminopyridine **3** can undergo copper-mediated coupling with a suitably substituted arene **4** (where M is a boronic acid, boronic ester or stannane) to give aniline **6**. Alternatively, **6** could arise from a Buchwald *N*-arylation reaction of **3** with an aromatic halide **5** (where Hal is a halide). Ring closure to generate carboline **7** can be achieved using a Pd catalyst in the presence of a base, such as sodium acetate. In the final step, the carboline nitrogen can be substituted under Mitsunobu conditions using triphenylphosphine and diisopropyl azodicarboxylate (DIAD) with an alkylating agent **8** (where X is OH). Alternatively, functionalized carboline **10** can be generated from a displacement reaction between the carboline **7** and an alkylating agent **9**, where L is a leaving group such as a halide, mesylate or triflate, in the presence of a base, such as potassium carbonate. In cases where **10** is a racemate, chiral separation can provide enantiomerically pure products. Further derivatization of $R^1$ can provide additional compounds of the invention. For example, when $R^1$ is an ester, addition of a Grignard reagent or alkyl lithium can generate tertiary alcohols. The same $R^1$ ester could instead be hydrolyzed using, for example, sodium hydroxide to give a carboxylic acid ($R^1$ = $CO_2H$) as the final substituent.

## Scheme 2

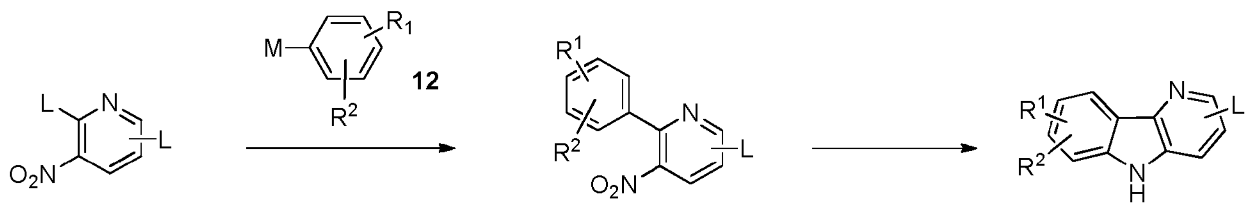

12

11 13 14

An alternative synthesis of the carbolines **7** and **10** starts from nitropyridine **11** as shown in Schemes 2 to 4. A Suzuki reaction between, for example, 2,5-dibromo-3-nitropyridine and an appropriately substituted arene (**12**, where M is a suitable coupling partner, such as boronic acid or boronic ester) can give the functionalized pyridine **13**. Reductive

cyclization mediated by a phosphine reagent, such as 1,2-bis(diphenylphosphino)ethane (dppe), can provide carboline **14**. Coupling of **14** with the aromatic heterocycle A (**2**, where M is a suitable coupling partner, such as boronic acid, boronic ester or stannane) using a suitable catalyst then generates carboline **7** as shown in Scheme 3.

Alternately, the carboline nitrogen of intermediate **14** can be first substituted under Mitsunobu conditions with an alkylating agent **8** (where X is OH) or with alkylating agent **9**, where L is a leaving group such as a halide, mesylate or triflate, in the presence of a base, such as potassium carbonate as previously described in Scheme 1 to give intermediate **15**. Then coupling of **15** with the aromatic heterocycle A (**2**, where M is a suitable coupling partner, such as boronic acid, boronic ester or stannane) using a suitable catalyst then generates the final carboline **10** as shown in Scheme 4.

## Scheme 3

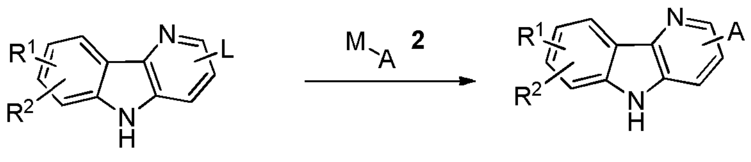


**14** **7**

## Scheme 4

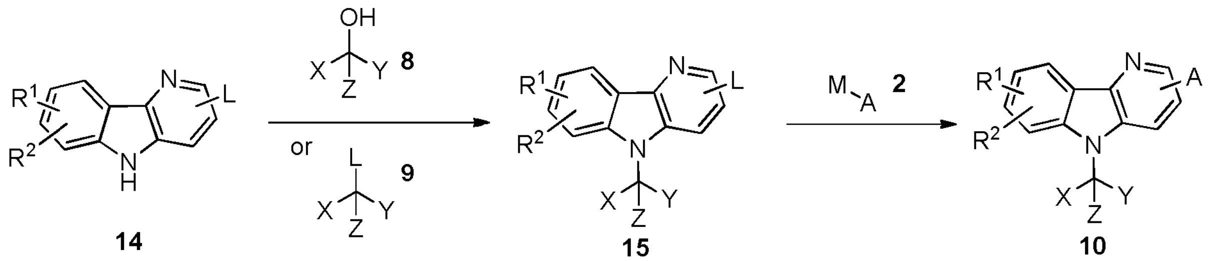


**14** **15** **10**

An alternate synthesis of carbolines **10** can be achieved as outlined in Scheme 5. The leaving group, L, of **15** (prepared as in Scheme 4) can be converted to a suitable coupling partner, M (preferably a boronic ester or boronic acid) by the action of a palladium catalyst, affording **16**. Coupling of **16** with the aromatic heterocycle A (**17**, where L is a suitable leaving group, such as a halogen or triflate) using a suitable catalyst can give carbo lines **10**.

## Scheme 5

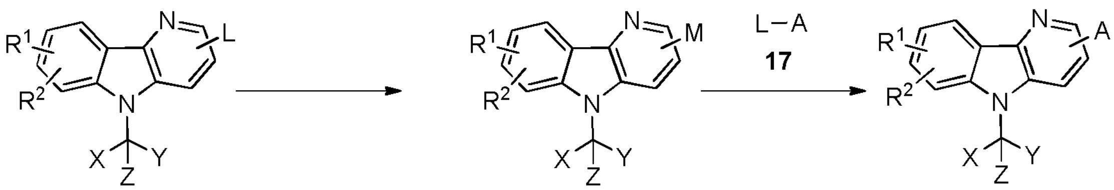


**15** **16** **10**

Hydroxymethyl pyrazole derivatives such as **20** can be accessed according to Scheme 6. Intermediate **16** (where M is a suitable coupling partner such as a boronic acid or boronic ester; prepared as in Scheme 5) can be coupled to an appropriately protected triazole **18** by the action of a suitable catalyst. Triazole **18** is available in one step from a copper-mediated cycloaddition reaction of (azidomethyl)trimethylsilane with a protected propargyl alcohol. Intermediate **19** can then be deprotected using a variety of conditions. For example, when PG is *tert*-butyldimethylsilyl, treatment with tetrabutylammonium fluoride can give the final compound **20**. Further derivatization of the hydroxyl group (for example: alkylation, conversion to a leaving group and displacement, oxidation to either an aldehyde or carboxylic acid and subsequent elaboration) can provide additional compounds of the invention by application of methods which will be readily apparent to one of ordinary skill in the art.

## Scheme 6

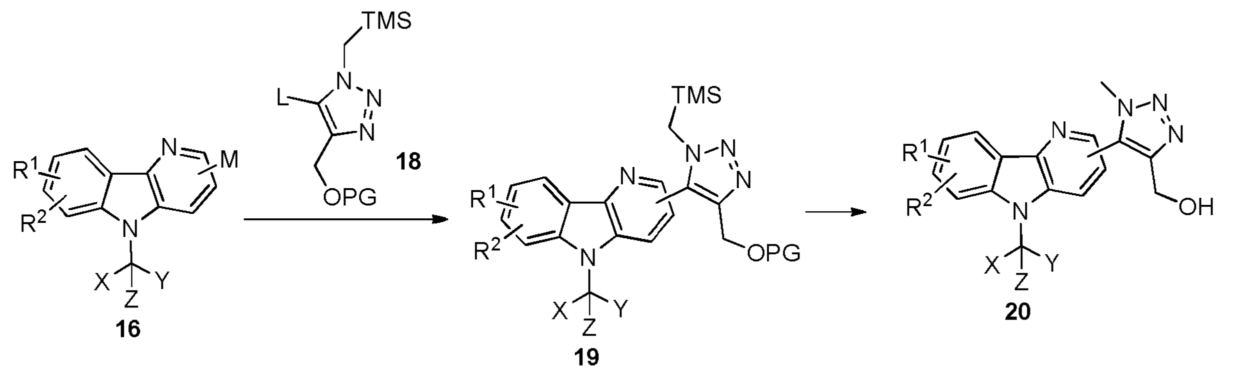

16 18 19 20

Alternately, intermediate 15 (prepared as in Scheme 4) can be directly coupled with a suitable aromatic heterocycle, 21, via palladium-mediated C-H activation to afford compounds 10. This is illustrated in Scheme 7.

## Scheme 7

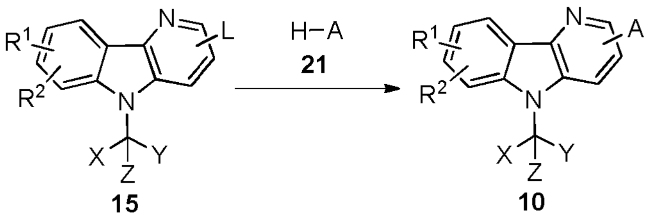

15 21 10

Alternately, aromatic heterocycle **21** can be deprotonated with a strong base such as n-BuLi and transmetallated to zinc, tin, or boron to afford compounds **2**. Compounds **2** can then be coupled in a Negishi, Stille, or Suzuki coupling to intermediate **15** (prepared as in Scheme 4) by the action of a suitable palladium catalyst to afford compounds **10**. This is illustrated in Scheme 8.

## Scheme 8

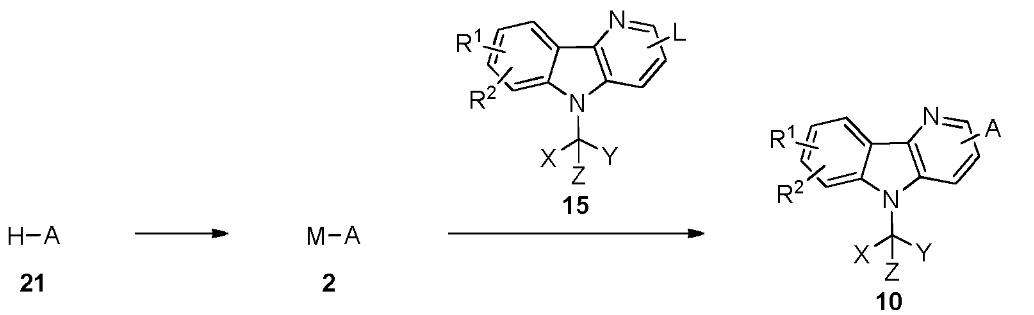

21 2 15 10

An alternate synthesis of carbolines **14** can be achieved as outlined in Scheme 9. Aniline **22** can be coupled to pyridine **23**, where L and L' are two leaving groups such as halide or triflate, using a Buchwald *N*-arylation reaction to give intermediate **24**. For example, **24** could arise from a Buchwald N-arylation reaction between 3,5-dibromopyridine and a suitable aniline. Oxidative ring closure, using an appropriate catalyst such as $Pd(OAc)_2$ in an acidic media such as trifluoroacetic acid, can afford carbolines **14**. This is illustrated in Scheme 9.

## Scheme 9

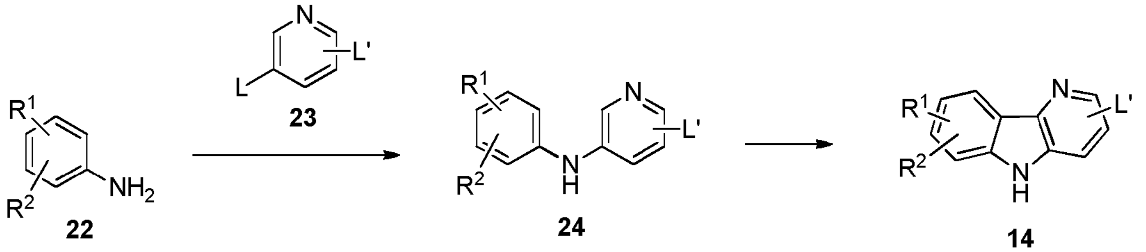

22 24 14

Pyridines **23** (where L and L' are suitable leaving groups such as halides or triflates) can also be coupled to aromatic heterocycles **2** (where M is a suitable coupling partner such as a boronic ester, boronic acid, or stannane) or **21** by methods analogous to those illustrated in Schemes 1, 3, 4, 7, and 8. Pyridines **25** can be coupled to anilines **22**, using a Buchwald *N*-arylation reaction to give intermediate **26**. Oxidative ring closure, using an appropriate catalyst such as $Pd(OAc)_2$ in an acidic media such as trifluoroacetic acid, can afford carbolines **7**. This is illustrated in Scheme 10.

## Scheme 10

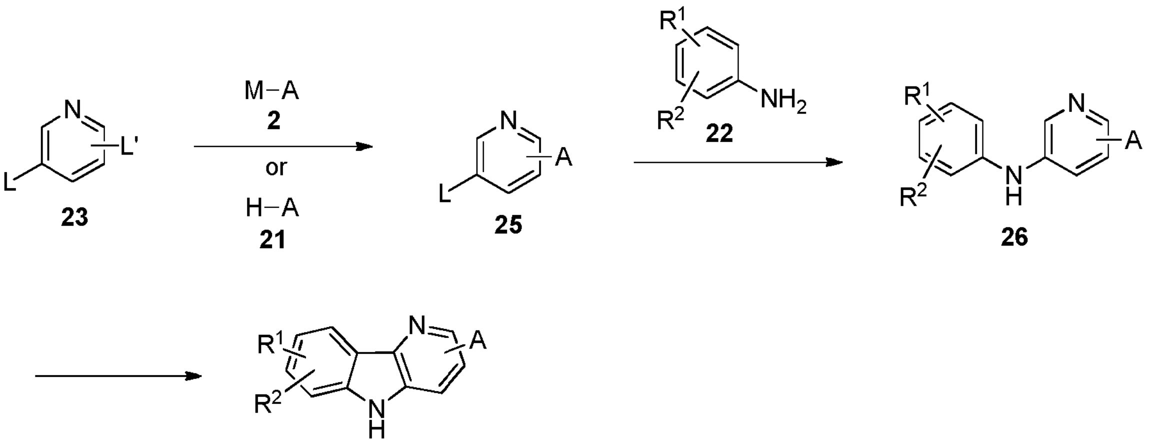

2 or 21

23 25 26

7

Alkoxy-substituted triazoles **32** can be prepared as illustrated in Scheme 11. Aldehyde **27** can be converted to acetal **29** by treatment with alcohol **28** (where Alk is a $C_1$-$C_6$ alkyl or $C_3$-$C_6$ cycloalkyl optionally substituted with deuterium) in the presence of acid or a dehydrating agent such as $CaCl_2$. Acetal **29** can be converted to alkoxy-substituted alkynes **30** by treatment with a strong base such as lithium diethylamide or sodium amide. Compounds **30** can be converted to triazoles **32** through a copper-catalyzed 3+2 cycoaddition reaction with azide **31**. Triazoles **32** can be directly coupled to carbolines as illustrated in Scheme 7. In most cases, said coupling results in loss of the trimethylsilyl group. In cases where the trimethylsilyl group is not lost, it can be removed by treatment with tetrabutylammonium fluoride.

## Scheme 11

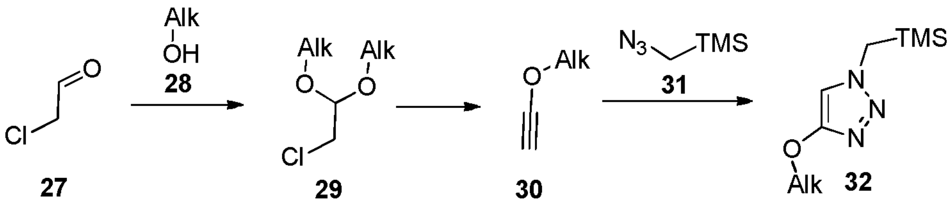

28 31

27 29 30 32

Alkyl-substituted triazoles **39** can be prepared as illustrated in Scheme 12. Acetylene **33** can be alkylated with **34** (where Alk is a $C_1$-$C_6$ alkyl or $C_3$-$C_6$ cycloalkyl optionally substituted with deuterium and where L is an appropriate leaving group such as iodide, bromide, chloride, or sulfonate) by the action of a strong base such as n-BuLi. Alkyne **35** can be converted to triazoles **36** through a copper-catalyzed 3+2 cycoaddition reaction with **31**. Triazoles **36** can be

directly coupled to carbolines as illustrated in Scheme 7. Alternately, the trimethylsilyl group of **36** can be removed directly by the action of tetrabutyl ammonium fluoride to give N-methyl-triazole **37**. Deprotonation of **37** with a strong base such as n-BuLi, followed by reaction with an appropriate electrophile **38** (where L is a leaving group such as a halide or alkoxide and M is an appropriate group to facilitate metal-mediated couplings such as tributyltin or a boronic ester; e.g. M-L = $Bu_3SnCl$ or $B(OMe)_3$) can afford triazoles **39** which can readily be coupled as illustrated in Schemes 1, 3, 4, 8, and 10.

## Scheme 12

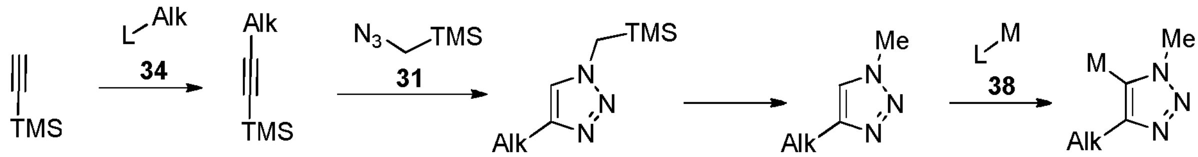

33 35 36 37 39

One can vary the substituents of the triazole as shown in Scheme 13. The leaving group of **34** (where Alk is a $C_1$-$C_6$ alkyl or $C_3$-$C_6$ cycloalkyl optionally substituted with deuterium and where L is an appropriate leaving group such as iodide, bromide, chloride, or sulfonate) can be displaced by treatment with sodium azide to afford **40**. Alkynes **41** or **42** can be coupled to azides **40** to give triazoles **43** through a copper-catalyzed 3+2 cycoaddition reaction. Triazoles **43** can be directly coupled to carbolines as illustrated in Scheme 7. Alternately, deprotonation of **43** with a strong base such as n-BuLi, followed by reaction with an appropriate electrophile **38** (where L is a leaving group such as a halide or alkoxide and M is an appropriate group to facilitate metal-mediated couplings such as tributyltin or a boronic ester; e.g. M-L = $Bu_3SnCl$ or $B(OMe)_3$) can afford triazoles **44** which can readily be coupled as illustrated in Schemes 1, 3, 4, 8, and 10.

## Scheme 13

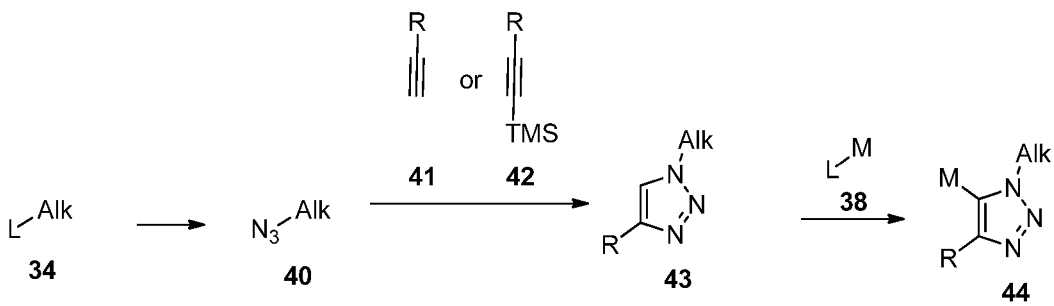

34 40 43 44

EXAMPLES

The invention is further defined in the following Examples. It should be understood that the Examples are given by way of illustration only. From the above discussion and the Examples, one skilled in the art can ascertain the essential characteristics of the invention, and without departing from the spirit and scope thereof, can make various changes and modifications to adapt the invention to various uses and conditions.

ABBREVIATIONS

| | |
|---|---|
| MeCN | Acetonitrile |
| AcOH | acetic acid |
| $AlMe_3$ | trimethyl aluminum |
| aq | Aqueous |
| Bn | Benzyl |

(continued)

| | |
|---|---|
| Boc | *tert*-butoxycarbonyl |
| $BoC_2O$ | di-tert-butyl dicarbonate |
| CBz | benzyloxycarbonyl |
| DCC | 1,3 -dicyclohexylcarbodiimide |
| DCM | dichloromethane |
| DDQ | 2,3-dichloro-5,6-dicyano-1,4-benzoquinone |
| DIAD | diisopropyl azodicarboxylate |
| DIEA | diisopropylethylamine |
| DMAP | 4-dimethylaminopyridine |
| DMA | dimethylacetamide |
| DME | dimethoxyethane |
| DMF | dimethylformamide |
| DMSO | dimethyl sulfoxide |
| EDC | 1-(3-dimethylaminopropyl)-3-ethylcarbodiimide hydrochloride |
| $Et_2AlCl$ | diethyl aluminum chloride |
| $Et_3N$ | triethyl amine |
| $Et_2O$ | diethyl ether |
| EtOH | Ethanol |
| EtOAc | ethyl acetate |
| equiv. | equivalent(s) |
| g | gram(s) |
| h or hr | hour(s) |
| HOBt | hydroxybenzotriazole |
| HPLC | high pressure liquid chromatography |
| iPrOH | isopropyl alcohol |
| KOtBu | potassium *tert*-butoxide |
| LCMS | Liquid Chromatography-Mass Spectroscopy |
| LDA | lithium diisopropylamide |
| LiHMDS | lithium bis(trimethylsilyl)amide |
| Me | Methyl |
| MeI | methyl iodide |
| MeOH | Methanol |
| min | minute(s) |
| mL | milliliter(s) |
| mmol | Millimolar |
| MTBE | methyl t-butyl ether |
| NaHMDS | sodium bis(trimethylsilyl)amide |
| *n*-BuLi | n-butyl lithium |
| $NH_4OAc$ | ammonium acetate |

(continued)

| | |
|---|---|
| NMP | *N*-methylpyrrolidinone |
| $Pd(OAc)_2$ | palladium acetate |
| $Pd(dppf)Cl_2$ | [1,1'-*bis*(diphenylphosphino)ferrocene]dichloropalladium(II) |
| RT or Rt | retention time |
| sat | Saturated |
| SFC | Supercritical fluid chromatography |
| *t*-Bu | tertiary butyl |
| *t*-BuLi | t-butyl lithium |
| *t*-BuOH | tertiary butyl alcohol |
| *t*-BuOMe | *tert*-butyl methyl ether |
| TBTU | O-(1 H-benzotriazol-1-yl)-N,N,N',N'-tetramethyluronium tetrafluoroborate |
| TCTU | O-(1H-6-chlorobenzotriazol-1-yl)-N,N,N',N'-tetramethyluronium tetrafluoro borate |
| TEA | Triethylamine |
| TFA | trifluoroacetic acid |
| $Tf_2O$ | trifluoromethylsulfonic anhydride |
| THF | Tetrahydrofuran |

The following HPLC conditions may be used where indicated:

Analytical HPLC Method 1: Column: Waters Acquity UPLC BEH C18, 2.1 x 50 mm, 1.7 μm particles; Mobile Phase A: water with 0.05% TFA; Mobile Phase B: acetonitrile 0.05% TFA; Gradient: 2-98% B over 1 min, then a 0.5-min hold at 98% B; Flow: 0.8 mL/min; Detection: UV at 254 nm.

Analytical HPLC Method 2: Column: Waters Acquity UPLC BEH C18, 2.1 x 50 mm, 1.7 μm particles; Mobile Phase A: 5:95 acetonitrile:water with 10 mM ammonium acetate; Mobile Phase B: 95:5 acetonitrile:water with 10 mM ammonium acetate; Gradient: 0-100% B over 3 min, then a 0.7-min hold at 100% B; Flow: 1.11 mL/min; Detection: UV at 254 nm.

LC/MS Method 1: Column: Phenomenex-Luna 2.0 X 30 mm, 3 um particles; Mobile Phase A: 10/90 methanol:water, 0.1% TFA; Mobile Phase B: 90/10 methanol:water, 0.1% TFA; Temperature 40 °C; Gradient 0% -100% B over 2 min; Flow 1 mL/min; Detection: UV at 220 nm.

LC/MS Method 2: Column: Waters Acquity SDS; Mobile Phase A: 100% Water, 0.1% TFA; Mobile Phase B: 100% acetonitrile, 0.1% TFA; Temperature 50 °C; Gradient 2% -98 % B over 2.2 min; Flow 0.8 mL/min; Detection: UV at 220 nm.

LC/MS Method 3: Waters BEH C18, 2.0 x 50 mm, 1.7-μm particles; Mobile Phase A: 5:95 acetonitrile:water with 10 mM ammonium acetate; Mobile Phase B: 95:5 acetonitrile:water with 10 mM ammonium acetate; Temperature: 50 °C; Gradient: 0%B, 0-100% B over 3 min, then a 0.5-min hold at 100% B; Flow: 1 mL/min; Detection: UV at 220 nm.

LC/MS Method 4: Waters BEH C18, 2.0 x 50 mm, 1.7-μm particles; Mobile Phase A: 5:95 methanol:water with 10 mM ammonium acetate; Mobile Phase B: 95:5 methanol:water with 10 mM ammonium acetate; Temperature: 50 °C; Gradient: 0%-100% B over 3 min, then a 0.5-min hold at 100% B; Flow: 0.5 mL/min; Detection: UV at 220 nm.

Preparative HPLC Method 1: Column: XBridge C18, 19 x 200 mm, 5-μm particles; Mobile Phase A: 5:95 acetonitrile: water with 10-mM ammonium acetate; Mobile Phase B: 95:5 acetonitrile: water with 10-mM ammonium acetate; Gradient: 30-70% B over 20 min, then a 5-min hold at 100% B; Flow: 20 mL/min.

Preparative HPLC Method 2: Column: XBridge C18, 19 x 200 mm, 5-μm particles; Mobile Phase A: 5:95 methanol:

water with 10-mM ammonium acetate; Mobile Phase B: 95:5 methanol: water with 10-mM ammonium acetate; Gradient: 35-75% B over 20 min, then a 5-min hold at 100% B; Flow: 20 mL/min.

Preparative HPLC Method 3: Column: XBridge C18, 19 x 200 mm, 5-μm particles; Mobile Phase A: 5:95 acetonitrile: water with 0.1% trifluoroacetic acid; Mobile Phase B: 95:5 acetonitrile: water with 0.1% trifluoroacetic acid; Gradient: 15-55% B over 20 min, then a 5-min hold at 100% B; Flow: 20 mL/min.

**Reference Examples 1 & 2**

**2-[3-(Dimethyl-1,2-oxazol-4-yl)-5-[oxan-4-yl(phenyl)methyl]-5*H*-pyrido[3,2-*b*]indol-7-yl]propan-2-ol**

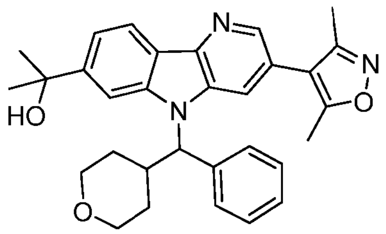

**Enantiomer A, Ref. Example 1**

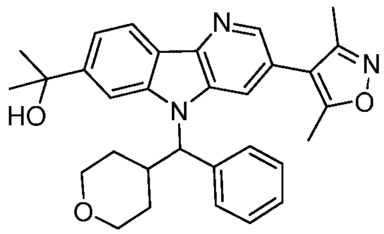

**Enantiomer B, Ref. Example 2**

**Step 1: 2-Chloro-5-(3,5-dimethylisoxazol-4-yl)pyridin-3-amine**

To a 500 mL round bottom flask containing 5-bromo-2-chloropyridin-3-amine (Matrix, 4.0 g, 19.3 mmol) and (3,5-dimethylisoxazol-4-yl)boronic acid (AOBChem, 3.26 g, 23.1 mmol) in THF (150 mL) was added tripotassium phosphate (2M aq., 28.9 mL, 57.8 mmol) to give a yellow suspension. Pd(dppf)$Cl_2$-$CH_2Cl_2$ (1.58 g, 1.93 mmol) was then added and $N_2$ was bubbled into the mixture for 4 min. The resulting reaction mixture was heated at 80 °C for 1 h, concentrated and then diluted with 10% LiCl solution and extracted with $CH_2Cl_2$. The organic layer was concentrated and filtered through Celite®. The mother liquor was purified using ISCO silica gel chromatography (220 g column, gradient from 0% to 50% EtOAc/$CH_2Cl_2$). Trituration with cold $Et_2O$ gave the title compound (3.14 g, 73%) as a pale orange solid. $^1H$ NMR (400 MHz, $CDCl_3$) δ 7.71 (d, *J*=2.1 Hz, 1H), 6.92 (d, *J*=2.1 Hz, 1H), 4.20 (br. s., 2H), 2.42 (s, 3H), 2.27 (s, 3H); LCMS (M+H) = 224.1. HPLC RT = 1.39 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 2: Methyl 3-((2-chloro-5-(3,5-dimethylisoxazol-4-yl)pyridin-3-yl)amino)benzoate**

To a 250 mL round bottom flask containing 2-chloro-5-(3,5-dimethylisoxazol-4-yl)pyridin-3-amine (2.0 g, 8.9 mmol), (3-(methoxycarbonyl)phenyl)boronic acid (Aldrich, 3.22 g, 17.9 mmol), $Cu(OAc)_2$ (2.43 g, 13.4 mmol) and powdered 4Å molecular sieves (7.0 g) was added $CHCl_3$ (50 mL) and pyridine (1.45 mL, 17.9 mL). The atmosphere was exchanged with $O_2$, and the reaction was stirred under an $O_2$ balloon for 6 h. Additional (3-(methoxycarbonyl)phenyl)boronic acid (3.22 g, 17.9 mmol), pyridine (1.45 mL, 17.9 mmol) and 4Å molecular sieves (1.7 g) were added. The reaction mixture was stirred at room temperature overnight. Additional (3-(methoxycarbonyl)phenyl)boronic acid (3.22 g, 17.9 mmol), pyridine (1.45 mL, 17.9 mmol) and $Cu(OAc)_2$ (400 mg) were added to the reaction. After stirring at room temperature for 7 h, the reaction mixture was filtered through Celite® rinsing with $CHCl_3$. The filtrate was diluted with water and ammonium hydroxide (18.6 mL, 143 mmol) was added. The aqueous layer was extracted with $CHCl_3$ and the combined organic layers were washed with 10% LiCl. The organic layer was concentrated and purified by silica gel chromatography (220 g column, gradient from 0% to 50%EtOAc/$CH_2Cl_2$). The fractions were concentrated *in vacuo* until a white precipitate formed which collected via filtration and rinsed with EtOAc to give the title compound (1.33 g, 57%) as a white solid. $^1H$ NMR (400 MHz, $CDCl_3$) δ 7.94 (t, *J*=1.8 Hz, 1H), 7.85 (d, *J*=2.1 Hz, 1H), 7.82 (dt, *J*=7.7, 1.3 Hz, 1H), 7.48 (t, *J*=7.9 Hz, 1H), 7.40 (d, *J*=2.1 Hz, 1H), 7.36 (ddd, *J*=8.0, 2.3, 1.0 Hz, 1H), 6.32 (s, 1H), 3.94 (s, 3H), 2.45 (s, 3H), 2.29 (s, 3H); LCMS (M+H) = 358.2; HPLC RT = 2.70 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 3: Methyl 3-(3,5-dimethylisoxazol-4-yl)-5*H*-pyrido[3,2-b]indole-7-carboxylate**

To a 40 mL vial containing methyl 3-((2-chloro-5-(3,5-dimethylisoxazol-4-yl)pyridin-3-yl)amino)benzoate (515 mg, 1.44 mmol) and sodium acetate trihydrate (480 mg, 3.52 mmol) in DMA (5.0 mL) was added bis(triphenylphosphine)palladium(II) chloride (81 mg, 0.12 mmol). $N_2$ was bubbled through the reaction mixture for 1 min. The vial was capped and heated at 180 °C for 15-30 min. The reaction mixture was then concentrated and purified directly using ISCO silica gel chromatography (40 g column, gradient from 0% to 100% EtOAc/$CH_2Cl_2$). The resulting orange oil was dissolved in EtOAc (7 mL) and stirred at room temperature overnight. The resulting yellow precipitate was collected via filtration and washed with EtOAc. The mother liquor was concentrated and repurified using ISCO silica gel chromatography (40 g column, gradient from 0% to 50% EtOAc/$CH_2Cl_2$). After trituration with cold EtOAc, the solids were combined to give the title compound (301 mg, 65%). HPLC RT = 2.01 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 4: Methyl 3-(3,5-dimethylisoxazol-4-yl)-5-(phenyl(tetrahydro-2*H*-pyran-4-yl)methyl)-5*H*-pyrido[3,2-b]indole-7-carboxylate**

To a 5 mL vial containing methyl 3-(3,5-dimethylisoxazol-4-yl)-5*H*-pyrido[3,2-b]indole-7-carboxylate (87 mg, 0.27 mmol) and phenyl(tetrahydro-2*H*-pyran-4-yl)methanol (104 mg, 0.54 mmol) [Orjales, A. et al. J. Med. Chem. 2003, 46, 5512-5532] in THF (2.0 mL) was added $Ph_3P$ (141 mg, 0.54 mmol) and DIAD (0.11 mL, 0.54 mmol). The resulting suspension was stirred at room temperature overnight and then concentrated. The residue was purified using ISCO silica gel chromatography (40 g column, gradient from 0% to 50% EtOAc/$CH_2Cl_2$) to give the title compound (139 mg) as an impure mixture which was carried on to the subsequent step without further purification. LCMS (M+H) = 496.2; HPLC RT = 3.06 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 5: 2-[3-(Dimethyl-1,2-oxazol-4-yl)-5-[oxan-4-yl(phenyl)methyl]-5*H*-pyrido[3,2-*b*]indol-7-yl]propan-2-ol**

A 250 mL round bottom flask containing methyl 3-(3,5-dimethylisoxazol-4-yl)-5-(phenyl(tetrahydro-2*H*-pyran-4-yl)methyl)-5*H*-pyrido[3,2-b]indole-7-carboxylate (1.58 g, 3.19 mmol) in $CH_2Cl_2$ (50 mL) was cooled in an ice/MeOH bath. MeMgBr, (3M in $Et_2O$, 17.0 mL, 51.0 mmol) was added slowly over 2 min. The resulting suspension was stirred for 2.5 h and then quenched carefully with sat. $NH_4Cl$. Ice was added to the reaction mixture followed by 10% LiCl solution. The aqueous layer was extracted with $CH_2Cl_2$ (2x). The organic layer was dried over $MgSO_4$, filtered and concentrated to give racemic 2-[3-(dimethyl-1,2-oxazol-4-yl)-5-[oxan-4-yl(phenyl)methyl]-5*H*-pyrido[3,2-*b*]indol-7-yl]propan-2-ol, which was separated using chiral prep SFC (Column: Chiral OD-H 25 x 3 cm, 5 μm; Mobile Phase: 70/30 $CO_2$/MeOH; Flow: 85 mL/min). The faster eluting peak was concentrated to a small volume. Water was added to form a white precipitate which was collected via filtration, rinsing with water, to give a white solid which was assigned as Enantiomer A (0.59 g, 36%). The slower eluting peak was treated in an identical manner and assigned as Enantiomer B (0.51 g, 31%). Enantiomer A: $^1H$ NMR (500 MHz, $CDCl_3$) δ 8.40 (d, *J*=1.8 Hz, 1H), 8.33 (d, *J*=8.2 Hz, 1H), 7.93 (s, 1H), 7.53 (d, J=1.8 Hz, 1H), 7.46 (d, *J*=7.3 Hz, 2H), 7.42 (dd, *J*=8.2, 1.4 Hz, 1H), 7.37 - 7.31 (m, 2H), 7.30 - 7.28 (m, 1H), 5.56 (d, *J*=10.5 Hz, 1H), 4.06 (d, *J*=8.9 Hz, 1H), 3.89 - 3.83 (m, 1H), 3.55 (td, *J*=11.9, 2.1 Hz, 1H), 3.35 (td, *J*=11.9, 2.1 Hz, 1H), 3.10 (q, *J*=10.8 Hz, 1H), 2.39 (s, 3H), 2.23 (s, 3H), 2.03 (d, *J*=14.2 Hz, 1H), 1.89 (s, 1H), 1.74 (s, 6H), 1.68 - 1.59 (m, 1H), 1.46 - 1.36 (m, 1H), 1.12 (d, *J*=12.2 Hz, 1H); LCMS (M+H) = 496.4; HPLC RT = 2.46 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min); SFC RT = 5.36 min (Column: Chiralcel OD-H 250 x 4.6 mm, 5 μm; Mobile Phase: 70/30 $CO_2$/MeOH; Flow: 2 mL/min). Enantiomer B: $^1H$ NMR (500 MHz, $CDCl_3$) δ 8.40 (d, *J*=1.8 Hz, 1H), 8.33 (d, *J*=8.2 Hz, 1H), 7.94 (s, 1H), 7.53 (d, *J*=1.8 Hz, 1H), 7.46 (d, *J*=7.3 Hz, 2H), 7.42 (dd, *J*=8.2, 1.4 Hz, 1H), 7.36 - 7.31 (m, 2H), 7.30 - 7.28 (m, 1H), 5.56 (d, *J*=10.7 Hz, 1H), 4.06 (dd, *J*=11.7, 2.5 Hz, 1H), 3.86 (dd, *J*=11.5, 2.8 Hz, 1H), 3.55 (td, *J*=11.9, 2.1 Hz, 1H), 3.35 (td, *J*=11.9, 2.0 Hz, 1H), 3.15 - 3.05 (m, 1H), 2.39 (s, 3H), 2.23 (s, 3H), 2.03 (d, *J*=13.6 Hz, 1H), 1.90 (s, 1H), 1.74 (s, 6H), 1.68 - 1.58 (m, 1H), 1.46 - 1.36 (m, 1H), 1.12 (d, *J*=12.4 Hz, 1H); LCMS (M+H) = 496.4; HPLC RT = 2.46 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min); SFC RT = 14.95 min (Column: Chiralcel OD-H 250 x 4.6 mm, 5 μm; Mobile Phase: 70/30 $CO_2$/MeOH; Flow: 2 mL/min).

**Reference Examples 3 & 4**

**2-[3-(Dimethyl-1,2-oxazol-4-yl)-5-[oxan-4-yl(phenyl)methyl]-5*H*-pyrido[3,2-*b*]indol-7-yl]propan-2-ol**

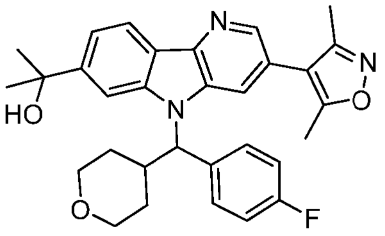

**Enantiomer A, Ref. Example 3**

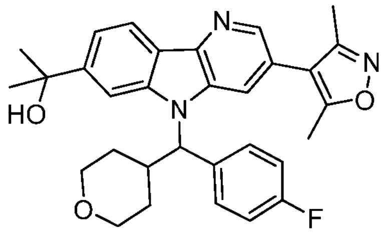

**Enantiomer B, Ref. Example 4**

**Step 1: (4-Fluorophenyl)(tetrahydro-2*H*-pyran-4-yl)methanol**

To a 40 mL vial containing magnesium (0.39 g, 16.1 mmol) in THF (15 mL) was slowly added 4-bromotetrahydro-2*H*-pyran (PharmaBlock, 1.8 mL, 16.1 mmol) cooling in a water bath as needed. The resulting reaction mixture was stirred at room temperature for 1.5 h and then cooled in a water bath. 4-Fluorobenzaldehyde (Aldrich, 1.2 mL, 10.7 mmol) was added slowly. The resulting orange reaction mixture was removed from the water bath and quenched with sat. $NH_4Cl$ after 10 min. 10% LiCl solution was added and the mixture was extracted with $Et_2O$ (2x). The organic layer was dried over $MgSO_4$, filtered and concentrated. The residue was purified using ISCO silica gel chromatography (80 g column, gradient from 0% to 50% EtOAc/hexanes) to give the title compound (1.12 g, 33%) as a colorless oil. $^1H$ NMR (500 MHz, $CDCl_3$) δ 7.31 - 7.27 (m, 2H), 7.08 - 7.02 (m, 2H), 4.37 (dd, *J*=7.7, 2.4 Hz, 1H), 4.06 - 3.99 (m, 1H), 3.94 - 3.87 (m, 1H), 3.37 (td, *J*=11.9, 2.2 Hz, 1H), 3.29 (td, *J*=11.8, 2.3 Hz, 1H), 1.94 - 1.87 (m, 2H), 1.81 (tdt, *J*=11.6, 7.7, 3.8 Hz, 1H), 1.45 (qd, *J*=12.3, 4.7 Hz, 1H), 1.36 - 1.27 (m, 1H), 1.16 (ddq, *J*=13.2, 3.9, 2.0 Hz, 1H); LCMS (M+H-$H_2O$) = 193.1; HPLC RT = 1.65 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 2: 2-[3-(Dimethyl-1,2-oxazol-4-yl)-5-[oxan-4-yl(phenyl)methyl]-5*H*-pyrido[3,2-*b*]indol-7-yl]propan-2-ol**

Following procedures analogous to those described in Steps 4 and 5 of Reference Example 1, methyl 3-(3,5-dimethylisoxazol-4-yl)-5*H*-pyrido[3,2-*b*]indole-7-carboxylate (100 mg, 0.31 mmol) and (4-fluorophenyl)(tetrahydro-2*H*-pyran-4-yl)methanol (131 mg, 0.62 mmol) were converted to racemic 2-[3-(dimethyl-1,2-oxazol-4-yl)-5-[oxan-4-yl(phenyl)methyl]-5*H*-pyrido[3,2-*b*]indol-7-yl]propan-2-ol, which was separated by chiral prep SFC to give Enantiomer A (18 mg, 11%) and Enantiomer B (22 mg, 12%). Enantiomer A: $^1H$ NMR (500 MHz, $CDCl_3$) δ 8.41 (d, *J*=1.8 Hz, 1H), 8.33 (d, *J*=8.2 Hz, 1H), 7.91 (s, 1H), 7.50 (d, *J*=1.5 Hz, 1H), 7.46 - 7.37 (m, 3H), 7.06 - 6.99 (m, 2H), 5.53 (d, *J*=10.5 Hz, 1H), 4.07 (dd, *J*=11.7, 2.5 Hz, 1H), 3.87 (dd, *J*=11.8, 3.0 Hz, 1H), 3.54 (td, *J*=11.9, 2.1 Hz, 1H), 3.34 (td, *J*=11.9, 2.1 Hz, 1H), 3.11 - 3.01 (m, 1H), 2.41 (s, 3H), 2.25 (s, 3H), 1.98 (d, *J*=13.4 Hz, 1H), 1.89 (s, 1H), 1.73 (s, 6H), 1.66 - 1.59 (m, 1H), 1.46 - 1.36 (m, 1H), 1.13 (d, *J*=13.3 Hz, 1H); LCMS (M+H) = 514.4; HPLC RT = 2.55 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min); SFC RT = 6.56 min (Column: Chiralcel OD-H 250 x 4.6 mm, 5 μm; Mobile Phase: 75/25 $CO_2$/MeOH; Flow: 2 mL/min). Enantiomer B: $^1H$ NMR (500 MHz, $CDCl_3$) δ 8.41 (d, *J*=1.8 Hz, 1H), 8.33 (d, *J*=8.2 Hz, 1H), 7.91 (s, 1H), 7.50 (d, *J*=1.5 Hz, 1H), 7.46 - 7.37 (m, 3H), 7.06 - 6.99 (m, 2H), 5.53 (d, *J*=10.5 Hz, 1H), 4.07 (dd, *J*=11.7, 2.5 Hz, 1H), 3.87 (dd, *J*=11.8, 3.0 Hz, 1H), 3.54 (td, *J*=11.9, 2.1 Hz, 1H), 3.34 (td, *J*=11.9, 2.1 Hz, 1H), 3.11 - 3.01 (m, 1H), 2.41 (s, 3H), 2.25 (s, 3H), 1.98 (d, *J*=13.4 Hz, 1H), 1.89 (s, 1H), 1.73 (s, 6H), 1.66 - 1.59 (m, 1H), 1.46 - 1.36 (m, 1H), 1.13 (d, *J*=13.3 Hz, 1H); LCMS (M+H) = 514.4; HPLC RT = 2.55 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min); SFC RT = 8.58 min (Column: Chiralcel OD-H 250 x 4.6 mm, 5 μm; Mobile Phase: 75/25 $CO_2$/MeOH; Flow: 2 mL/min); $[\alpha]_D^{20}$ = +89.91, (c = 0.14, $CHCl_3$).

**Reference Examples 5 & 6**

**2-{5-[(4-Fluorophenyl)(oxan-4-yl)methyl]-3-(5-methyl-1,2-oxazol-4-yl)-5*H*-pyrido[3,2-*b*]indol-7-yl}propan-2-ol**

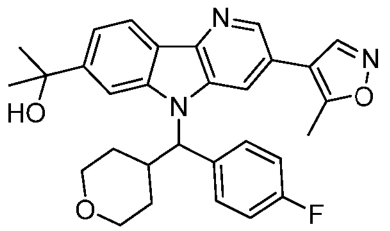

**Enantiomer A, Ref. Example 5**

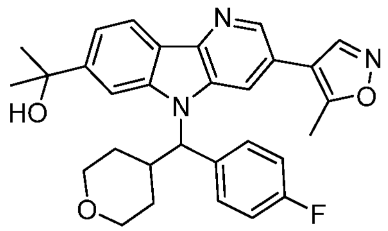

**Enantiomer B, Ref. Example 6**

**Step 1: Methyl 4-(5-bromo-3-nitropyridin-2-yl)benzoate**

To a mixture of 2,5-dibromo-3-nitropyridine (2.00 g, 7.09 mmol), (4-(methoxycarbonyl)phenyl)boronic acid (1.28 g, 7.09 mmol) Pd(dppf)$Cl_2$ (0.36 g, 0.50 mmol) in THF (30 mL) was added tripotassium phosphate (3M in water) (7.09 mL, 21.3 mmol). The reaction mixture was purged with $N_2$ (3x) and then stirred at 80 °C for 3 h. The aqueous layer was separated. The organic layer dried with $Na_2SO_4$, filtered through a small plug of Celite® washing with EtOAc and concentrated. The crude residue was purified using ISCO silica gel chromatography (120 g column, gradient from 0% to 50% EtOAc/$CH_2Cl_2$) to give the title compound (1.64 g, 69%) as a white solid. $^1H$ NMR (400 MHz, DMSO-$d_6$) δ 9.15 (d, *J*=2.0 Hz, 1H), 8.88 (d, *J*=2.2 Hz, 1H), 8.17 - 8.02 (m, 2H), 7.78 - 7.63 (m, 2H), 3.90 (s, 3H).

**Step 2: Methyl 3-bromo-5*H*-pyrido[3,2-*b*]indole-7-carboxylate**

A mixture of methyl 4-(5-bromo-3-nitropyridin-2-yl)benzoate (1.64 g, 4.86 mmol) and 1,2-bis(diphenylphosphino)ethane (2.42 g, 6.08 mmol) in 1,2-dichlorobenzene (30 mL) was purged with $N_2$ (3x) and then warmed to reflux. After 4 h, the mixture was cooled to room temperature and concentrated. The residue was suspended in $CHCl_3$, sonicated and then filtered. The solid was washed with $CHCl_3$ and dried to give the title compound (0.84 g, 57%) as a beige solid. $^1H$ NMR (400 MHz, DMSO-$d_6$) δ 11.85 (s, 1H), 8.60 (d, *J*=2.0 Hz, 1H), 8.29 (dd, *J*=5.1, 3.1 Hz, 2H), 8.22 (s, 1H), 7.88 (dd, *J*=8.3, 1.4 Hz, 1H), 3.93 (s, 3H); LCMS (M+H) = 305.1.

**Step 3: Methyl 3-bromo-5-((4-fluorophenyl)(tetrahydro-2*H*-pyran-4-yl)methyl)-5*H*-pyrido[3,2-*b*]indole-7-carboxylate**

Following a procedure analogous to that described in Step 4 Reference Example 1, methyl 3-bromo-5*H*-pyrido[3,2-*b*]indole-7-carboxylate (840 mg, 2.75 mmol) and (4-fluorophenyl)(tetrahydro-2*H*-pyran-4-yl)methanol (Step 1 of Reference Example 3, 868 mg, 4.13 mmol) was converted to the title compound as a racemate (1.26 g, 92%), which was used without further purification in the subsequent step. LCMS (M+H) = 497.2.

**Step 4: Methyl 5-((4-fluorophenyl)(tetrahydro-2*H*-pyran-4-yl)methyl)-3-(4,4,5,5-tetramethyl-1,3,2-dioxaborolan-2-yl)-5*H*-pyrido[3,2-*b*]indole-7-carboxylate**

To a mixture of methyl 3-bromo-5-((4-fluorophenyl)(tetrahydro-2*H*-pyran-4-yl)methyl)-5*H*-pyrido[3,2-*b*]indole-7-carboxylate (500 mg, 1.01 mmol), 4,4,4',4',5,5,5',5'-octamethyl-2,2'-bi(1,3,2-dioxaborolane) (306 mg, 1.21 mmol), Pd(dppf)$Cl_2$ (37 mg, 0.050 mmol) and KOAc (197 mg, 2.01 mmol) in a screw cap vial was added dioxane (10 mL). The vial was fitted with a teflon lined septum cap and purged with $N_2$ (3x). The reaction mixture was heated at 90 °C for 16 h, then cooled to room temperature and filtered. The filtrate was purified using ISCO silica gel chromatography (40 g column, gradient from 0% to 90% EtOAc/$CH_2Cl_2$) to give the title compound (254 mg, 46%). LCMS (M+H) = 463.4.

**Step 5: Methyl 5-((4-fluorophenyl)(tetrahydro-2*H*-pyran-4-yl)methyl)-3-(5-methylisoxazol-4-yl)-5*H*-pyrido[3,2-*b*]indole-7-carboxylate**

To a screw top vial was added methyl 5-((4-fluorophenyl)(tetrahydro-2*H*-pyran-4-yl)methyl)-3-(4,4,5,5-tetramethyl-1,3,2-dioxaborolan-2-yl)-5*H*-pyrido[3,2-*b*]indole-7-carboxylate (125 mg, 0.23 mmol), 4-iodo-5-methylisoxazole (48 mg, 0.23 mmol), Pd(dppf)$Cl_2$ (8 mg, 0.011 mmol) and phosphoric acid, potassium salt (0.23 mL, 0.69 mmol) followed by THF (1.5 mL). The resulting suspension was heated at 80 °C for 45 min, then cooled to room temperature, diluted with EtOAc and quenched with water. The organic layer was washed with water, sat. NaCl, dried and concentrated *in vacuo.* The crude product was purified using ISCO silica gel chromatography (24 g column, gradient from 0% to 100% EtOAc/$CH_2Cl_2$) to give the title compound (83 mg, 72%). LCMS (M+H) = 500.1; HPLC RT = 3.68 min (Column: Waters Sunfire C18 5 μm, 2.1 x 50 mm; Mobile Phase A: 10:90 MeOH:water with 0.1% TFA; Mobile Phase B: 90:10 MeOH:water with 0.1% TFA; Gradient 0-100% B over 4 min; Flow: 1 mL/min; Detection: UV at 220 nm).

**Step 6: 2-{5-[(4-Fluorophenyl)(oxan-4-yl)methyl]-3-(5-methyl-1,2-oxazol-4-yl)-5*H*-pyrido[3,2-*b*]indol-7-yl}propan-2-ol**

Following a procedure analogous to that described in Step 5 Reference Example 1, methyl 5-((4-fluorophenyl)(tetrahydro-2*H*-pyran-4-yl)methyl)-3-(5-methylisoxazol-4-yl)-5*H*-pyrido[3,2-*b*]indole-7-carboxylate (105 mg, 0.21 mmol) was converted to racemic 2-{5-[(4-fluorophenyl)(oxan-4-yl)methyl]-3-(5-methyl-1,2-oxazol-4-yl)-5*H*-pyrido[3,2-*b*]indol-7-yl}propan-2-ol, (60 mg, 57%) which was separated by chiral prep SFC to give Enantiomers A and B. Enantiomer A: $^1$H NMR (400 MHz, $CD_3OD$) δ 8.75 (s, 1H), 8.52 (d, *J*=1.8 Hz, 1H), 8.34 - 8.13 (m, 2H), 8.05 (s, 1H), 7.79 - 7.62 (m, 2H), 7.46 (dd, *J*=8.4, 1.3 Hz, 1H), 7.10 (t, *J*=8.8 Hz, 2H), 5.76 (d, *J*=11.0 Hz, 1H), 4.19 - 3.75 (m, 3H), 3.68 - 3.54 (m, 1H), 3.50 - 3.35 (m, 2H), 2.64 (s, 2H), 2.08 - 1.86 (m, 1H), 1.86 - 1.74 (m, 1H), 1.74 - 1.53 (m, 6H), 1.53 - 0.99 (m, 4H); LCMS (M+H) = 500.5; SFC RT = 7.68 (Column: Chiralcel OD-H 250 x 4.6 mm, 5 μm; Mobile Phase: 75/25 $CO_2$/MeOH; Flow: 2 mL/min); $[\alpha]_D^{20}$ = -79.08 (c = 0.11, MeOH). Enantiomer B: $^1$H NMR (400 MHz, $CD_3OD$) δ 8.75 (s, 1H), 8.52 (d, *J*=1.5 Hz, 1H), 8.26 (s, 2H), 8.12 - 7.90 (m, 1H), 7.81 - 7.60 (m, 2H), 7.58 - 7.34 (m, 1H), 7.10 (s, 2H), 5.88 - 5.64 (m, 1H), 4.15 - 3.74 (m, 3H), 3.72 - 3.55 (m, 1H), 3.37 (s, 2H), 2.64 (s, 2H), 2.10 - 1.90 (m, 1H), 1.68 (d, *J*=3.7 Hz, 6H), 1.51 - 1.26 (m, 2H), 1.25 - 1.01 (m, 2H); LCMS (M+H) = 500.5; SFC RT = 10.51 (Column: Chiralcel OD-H 250 x 4.6 mm, 5 μm; Mobile Phase: 75/25 $CO_2$/MeOH; Flow: 2 mL/min).

**Example 1 & Reference Example 7**

**2-[3-(Dimethyl-1*H*-1,2,3-triazol-5-yl)-5-[oxan-4-yl(phenyl)methyl]-5*H*-pyrido[3,2-*b*]indol-7-yl]propan-2-ol**

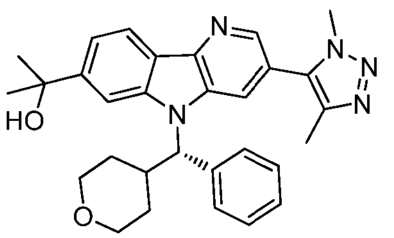

**Enantiomer A, Example 1**

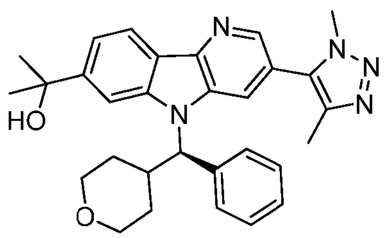

**Enantiomer B, Ref. Example 7**

**Step 1: 2-Chloro-5-(1,4-dimethyl-1*H*-1,2,3-triazol-5-yl)pyridin-3-amine**

To a 100 mL round bottom flask containing 5-bromo-2-chloropyridin-3-amine (2.90 g, 14.0 mmol), 1,4-dimethyl-5-(tributylstannyl)-1*H*-1,2,3-triazole (2.70 g, 6.99 mmol) [Seefeld, M.A. et al. PCT Int. Appl., 2008, WO2008098104] and $Pd(PPh_3)_4$ (0.61 g, 0.52 mmol) in DMF (20 mL) was added cuprous iodide (0.20 g, 1.05 mmol) and $Et_3N$ (1.9 mL, 14.0 mmol). The reaction mixture was purged with $N_2$ for 3 min and then heated at 100 °C for 1 h. After cooling to room temperature, the mixture was diluted with 10% LiCl solution and extracted with EtOAc (2x). The combined organics were washed with sat. NaCl, dried over $MgSO_4$, filtered and concentrated. $CH_2Cl_2$ was added, and the resulting precipitate was collected by filtration. The mother liquor was concentrated and purified using ISCO silica gel chromatography (40 g column, gradient from 0% to 100% EtOAc/$CH_2Cl_2$). The resulting solid was combined with the precipitate and triturated with cold EtOAc to give the title compound (740 mg, 47%) as a light tan solid. LCMS (M+H) = 224.1; HPLC RT = 1.03

min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 2: Methyl 3-((2-chloro-5-(1,4-dimethyl-1*H*-1,2,3-triazol-5-yl)pyridin-3-yl)amino)benzoate**

Following a procedure analogous to that described in Step 2 of Reference Example 1, 2-chloro-5-(1,4-dimethyl-1*H*-1,2,3-triazol-5-yl)pyridin-3-amine (740 mg, 3.31 mmol) was converted to the title compound (644 mg, 54%). $^1$H NMR (400 MHz, $CDCl_3$) δ 7.94 (t, *J*=1.9 Hz, 1H), 7.88 (d, *J*=2.1 Hz, 1H), 7.83 (dt, *J*=7.8, 1.3 Hz, 1H), 7.49 (t, *J*=7.9 Hz, 1H), 7.40 (d, *J*=2.1 Hz, 1H), 7.36 (ddd, *J*=8.0, 2.3, 0.9 Hz, 1H), 6.38 (s, 1H), 3.99 (s, 3H), 3.93 (s, 3H), 2.34 (s, 3H); LCMS (M+H) = 358.2; HPLC RT = 2.34 min (Column: Chromolith ODS S5 4.6 x 50 mm; Mobile Phase A: 10:900 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 3: Methyl 3-(1,4-dimethyl-1*H*-1,2,3-triazol-5-yl)-5*H*-pyrido[3,2-*b*]indole-7-carboxylate**

Following a procedure analogous to that described in Step 3 of Reference Example 1, methyl 3-((2-chloro-5-(1,4-dimethyl-1*H*-1,2,3-triazol-5-yl)pyridin-3-yl)amino)benzoate (2.82 g, 7.88 mmol) was converted to the title compound (1.58 g, 62%). $^1$H NMR (500 MHz, DMSO-$d_6$) δ 11.93 (s, 1H), 8.62 (d, *J*=1.8 Hz, 1H), 8.36 (dd, *J*=8.2, 0.6 Hz, 1H), 8.29 - 8.22 (m, 1H), 8.16 (d, *J*=1.8 Hz, 1H), 7.91 (dd, *J*=8.2, 1.4 Hz, 1H), 4.02 (s, 3H), 3.94 (s, 3H), 2.31 (s, 3H); LCMS (M+H) = 322.3; HPLC RT = 1.98 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Alternate synthesis of Methyl 3-(1,4-dimethyl-1*H*-1,2,3-triazol-5-yl)-5*H*-pyrido[3,2-*b*]indole-7-carboxylate**

A mixture of methyl 3-bromo-5H-pyrido[3,2-b]indole-7-carboxylate (Step 2 of Reference Example 5, 3.000 g, 9.83 mmol), 1,4-dimethyl-5-(tributylstannyl)-1H-1,2,3-triazole (4.18 g, 10.82 mmol), copper (I) iodide (0.281 g, 1.475 mmol), $Pd(Ph_3P)_4$ (0.738 g, 0.639 mmol) and triethylamine (2.74 mL, 19.66 mmol) in DMF (25 mL) was purged under a nitrogen stream and then heated in a heating block at 95 °C for 2 hours. After cooling to room temperature the reaction mixture was diluted with water and extracted into ethyl acetate. Washed with water, $NH_4OH$, brine and concentrated. The residue was triturated with 100 mL $CHCl_3$, filtered off the solid and rinsed with $CHCl_3$ to give. 1.6 g of product. The filtrate was loaded unto the ISCO column (330 g column, A: DCM; B: 10%MeOH/DCM, 0 to 100% gradient) and chromatographed to give an additional 0.7 g. of methyl 3-(1,4-dimethyl-1H-1,2,3-triazol-5-yl)-5H-pyrido[3,2-b]indole-7-carboxylate (2.30 g total, 7.16 mmol, 72.8 % yield).

**Step 4: Methyl 3-(1,4-dimethyl-1*H*-1,2,3-triazol-5-yl)-5-(phenyl(tetrahydro-2*H*-pyran-4-yl)methyl)-5H-pyrido[3,2-*b*]indole-7-carboxylate**

Following a procedure analogous to that described in Step 4 of Reference Example 1, methyl 3-(1,4-dimethyl-1*H*-1,2,3-triazol-5-yl)-5*H*-pyrido[3,2-*b*]indole-7-carboxylate (80 mg, 0.25 mmol) was converted to the title compound (65 mg, 53%) after purification by prep HPLC (Column: Phen Luna C18, 30 x 100 mm, 5 μm particles; Mobile Phase A: 5:95 acetonitrile:water with 0.1% TFA; Mobile Phase B: 95:5 acetonitrile:water with 0.1% TFA; Gradient: 10-100% B over 14 min, then a 2-min hold at 100% B; Flow: 40 mL/min). $^1$H NMR (400 MHz, $CDCl_3$) δ 8.51 (d, *J*=1.8 Hz, 1H), 8.50 (s, 1H), 8.47 (d, *J*=8.1 Hz, 1H), 8.10 (dd, *J*=8.1, 1.1 Hz, 1H), 7.63 (d, *J*=1.8 Hz, 1H), 7.46 (d, *J*=7.3 Hz, 2H), 7.40 - 7.30 (m, 3H), 5.62 (d, *J*=10.6 Hz, 1H), 4.11 - 4.03 (m, 4H), 3.92 - 3.83 (m, 4H), 3.56 (td, *J*=11.9, 1.8 Hz, 1H), 3.35 (td, *J*=11.9, 1.9 Hz, 1H), 3.18 - 3.05 (m, 1H), 2.30 (s, 3H), 2.04 (d, *J*=13.0 Hz, 1H), 1.71 - 1.58 (m, 1H), 1.50 - 1.37 (m, 1H), 1.09 (d, *J*=12.8 Hz, 1H); LCMS (M+H) = 496.3; HPLC RT = 2.93 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 5: 2-[3-(Dimethyl-1*H*-1,2,3-triazol-5-yl)-5-[oxan-4-yl(phenyl)methyl]-5*H*-pyrido[3,2-*b*]indol-7-yl]propan-2-ol,**

Following a procedure analogous to that described in Step 5 of Reference Example 1, methyl 3-(1,4-dimethyl-1*H*-1,2,3-triazol-5-yl)-5-(phenyl(tetrahydro-2*H*-pyran-4-yl)methyl)-5H-pyrido[3,2-b]indole-7-carboxylate (65 mg, 0.13 mmol) was converted to racemic 2-[3-(dimethyl-1*H*-1,2,3-triazol-5-yl)-5-[oxan-4-yl(phenyl)methyl]-5*H*-pyrido[3,2-*b*]indol-7-yl]propan-2-ol, which was separated by chiral prep SFC (Column: Chiralpak IB 25 x 2 cm, 5 μm; Mobile Phase: 70/30 $CO_2$/MeOH; Flow: 50 mL/min);to give Enantiomer A (24 mg, 36%) and Enantiomer B (26 mg, 38%). Enantiomer A: $^1$H NMR (500 MHz, $CDCl_3$) δ 8.44 (d, *J*=1.8 Hz, 1H), 8.36 (d, *J*=8.2 Hz, 1H), 7.98 (s, 1H), 7.56 (d, *J*=1.7 Hz, 1H), 7.47 -

7.41 (m, 3H), 7.37 - 7.32 (m, 2H), 7.31 - 7.28 (m, 1H), 5.59 (d, *J*=10.5 Hz, 1H), 4.06 (dd, *J*=11.8, 2.8 Hz, 1H), 3.90 - 3.84 (m, 4H), 3.55 (td, *J*=11.9, 2.0 Hz, 1H), 3.35 (td, *J*=11.9, 2.0 Hz, 1H), 3.15 - 3.04 (m, 1H), 2.30 (s, 3H), 2.04 (d, *J*=13.6 Hz, 1H), 1.92 (s, 1H), 1.75 (s, 6H), 1.69 - 1.58 (m, 1H), 1.47 - 1.38 (m, 1H), 1.12 (d, *J*=13.4 Hz, 1H); LCMS (M+H) = 496.4; HPLC RT = 2.46 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min). SFC RT = 5.50 min (Column: Chiralpak IB 250 x 4.6 mm, 5 μm; Mobile Phase: 70/30 $CO_2$/MeOH; Flow: 2 mL/min); SFC RT = 1.06 min (Column: Chiralcel OD-H 250 x 4.6 mm, 5 μm; Mobile Phase: 50/50 $CO_2$/(1:1 MeOH/CH3CN); Flow: 2 mL/min); $[\alpha]_D^{20}$ = -117.23 (c = 0.08, $CHCl_3$). Enantiomer B: $^1H$ NMR (500 MHz, $CDCl_3$) δ 8.44 (d, *J*=1.8 Hz, 1H), 8.36 (d, *J*=8.2 Hz, 1H), 7.98 (s, 1H), 7.56 (d, *J*=1.7 Hz, 1H), 7.47 - 7.41 (m, 3H), 7.37 - 7.32 (m, 2H), 7.31 - 7.28 (m, 1H), 5.59 (d, *J*=10.5 Hz, 1H), 4.06 (dd, *J*=11.8, 2.8 Hz, 1H), 3.90 - 3.84 (m, 4H), 3.55 (td, *J*=11.9, 2.0 Hz, 1H), 3.35 (td, *J*=11.9, 20 Hz, 1H), 3.15 - 3.04 (m, 1H), 2.30 (s, 3H), 2.04 (d, *J*=13.6 Hz, 1H), 1.92 (s, 1H), 1.75 (s, 6H), 1.69 - 1.58 (m, 1H), 1.47 - 1.38 (m, 1H), 1.12 (d, *J*=13.4 Hz, 1H); LCMS (M+H) = 496.4; HPLC RT = 2.46 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min). SFC RT = 8.30 min (Column: Chiralpak IB 250 x 4.6 mm, 5 μm; Mobile Phase: 70/30 $CO_2$/MeOH; Flow: 2 mL/min); SFC RT = 2.83 min (Column: Chiralcel OD-H 250 x 4.6 mm, 5 μm; Mobile Phase: 50/50 $CO_2$/(1:1 MeOH/CH3CN); Flow: 2 mL/min); $[\alpha]_D^{20}$ = +88.78 (c = 0.10, $CHCl_3$).

**Alternate Synthesis of Example 1**

**2-(3-(Dimethyl-1*H*-1,2,3-triazol-5-yl)-5-[oxan-4-yl(phenyl)methyl]-5*H*-pyrido[3,2-*b*]indol-7-yl]propan-2-ol.**

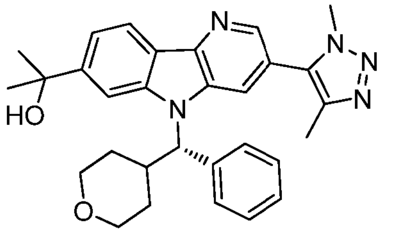

**Enantiomer A, Example 1**

**Step 1: (S)-methyl 3-(1,4-dimethyl-1H-1,2,3-triazol-5-yl)-5-(phenyl(tetrahydro-2H-pyran-4-yl)methyl)-5H-pyrido[3,2-b]indole-7-carboxylate**

The enantiomers of phenyl(tetrahydro-2*H*-pyran-4-yl)methanol (2.0 g, 10.4 mmol) [Orjales, A. et al. J. Med. Chem. 2003, 46, 5512-5532], were separated on preperative SFC. (Column: Chiralpak AD 5 x 25 cm, 5 μm; Mobile Phase: 74/26 $CO_2$/MeOH; Flow: 270 mL/min; Temperature 30°C). The separated peaks were concentrated and dried under vacuum to give white solids. Enantiomer A: (S)-phenyl(tetrahydro-2H-pyran-4-yl)methanol: (0.91 g, 45.5%) SFC RT = 2.32 min (Column: Chiralpac AD 250 x 4.6 mm, 5 μm; Mobile Phase: 70/30 $CO_2$/MeOH; Flow: 3 mL/min); Temperature 40°C. Enantiomer B: (R)-phenyl(tetrahydro-2H-pyran-4-yl)methanol. (0.92 g, 46%) SFC RT = 3.09 min (Column: Chiralpac AD 250 x 4.6 mm, 5 μm; Mobile Phase: 70/30 $CO_2$/MeOH; Flow: 3 mL/min); Temperature 40°C.

Following a procedure analogous to that described in Step 4 of Reference Example 1 except using toluene (120mL) as the solvent, methyl 3-(1,4-dimethyl-1H-1,2,3-triazol-5-yl)-5H-pyrido[3,2-b]indole-7-carboxylate (4 g, 12.45 mmol) and (R)-phenyl(tetrahydro-2H-pyran-4-yl)methanol (Enantiomer B above, 5.86 g, 30.5 mmol) was converted to the title compound (5.0 g, 81%). HPLC RT = 2.91 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min).

**Step 2. (S)-2-[3-(Dimethyl-1H-1,2,3-triazol-5-yl)-5-[oxan-4-yl(phenyl)methyl]-5H-pyrido[3,2-b]indol-7-yl]propan-2-ol**

A 500 mL round bottom flask containing (S)-methyl 3-(1,4-dimethyl-1*H*-1,2,3-triazol-5-yl)-5-(phenyl(tetrahydro-2*H*-pyran-4-yl)methyl)-5H-pyrido[3,2-b]indole-7-carboxylate (5.0 g, 10.09 mmol) in THF (150 mL) was cooled in an ice/MeOH bath. MeMgBr, (3M in $Et_2O$, 17.0 mL, 51.0 mmol) was added slowly over 4 min. The resulting solution was

stirred for 2 h and then quenched carefully with sat. $NH_4Cl$. The reaction mixture was diluted with 10% LiCl solution extracted with EtOAc. The organic layer was dried over $MgSO_4$, filtered and concentrated. The crude material was purified using ISCO silica gel chromatography (120 g column, gradient from 0% to 6% MeOH/$CH_2Cl_2$). The product was collected and concentrated then dissolved in hot MeOH(35mL). To the mixture was added 15mL water and the mixture was cooled to room temperature. The resulting white precipitate was collected by filtration with 2:1 MeOH/water rinse then dried under vacuum to give the title compound (3.2 g, 62%). $^1H$ NMR (500 MHz, $CDCl_3$) $\delta$ 8.40 (d, *J*=1.8 Hz, 1H), 8.33 (d, *J*=8.2 Hz, 1H), 7.93 (s, 1H), 7.53 (d, *J*=1.8 Hz, 1H), 7.46 (d, *J*=7.3 Hz, 2H), 7.42 (dd, *J*=8.2, 1.4 Hz, 1H), 7.37 - 7.31 (m, 2H), 7.30 - 7.28 (m, 1H), 5.56 (d, *J*=10.5 Hz, 1H), 4.06 (d, *J*=8.9 Hz, 1H), 3.89 - 3.83 (m, 1H), 3.55 (td, *J*=I1.9, 2.1 Hz, 1H), 3.35 (td, *J*=I1.9, 2.1 Hz, 1H), 3.10 (q, *J*=10.8 Hz, 1H), 2.39 (s, 3H), 2.23 (s, 3H), 2.03 (d, *J*=14.2 Hz, 1H), 1.89 (s, 1H), 1.74 (s, 6H), 1.68 - 1.59 (m, 1H), 1.46 - 1.36 (m, 1H), 1.12 (d, *J*=12.2 Hz, 1H); LCMS (M+H) = 496.3; HPLC RT = 2.44 min (Column: Chromolith ODS S5 4.6 x 50 mm; Mobile Phase A: 10:90 MeOH:water with 0.1% TFA; Mobile Phase B: 90:10 MeOH:water with 0.1% TFA; Temperature: 40 °C; Gradient: 0-100% B over 4 min; Flow: 4 mL/min); SFC RT = 2.01 min (Column: Chiralcel OD-H 250 x 4.6 mm, 5 $\mu$m; Mobile Phase: 60/40 $CO_2$/(1:1 MeOH/CH3CN); Flow: 2 mL/min). SFC RT = 1.06 min (Column: Chiralcel OD-H 250 x 4.6 mm, 5 $\mu$m; Mobile Phase: 50/50 $CO_2$/(1:1 MeOH/CH3CN); Flow: 2 mL/min).

EVALUATION OF BIOLOGICAL ACTIVITY

Exemplary compounds were tested for inhibition of BRD2, BRD3, BRD4 and BRDT activity. Experimental procedures and results are provided below.

Cloning, Expression, and Purification of Human Bromodomains for Thermal Shift Assays (TSA)

Recombinant DNA clones encoding bromodomains of human proteins were optimized for E. coli expression, chemically synthesized (GenScript, Piscataway NJ), and inserted into a modified pET28 expression vector to construct tobacco vein mottling virus (TVMV) protease cleavable N-terminal hexahistidine fusions. The non-native amino acids (MGSSHHHHHHSSGETVRFQSM) (SEQ ID NO: 1) were immediately followed by bromodomain proteins with the amino acid residue sequences (followed by accessions referenced from and numbered according to the Uniprot Knowledgebase; Uniprot Consortium; www.uniprot.org) as follows:

CECR2(420-543), Q9BXF3-1; FALZ(2917-3037), Q12830-1; GCN5(731-837), Q92830-1; PCAF(715-831), Q92831-1; BRD2(24-472), P25440-1; BRD3(1-434), Q15059-1; BRD4(44-168), BRD4(333-460), BRD4(44-460), 060885-1; BRDT(1-383), Q58F21-1; BAZ1B(1340-1457), Q9UIG0-1; CREBBP(1081-1197), Q92793-1; EP300(1040-1161), Q09472-1; WDR9(1310-1430), Q9NSI6-1; ATAD2(981-1108), Q6PL18-1; BRD1(556-688), O95696-1; BRD7(129-236), Q9NPI1-1; BRD9(134-239), Q9H8M2-1; BRPF1(626-740), P55201-2; ATAD2B(952-1086), Q9ULI0-1; BAZ2B(2054-2168), Q9UIF8-1; SP140L(400-580), Q9H930-4; SP140(687-862), Q13342-1; TIF1(896-1014), O15164-1; TRIM28(619-805), Q13263-1; BRWD3(1295-1443), Q6RI45-1; TAF1(1377-1503), TAF1(1501-1635), P21675-1; TAF1L(1402-1522), TAF1L(1523-1654), Q8IZX4-1; ASH1L(2433-2564), Q9NR48-1; PB1(43-156), PB1(178-291), PB1(388-494), PB1(645-766), PB1(773-917), Q86U86-1; SMARCA2(1367-1511), P51531-1; SMARCA2-2(1367-1493), P51531-2.

The recombinant vectors were transformed into E. coli BL21(DE3). The transformed cells were cultured in 1L terrific broth in 2.5L Thomson Ultra Yield shaker flasks at 37°C, 230 rpm and, at a cell density of OD600nm = 1.0, were induced with 0.5 mM IPTG and incubated in the shaker at 20°C for 16-18 hours. The cell pellets were harvested by sedimentation and lysed by sonication in buffer containing 0.1 mg/ml lysozyme. Each sample was clarified by sedimentation, and the supernatant was loaded onto a HisTrap affinity column (GE Healthcare Life Sciences). The column was washed and then eluted with an imidazole gradient. The peak protein fractions containing the bromodomain protein were pooled, concentrated, and the protein was purified further by size exclusion chromatography on a Superdex 200 column (GE Healthcare Life Sciences) equilibrated with the final storage buffer (20 mM Tris-HCl pH 8.0, 200 mM NaCl, 5% glycerol, 2 mM DTT). The SEC peak fractions containing purified protein at 2-5 mg/ml were pooled, and the pool was divided into aliquots, flash frozen in liquid nitrogen, and store at -80°C.

**Cloning, Expression, and Purification of Biotinylated Human Bromodomains for TR-FRET Assays**

Recombinant DNA clones encoding bromodomains of human BRD2, BRD3, BRD4 and BRDT were optimized for E. coli expression, chemically synthesized (GenScript, Piscataway NJ), and inserted into a modified pET28 expression vector to construct tobacco vein mottling virus (TVMV) protease cleavable N-terminal hexahistidine fusions followed by a site specific biotinylation motif recognized by E. coli biotin ligase (BirA). The non-native amino acids (MGSSHHHHHHSSGETVRFQGLNDIFEAQKIEWHEDTGHM) (SEQ ID NO: 2) were immediately followed by bromodomain constructs of BRD4 with the amino acid residue sequences (followed by the BRD4 accession referenced from and numbered according to the Uniprot Knowledgebase; Uniprot Consortium; www.uniprot.org) as follows: BRD4(44-168), BRD4(333-460), BRD4(44-460), BRD4(1-477), 060885-1.

Each of the recombinant vectors were co-transformed into E. coli BL21 STAR (DE3) together with a plasmid encoding BirA under chloramphenicol selection. The transformed cells were cultured at 37°C in 2.5 L Thomson Ultra Yield shaker flasks containing 1L M9-CAS medium (Teknova) supplemented with 40 μg/ml kanamycin, 35 μg/ml chloramphenicol, and 100 μM biotin. At a cell density corresponding to an OD600nm = 0.6, the cultures were induced with 0.5 mM IPTG and incubated in the shaker for an additional 20 hours at 20°C. The cell pellets were harvested by sedimentation and lysed by sonication in buffer containing 0.1 mg/ml lysozyme. Each sample was clarified by sedimentation, and the supernatant was loaded onto a HisTrap affinity column. The column was washed and then eluted with an imidazole gradient. The peak protein fractions containing the bromodomain protein were pooled and incubated for 18 hours at 4°C with purified His-TVMV protease (1:15 mass ratio of TVMV:BRD4 protein). The sample was exchanged into low imidazole buffer and passed through a HisTrap column to capture the cleaved His-tag and His-TVMV enzyme. The protein in the HisTrap column flow through was futher purified and exchanged into the final storage buffer (PBS pH 7.0, 5% Glycerol, 1 mM DTT) by size exclusion chromatography on a Superdex 200 column. To improve purity, the BRD4(1-477) and BRD4(44-460) proteins were subjected to an additional cation exchange chromatography purification step prior to size exclusion chromatography. Essentially quantitative mono-biotinylation (+226 Da) of each protein was confirmed by electrospray ionization mass spectrometry analysis on the final sample. The purified samples were divided into aliquots, flash frozen in liquid nitrogen, and stored at -80°C.

**Time Resolved Fluorescence Resonance Energy Transfer (TR-FRET) assay**

The binding of compounds to bromodomain BRD4 (44-168), BRD4 (333-460), and BRD4 (1-477 or 44-460) was assessed using a time resolved fluorescent resonance energy transfer binding assay (1), that measures the binding of a fluorescently labeled probe molecule to the bromodomain protein. The bromodomain protein, fluorescent probe molecule (either a biotinylated histone peptide or a fluorescently labeled small molecule), and dose-responsed test compound are incubated together to reach thermodynamic equilibrium. In the absence of a test compound, the bromodomain and small molecule are bound, resulting in a high fluorescent signal. In the presence of a sufficient concentration of inhibitor, this intercation is disrupted resulting in a lost of fluorescent resonance energy transfer.

All assay components were dissolved in buffer composition 20 mM Hepes pH 7.5, 150 mM NaCl, 5 mM DTT, 0.005% Tween 20, and 100 ug/ml BSA for BRD4 (1-477 and 44-460). The final concentrations of the bromodomain proteins are 1.6 nM BRD4(44-168), 1 nM BRD4(333-460), and 1 nM BRD4(1-477 or 44-460), and the fluorescent probe molecule is 100 nM, 50 nM, and 7.5 nM respectively. All proteins were biotinylated. A streptavidin labeled with terbium cryptate (Cisbio SA-Tb) was used as detection, and pre-mixed with the bromodomain protein at a final concentration of 0.2 nM. In some instances for BRD4 (44-460), anti-His terbium cryptate was used as a detection. 7.5 nl of dose-responsed test compound or dmso vehicle (0.0375 %) was pre-spotted in a black Corning 384 well plate and 10 ul each of bromodomain/ detection reagent and fluorescent small molecule solution were added to the plate, and the reaction incubated for 60 min at room temperature. Plates were then read on EnVision plate reader, (λex =340 nm, acceptor λEm=520 nm, and donor λEm = 615 nm, LANCE D400 mirror). Time resolved fluoresence intensity measurements were made at both emissions, and the ratio of acceptor/ donor was calcuated and used for data analysis. All data was normalized to 16 high vehicle wells and 8 low reference control wells, and then a four parameter curve fit was applied:

$$Y=a+((b-a)/(1+(10x/10c)d))$$

Where 'a' is the minimum, 'b' is the Hill slope, 'c' is the IC50, and 'd' is the maximum. Histone peptide: Purchased from GenScript
H4K5K8K12K16
Biotin-AHA-SGRGK(Ac)GGK(Ac)GLGK(Ac)GGAK(Ac)RHRKV (SEQ ID NO: 3)

The fluorescently labeled small molecule used was a BRD4 inhibitor known in the art

1. F.Degorce, A.Card, S.Soh, E. Trinquet, G. P. Knapik and B. Xie, HTRF: A technology tailored for drug discovery - a review of theoretical aspects and recent applications. Current Chemical Genomics (2009) 3, 22-32

**Thermal Shift Assay**

The effect of compound binding on the thermal stability of the bromodomains was measured using a BioRad CFX real time PCR instrument by monitoring the fluorescence enhancement of an external probe (SYPRO orange) as it binds preferentially to the unfolded protein. The unfolding reactions were carried out in a 384-well plate in a 4uL volume with 2-8uM of bromodomain protein, 1-2% (v/v) DMSO in buffer containing 10 mM Hepes, pH 7.4, 500 mM NaCl. SYPRO

orange dye was added at a dilution of 1:500. Compound concentrations ranged from 1.6-100uM. Unfolding reactions were monitored by first equilibrating the instrument at 25 °C for 2.4 sec, followed by ramping the temperature in 0.5 °C increments from 25 to 95 °C with 60 s equilibration prior to a read at each temperature. Excitation and emission filters for the SYPRO orange dye were set to FRET with the excitation range from 450-490 nm and the emission range from 560-580 nm. The midpoint temperature was determined by calculating the inflection point using the second derivative. The observed temperature shifts were recorded as the difference between the midpoint between a reference well containing protein with dmso but no ligand and a well containing protein with compound.

The thermal shift assay is a biophysical technique that compares the change in unfolding transition temperature of a protein obtained in the presence and absence of a ligand (1). Typically, a fluorescent dye is used to monitor the protein unfolding as the protein is heated. During the unfolding process, hydrophobic regions of the protein are exposed, resulting in an increase in the dye binding and an increase in fluorescence intensity. The midpoint of the protein unfolding transition is defined as the Tm. A ligand that binds to the protein causes an increase in the protein thermal stability, thus increasing the Tm, proportionally to both the ligand concentration and its binding affinity.

1. M.W. Pantoliano, E.C. Petrella, J.D. Kwasnoski, V.S. Lobanov, J. Myslik, E.Graf, T. Carver, E. Asel, B.A. Springer, P. Lane, F.R. Salemme, High-density miniaturized thermal shift assays as a general strategy for drug discovery. J. Biomol. Screen 6(2001) 429-440.
2. M.D. Cummings, M. A. Famum, M. I. Nelen, Universal screening methods and application of ThermoFluor. J. Biomol. Screen 11 (2006) 854-863

**MYC HCS Assay**

Tumor cells in complete RPMI growth media (Gibco, 11875-085) supplemented with 10% FBS were harvested and plated into 384 black clear-bottom PDL cell culture plates in 30ul media with 10,000 cells per well. After compound treatment at 37C for 4 hrs, cells were fixed in 4% Formaldehyde at room temperature for 30 min and subsequently permeabilized. After washing and blocking, the plates were then incubated with anti-myc primary antibody 1:1000 (Cell Signaling Technology, 5605) at RT overnight. The following day, cells were washed and blocked before adding secondary antibody Alexa 488 Goat-anti Rabbit 1:2000 (Invitrogen, A11034) at RT in the dark for 1hr. Cells were subsequently washed and scanned on the Cellomics ArrayScan with 10x objective lens.

**MTS cell proliferation assay**

Tumor cells were plated at certain seeding densities in 384-well black clear bottom Matrix plates at 40ul per well and incubated overnight at 37°C in 5% $CO_2$ before assaying. On the next day, one set of cell plates (T0 plates) were used to determine time zero cell density, and 3-(4,5-dimethylthiazol-2-yl)-5-(3-carboxymethoxyphenyl)-2-(4-sulfophenyl)-2H-tetrazolium from the CellTiter 96 AQueous Non-Radioactive Cell proliferation Kit (Promega, G5440) was added at 4 μl/well into T0 plates followed by incubation at 37°C in 5% $CO_2$ for three hours. Absorbance at 490 nm was measured on an Envision reader (Perkin Elmer, Boston, MA). On the same day, the remaining cell plates (T72 plates) were treated with compounds at 37°C in 5% $CO_2$. After 72 hours, 4ul MTS reagents were then added onto those cell plates. The plates were further incubated at 37°C in 5% $CO_2$ for three hours and the absorbance values at A490 were measured on an Envision reader.

**Human Tumor Xenograft Models in Mice**

All rodents were obtained from Jackson Laboratory. (Bar Harbor, Maine), and maintained in an ammonia-free environment in a defined and pathogen -free colony. All mice were quarantined approximately 1 week prior to their use for tumor propagation and drug efficacy testing. Mice were fed food and water ad libitum. The animal care program of Bristol-Myers Squibb Pharmaceutical Research Institute is fully accredited by the American Association for Accreditation of Laboratory Animal Care (AAALAC). All experiments were performed in accordance with Bristol-Myers Squibb (BMS) animal test methods and guidelines.

Tumor xenografts were grown and maintained subcutaneously (SC) in NSG (NOD scid IL2 receptor gamma chain knockout) mice (Jackson Lab). Tumors were propagated as subcutaneous transplants using tumor fragments obtained from donor mice.

**Preclinical chemotherapy trials**

The required numbers of animals needed to detect a meaningful response were pooled at the start of the experiment and each was given bilateral subcutaneous implants of two tumor fragments (~ 20 mg) with a 13-gauge

trocar. Tumors were allowed to grow to the pre-determined size window (tumors outside the range were excluded) and animals were evenly distributed to various treatment and control groups. There were typically 6-8 mice per treatment and control groups, consisting of 10-12 tumors. Treatment of each animal was based on individual body weight. Treated animals were checked daily for treatment related toxicity/mortality. Each group of animals was weighed before the initiation of treatment ($Wt_1$) and then again following the last treatment dose ($Wt_2$). The difference in body weight ($Wt_2$-$Wt_1$) provides a measure of treatment-related toxicity.

Tumor response was determined by measurement of tumors with a caliper twice a week, until the tumors reached a predetermined "target" size of 0.5 gm or 1 gm depending on the tumor type. Tumor weights (mg) were estimated from the formula:

$$\text{Tumor weight} = (\text{length x width}^2) \div 2$$

Tumor response criteria are expressed in terms of tumor growth inhibition (%TGI). Tumor growth delay is defined as the difference in time (days) required for the treated tumors (T) to reach a predetermined target size compared to those of the control group (C). For this purpose, the tumor weight of a group is expressed as medium tumor weight (MTW).

Tumor growth inhibition is calculated as follows:

$$\%\ \text{Tumor Growth Inhibition} = \frac{\left(1 - \frac{T_t}{T_0} * \frac{C_0}{C_t}\right)}{\left(1 - \frac{C_0}{C_t}\right)}$$

where,

$C_t$ = Median control tumor size at end of treatment
$C_0$ = Median control tumor size at treatment initiation
$T_t$ = Median tumor size of treated group at end of treatment
$T_0$ = Median tumor size of treated group at treatment initiation

Activity is defined as the achievement of durable tumor growth inhibition of 50% or greater (i.e. TGI ≥ 50%) for a period equivalent to at least 1 tumor volume doubling time and drug treatment must be for a period equivalent to at least 2 tumor volume doubling time.

Tumor response was also expressed in terms of tumor growth delay and expressed as log cell kill (LCK value), defined as the difference in time (days) required for the treated tumors (T) to reach a predetermined target size compared to those of the control group (C).

Whenever possible, antitumor activity was determined at a range of dose levels up to the maximum tolerated dose (MTD) which is defined as the dose level immediately below which excessive toxicity (i.e. more than one death) occurred. When death occurred, the day of death was recorded. Treated mice dying prior to having their tumors reach target size were considered to have died from drug toxicity. No control mice died bearing tumors less than target size. Treatment groups with more than one death caused by drug toxicity were considered to have had excessively toxic treatments and their data were not included in the evaluation of a compound's antitumor efficacy.

Potential drug toxicity interaction affecting treatment tolerability is an important consideration in combination chemotherapy trials. Interpretation of combination therapeutic results must be based on comparison of antitumor activity of the best possible response for the single agents versus the combination at comparably tolerated doses. Therefore, therapeutic synergism was defined as a therapeutic effect achieved with a tolerated regimen of the combined agents that exceeded the optimal effect achieved at any tolerated dose of monotherapy. Statistical evaluations of data were performed using Gehan's generalized Wilcoxon test. Statistical significance was declared at $P < 0.05$.

**Drug Administration**

For administration of BET inhibitors to rodents, compounds were dissolved in 90% PEG300/10% TPGS/10% Ethanol. BET inhibitors were typically administered orally on a schedule of QDx7 or QDx10 (5 day-on-2 day-off), although other schedules had also been evaluated and shown to be efficacious

**Results:**

The following Table shows the results of certain compounds against the H187 Human Small Cell Carcinoma and the JJN3R multiple myeloma cell line.

| Ex. | Cell Line | Dose (mg/kg) | Treatment Schedule | % TGI | LCK |
|---|---|---|---|---|---|
| Ref. 1 | H187 | 15 | 2QDx7 | 104.0 | 1.2 |
| Ref. 1 | H187 | 5 | 2QDx7 | 88.0 | 1.0 |
| 1 | H187 | 10 | QDx7 | 102.0 | 0.9 |
| 1 | H187 | 5 | QDx7 | 100.0 | 0.7 |

The activity data shown below is based on the use of one of the FRET assays described. Compounds with an ICso less than 1500 nM are shown with (+), compounds with an ICso less than 25 nM are shown with (++) and those with an ICso less than 5 nM are shown with (+++).

| Example # | FRET BRD4 $IC_{50}$ (nM) |
|---|---|
| Ref. Example 1 | +++ |
| Ref. Example 2 | +++ |
| Ref. Example 3 | +++ |
| Ref. Example 4 | ++ |
| Ref. Example 5 | ++ |
| Ref. Example 6 | + |
| Example 1 | +++ |
| Ref. Example 7 | +++ |

## Claims

1. A compound of the formula

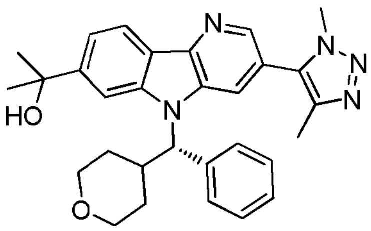

.

2. A pharmaceutically acceptable salt of a compound having the structure

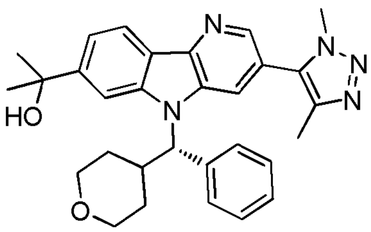

.

3. A pharmaceutical composition which comprises a compound according to claim 1 or a pharmaceutically acceptable

salt thereof and one or more pharmaceutically acceptable carriers, diluents or excipients.

4. The compound according to claim 1 for use in treating cancer in a patient wherein the cancer is small cell lung cancer, non-small cell lung cancer, colorectal cancer, multiple myeloma or AML.

5. The pharmaceutically acceptable salt according to claim 2 for use in treating cancer in a patient wherein the cancer is small cell lung cancer, non-small cell lung cancer, colorectal cancer, multiple myeloma or AML.

## Patentansprüche

1. Verbindung der Formel

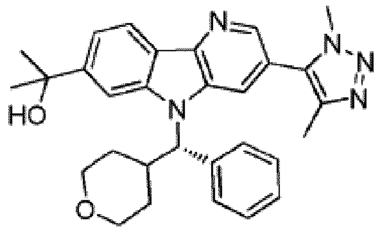

.

2. Pharmazeutisch verträgliches Salz einer Verbindung mit der Struktur

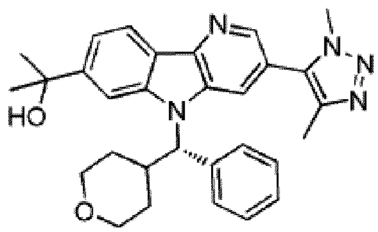

.

3. Pharmazeutische Zusammensetzung, die eine Verbindung nach Anspruch 1 oder ein pharmazeutisch verträgliches Salz davon und ein/-en oder mehrere pharmazeutisch verträgliche Träger, Verdünnungsmittel oder Hilfsstoffe umfasst.

4. Verbindung nach Anspruch 1, zur Verwendung in der Behandlung von Krebs bei einem Patienten, wobei der Krebs kleinzelliger Lungenkrebs, nicht kleinzelliger Lungenkrebs, Kolorektalkrebs, multiples Myelom oder AML ist.

5. Pharmazeutisch verträgliches Salz nach Anspruch 2, zur Verwendung in der Behandlung von Krebs bei einem Patienten, wobei der Krebs kleinzelliger Lungenkrebs, nicht kleinzelliger Lungenkrebs, Kolorektalkrebs, multiples Myelom oder AML ist.

## Revendications

1. Composé de formule

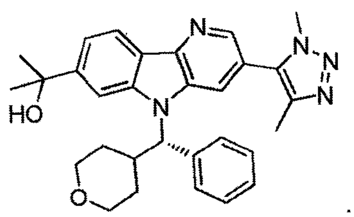

.

2. Sel pharmaceutiquement acceptable d’un composé ayant la structure

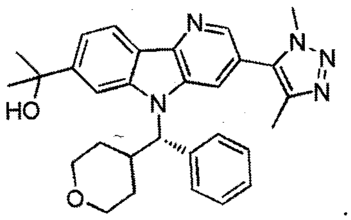

**3.** Composition pharmaceutique qui comprend un composé selon la revendication 1 ou un sel pharmaceutiquement acceptable de celui-ci et un ou plusieurs véhicules, diluants ou excipients pharmaceutiquement acceptables.

**4.** Composé selon la revendication 1, pour utilisation dans le traitement d'un cancer chez un patient, dans lequel le cancer est un cancer du poumon à petites cellules, un cancer du poumon non à petites cellules, un cancer colorectal, un myélome multiple ou une LAM.

**5.** Sel pharmaceutiquement acceptable selon la revendication 2, pour utilisation dans le traitement d'un cancer chez un patient, dans lequel le cancer est un cancer du poumon à petites cellules, un cancer du poumon non à petites cellules, un cancer colorectal, un myélome multiple ou une LAM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1170024 A
- WO 11107553 A
- WO 11131407 A
- WO 1387699 A
- WO 13119716 A
- WO 13132044 A
- WO 11140249 A
- WO 13169264 A
- WO 14036357 A
- WO 2012145493 A
- WO 2010077634 A
- WO 2007005874 A
- WO 201379174 A
- WO 1019570 A
- WO 1408218 A
- WO 08132601 A
- WO 0944273 A
- WO 1232433 A
- WO 06105021 A
- WO 09009116 A
- WO 11028683 A
- WO 2006122150 A
- WO 0775598 A
- WO 0836653 A
- WO 0836642 A
- WO 0973620 A
- WO 091156652 A
- WO 1156652 A
- WO 12142237 A
- WO 06029879 A
- WO 11109400 A
- WO 2008098104 A


### Non-patent literature cited in the description


- **LEROY et al.** *Mol. Cell,* 2008, vol. 30 (1), 51-60
- **HARGREAVES et al.** *Cell,* 2009, vol. 138 (1), 1294145
- **YOU et al.** *Cell,* 2004, vol. 117 (3), 349-60
- **PRINJHA et al.** *Trends in Pharmacological Sciences,* March 2012, vol. 33 (3), 146-153
- **CONWAY.** *ACS Med. Chem. Lett.,* 2012, vol. 3, 691-694
- **HEWINGS et al.** *J. Med. Chem.,* 2012, vol. 55, 9393-9413
- **WUTS, P. G. M. ; GREENE, T.W.** Protecting Groups in Organic Synthesis. Wiley, 2007
- The Peptides: Analysis, Synthesis, Biology. Academic Press, 1981, vol. 3
- Remington: The Science and Practice of Pharmacy. Pharmaceutical Press, 2012
- Design of Prodrugs. Elsevier, 1985
- Methods in Enzymology. Academic Press, 1985, vol. 112, 309-396
- **BUNDGAARD, H. et al.** Design and Application of Prodrugs,'' A Textbook of Drug Design and Development. Harwood Academic Publishers, 1991, 113-191
- **BUNDGAARD, H.** *Adv. Drug Deliv. Rev.,* 1992, vol. 8, 1-38
- **BUNDGAARD, H. et al.** *J. Pharm. Sci.,* 1988, vol. 77, 285
- **KAKEYA, N. et al.** *Chem. Pharm. Bull.,* 1984, vol. 32, 692
- Prodrugs and Targeted Delivery (Methods and Principles in Medicinal Chemistry). Wiley-VCH, 2011, vol. 47
- Medicinal Chemistry: Principles and Practice. The Royal Society of Chemistry, 2006
- **TESTA, B. et al.** Hydrolysis in Drug and Prodrug Metabolism. Chemistry, Biochemistry and Enzymology. VCHA and Wiley-VCH, 2003
- The Practice of Medicinal Chemistry. Academic Press, 2008
- **GREENE ; WUTS.** Protective Groups In Organic Synthesis. Wiley and Sons, 1999
- **ORJALES, A. et al.** *J. Med. Chem.,* 2003, vol. 46, 5512-5532
- **SEEFELD, M.A. et al.** *PCT Int. Appl.,* 2008
- **F.DEGORCE ; A.CARD, S.SOH ; E.TRINQUET ; G. P. KNAPIK ; B. XIE.** HTRF: A technology tailored for drug discovery - a review of theoretical aspects and recent applications. *Current Chemical Genomics,* 2009, vol. 3, 22-32
- **M.W. PANTOLIANO ; E.C. PETRELLA ; J.D. KWASNOSKI ; V.S. LOBANOV ; J. MYSLIK ; E.GRAF ; T. CARVER ; E. ASEL ; B.A. SPRINGER ; P. LANE.** High-density miniaturized thermal shift assays as a general strategy for drug discovery. *J. Biomol. Screen,* 2001, vol. 6, 429-440

- **M.D. CUMMINGS ; M. A. FAMUM ; M. I. NELEN.** Universal screening methods and application of ThermoFluor. *J. Biomol. Screen,* 2006, vol. 11, 854-863